# EUROPEAN PATENT SPECIFICATION

(11) **EP 1 079 854 B1**
(45) Date of publication and mention of the grant of the patent: **15.04.2009**
(21) Application number: 99930120.3
(22) Date of filing: 26.05.1999
(51) Int. Cl.: A61K 39/00, A61K 48/00, A01N 63/00

(54) **COMPOSITIONS AND METHODS OF MODULATING AN IMMUNE RESPONSE TO AN ANTIGEN BY ADMINISTRATION OF CYTOKINE-COATED CELLS**
ZUSAMMENSETZUNGEN UND VERFAHREN ZUR MODULATION EINER IMMUNANTWORT AUF EIN ANTIGEN DURCH VERABREICHUNG VON ZYTOKINE-BELADENEN ZELLEN
COMPOSITIONS COMPRENANT DES CELLUES TAPISSÉES DE CYTOKINES POUR LA MODULATION D'UNE REPONSE IMMUNITAIRE FACE A UN ANTIGENE ET PROCEDE CORRESPONDANT

(30) Priority: 26.05.1998 US 86780 P; 02.07.1998 US 91525 P
(43) Date of publication of application: 07.03.2001
(73) Proprietor: Genitrix LLC, Cambridge, MA 02142 (US)
(72) Inventor: SEGAL, Andrew, Cambridge, MA 02142 (US)
(74) Representative: Soames, Candida Jane
(86) International application number: PCT/US1999/011609
(87) International publication number: WO 1999/061051

(56) References cited:
- WO-A-97/49421
- WO-A-99/06544
- US-A- 5 374 548
- US-A- 5 665 383
- US-A- 5 747 024
- KATO K ET AL: "Gene transfer of CD40-ligand induces autologous immune recognition of chronic lymphocytic leukemia B cells." 1 March 1998 (1998-03-01), THE JOURNAL OF CLINICAL INVESTIGATION. 1 MAR 1998, VOL. 101, NR. 5, PAGE(S) 1133 - 1141 , XP002286229 ISSN: 0021-9738 * the whole document *
- MENDOZA R B ET AL: "Immunostimulatory effects of a plasmid expressing CD40 ligand (CD154) on gene immunization." JOURNAL OF IMMUNOLOGY (BALTIMORE, MD. : 1950) 15 DEC 1997, vol. 159, no. 12, 15 December 1997 (1997-12-15), pages 5777-5781, XP002286228 ISSN: 0022-1767
- DULLFORCE P ET AL: "Enhancement of T cell-independent immune responses in vivo by CD40 antibodies." NATURE MEDICINE. JAN 1998, vol. 4, no. 1, January 1998 (1998-01), pages 88-91, XP009026323 ISSN: 1078-8956
- KEDAR E ET AL.: "Delivery of cytokines by liposomes. III. Liposome-encapsulated GM-CSF and TNFalpha show improved pharmacokinetics and biological activity and reduced toxicitiy in mice" JOURNAL OF IMMUNOTHERAPY, vol. 20, no. 3, 1997, pages 180-193,
- KARPUSAS M ET AL.: "2 A crystal structure of an extracellular fragemtn of human CD ligand" STRUCTURE, vol. 3, no. 10, 15 October 1995 (1995-10-15), pages 1031-1039, XP004587925
- NAGARAJAN S. ET AL.: "Purification and optimization of functional reconstitution on the surface of leukemic cell lines of GPI-anchored Fcgamma receptor III" JOURNAL OF IMMUNOLOGICLA METHODS, vol. 184, 1995, pages 241-251,
- MCHUGH ET AL.: "Construction, purification , and functional incorporation on tumor cells of glycolipid-anchored human B7-1 (CD80)" PNAS, vol. 92, 1995, pages 8059-8063,

## Description

### FIELD OF THE INVENTION

The invention relates in general to the immune system, and in particular to compositions and methods of modulating an immune response to an antigen.

### BACKGROUND OF THE INVENTION

Cytokines are polypeptide molecules that regulate immune and/or inflammatory reactions by binding to cell-surface receptors on leukocytes. They are active as diffusible, extracellular hormones, although some cytokines also naturally occur in a transmembrane form. They can augment, attenuate, or change the character of an immune or inflammatory reponse. Examples of leukocyte properties that selected cytokines can modulate include proliferative state, transcriptional profile, and propensity to migrate.

Some cytokines can be grouped into families based on similarities of structure and/or function. Examples of such families include interleukins, interferons, chemokines, and colony-stimulating factors (CSF's). A cytokine may belong to more than one family.

An interleukin is a cytokine that is produced by a leukocyte. Among the interleukins are those cytokines that are designated "interleukin-" or "IL-" followed by a number indicating the order in which naming occurred. To date IL-1 through IL-18 have been described. In addition, most other known cytokines are produced by leukocytes and, thus, can be considered interleukins.

The interferons are grouped together on the basis of their ability to inhibit viral replication. The interferons include interferons (IFN) α, β, and γ.

The chemokines induce chemotaxis of leukocytes. In other words, in a concentration gradient of chemokine, a leukocyte will migrate towards the higher concentration. Chemokines comprise two subfamilies designated C-C chemokines, in which two conserved cysteines are contiguous, and the C-X-C chemokines, in which the two conserved cysteines are separated by another amino acid. The C-C chemokines generally attract monocytes and lymphocytes most potently, whereas the C-X-C chemokines generally attract neutrophils most potently. Examples of C-C chemokines include Macrophage Chemotactic and Activating Factor (MCAF), Macrophage Inflammatory Proteins (MIP) 1a and b, and RANTES. Examples of C-X-C chemokines include IL-8, Neutrophil Activating Protein 2 (NAP-2), and Platelet Factor 4 (PF-4).

Colony stimulating factors (CSF's) are grouped together because of their abilities to promote differentiation of bone marrow cells into selected types of leukocytes. Most CSF's also modulate functions of mature, peripheral leukocytes. Examples of CSF's include Macrophage CSF (M-CSF), Granulocyte CSF (G-CSF), and Granulocyte-Macrophage CSF (GM-CSF).

Cytokines can also be classified according to the types of receptors to which they bind. Examples of such types of receptors include the hematopoietin receptors (also known as Class I cytokine receptors), the interferon receptors (also known as the Class II cytokine receptors), the immunoglobulin superfamily receptors, the TNF receptors, and the chemokine receptors.

The hematopoietin receptors comprise two conserved amino acid motifs in their extracellular domains. One of these consists of four positionally conserved cysteines, while the other consists of the sequence Trp-Ser-X-Trp-Ser, where "X" is a nonconserved amino acid. Many receptors in this family comprise multiple polypeptide chains, some of which chains are common to more than one member of the family. The presence of certain common chains defines subfamilies of hematopoietin receptors. For example, members of the GM-CSF receptor subfamily, which include receptors for GM-CSF, IL-3, and IL-5, all comprise an identical signal-transducing β chain. Similarly, members of the IL-6 receptor subfamily, including the receptors for IL-6, IL-11, IL-12, Leukemia Inhibitory Factor (LIF), Oncostatin M, and Ciliary Neurotrophic Growth Factor (CNTF) all comprise the gp130 signal-transducing chain. The IL-2 receptor subfamily is defined by the presence of a signal-transducing γ chain. Members of this subfamily include receptors for IL-2, IL-4, IL-7, IL-9, and IL-15.

The interferon receptors are characterized by the presence of four positionally conserved extracellular cysteines, as in the hematopoietin receptors, and the absence of a Trp-Ser-X-Trp-Ser motif. Members of this family include receptors for interferons α, β, and γ.

Immunoglobulin superfamily receptors have globular domains that are homologous to the globular domains of immunoglobulin constant and variable regions. Cytokine receptors belonging to this family include receptors for IL-1, M-CSF, and c-kit.

Chemokine receptors belong to the large group of G-protein-coupled seven transmembrane domain receptors which contain seven hydrophobic alpha-helical segments that transverse the membrane. These receptors form a structurally related group within the superfamily of G-protein-coupled receptors which mediate signalling via heterotrimeric G-proteins.

It is known in the art that the carboxy-terminus of some cytokines, such as GM-CSF (Brown et al, Eur J Biochem 1994,225: 873-880), is important for interaction with a cognate receptor. Since GPI moieties attach to the carboxy-termini of proteins, one skilled in the art would expect that a heterologous GPI moiety could interfere with the binding of a cytokine such as GM-CSF to its receptor. Furthermore, since soluble cytokines such as chemokines are known to exercise their activities via concentration gradients, one of skill in the art would not expect that membrane-bound versions of such molecules could aid in modulating an immune response. WO 97/49421 refers to a method for inhibiting the growth of brain tumor cells in a patient; the method comprising; co-administering to the patient GM-CSF with tumor antigen.

CD40 is a transmembrane cell surface polypeptide expressed on certain leukocytes, including antigen presenting cells (APCs) such as B cells, macrophages, and dendritic cells. The native ligand for CD40, known as CD40 ligand (CD40L) orCD 154, is also a transmembrane cell-surface polypeptide which is expressed, for example, on CD4+ T helper cells. Engagement of CD40 on IgM-expressing B cells in the presence of IL-4 allows those cells to begin secreting IgG and IgE (Splawski et al, 1993, J Immunol 150: 1276-1285). In addition, engagement of CD40 on certain APCs allows those APCs to stimulate cytotoxic T cells (Ridge et al, 1998, Nature 393: 474478; Bennett et al, 1998, Nature 393: 478-480; Schoenberger et al, 1998, Nature 393: 480-483). On the other hand, ligation of CD40 on dendritic cells decreases their ability to take up exogenous antigen (Sallusto et al, 1995, J Exp Med 182: 389-400; Ruedl et al, 1997, Eur J Immunol 27: 1325-1330).

Mendoza et al (1997, J Immunol 159: 5777-5781) showed that co-injecting a plasmid encoding CD40L with a plasmid encoding a reporter antigen modulated the subject's immune response to the antigen. Dullforce et al (1998, Nature Medicine 4: 88-91) showed that administering anti-CD40 antibodies with polysaccharide antigen enhanced the T-cell independent antibody response to the antigen. Grossmann et al (1997, Human Gene Ther 8: 1935-1943) transferred the gene encoding CD40L into tumor cells and used those cells to vaccinate mice against wild-type tumor cells. Kato et al (1998, J Clin Invest 101: 1133-1141) transduced B cell leukemia cells with a gene encoding CD40L and showed that the transduced cells had APC function in vitro. Couderc et al (1998, Cancer Gene Ther 5: 163-75) also introduced the gene for CD40L into tumor cells and showed that vaccination with these cells induced an antitumor immune response. WO 99/06544 refers to a vaccine, comprising a cell having a membrane-brand fusion protein comprising a non-antibody immunology molecule operatively fused to a heterologous membrane attachment domain.

### SUMMARY OF THE INVENTION

The invention is based, at least in part, on the discovery that cytokine-coated cells, that is, cells which have been modified in such a manner as to bear a cell-surface associated cytokine, modulate the immune response in the recipient to a selected antigen or antigens contained in or attached to the cells.

The invention therefore encompasses a method of vaccinating a mammal to a selected antigen, the method comprising administering to the mammal a vaccine composition comprising a cytokine-coated cell, wherein the cytokine-coated cell comprises the selected antigen.

Preferably, the cytokine-coated cell is admixed with an exogenous engineered cytokine. It is further preferred that the engineered cytokine comprises a lipid moiety. It is preferred that the lipid moiety comprise glycosylphosphatidylinositol (GPI) and/or a fatty acid, e.g.,palmitate.

In a preferred method of the invention, the vaccine composition further comprises opsonin-enhanced cells, that is, cells which have been 1) modified so as to express an opsonin from a recombinant nucleic acid, 2) modified so as to express higher levels of an endogenous opsonin, or 3) mixed with an exogenous opsonin. If the opsonin-enhanced cells are admixed with an exogenous opsonin, the opsonin may be an engineered opsonin that comprises a lipid moiety.

In one embodiment of the invention, vaccination with cytokine-coated cells elicits an immune response mediated by antibodies against an antigen which is comprised by the cells. In another embodiment of the invention, vaccination with cytokine-coated cells elicits an immune response mediated by CD4+ T cells that recognize an antigen which is comprised by the cells. In yet another embodiment of the invention, vaccination with cytokine-coated cells elicits an immune response mediated by CD8+ T cells that recognize an antigen which is comprised by the cells. Of course, in the case of T cells, the antigen is recognized in the context of a major histocompatibility complex molecule.

Preferably, cytokine-coated cell is also an opsonin-enhanced cell. It is also preferred that a cell of the invention be each of a cytokine-coated cell, and an opsonin-enhanced cell.

The invention also encompasses a vaccine composition comprising a cytokine-coated cell for vaccinating a mammal to a selected antigen, wherein the cytokine-coated cell comprises the selected antigen.

As used herein, "contacting" refers to admixing *in vitro.*

As used herein, "time sufficient to permit internalization" refers to a period of time that is of a sufficient duration to allow internalization of the selected antigen by the APC (for example no more than about fourteen days, or seven days, or five or three days, or as little as about 24, 12, 6, 3, 2 or 1 hour, or even as little as about 30, 20, 10, 5, or 1 minute).

Preferably, the cytokine-coated cell comprises an exogenous opsonin and/or an engineered opsonin.

Preferably, the cytokine is a ligand for a receptor for GM-CSF, IL-4, IL-2, IL-6, IL-12, IL-10 or TNF-α.

It is preferred that the cytokine be an "antitumor cytokine". According to the invention, an "antitumor cytokine" is a cytokine that can limit the growth or metastasis of tumor cells in vitro or in vivo, or can prolong the survival of a tumor-bearing animal, as compared to non-tumor bearing control animals, when admixed with the cells or administered to the animal. In a preferred embodiment, an antitumor cytokine can limit the growth or metastasis of tumor cells in vitro or in vivo, by at least 5%, or at least 10-50%, or at least 50-100% or at least 100% or greater, or can prolong the survival of a tumor-bearing animal by at least 5%, or at least 10-50%, or at least 50-100% or at least 100% or greater, as compared to non-tumor bearing control animals, when admixed with the cells or administered to the animal.

As used herein, "expressed" refers to being produced at a level that is detectable by methods including but not limited to ELISA, immunoblotting, immunoprecipitation, flow cytometry, fluorescence microscopy or confocal laser microscopy. Preferably in the method and compositions of the invention a useful protein according to the invention, (e.g. an opsonin, or antigen) is expressed at a level that is at least about 5% greater, preferably about 10-25% greater and more preferably greater than 50-100 of the level of expression of the native form of a useful protein when it is naturally expressed or present in a cell.

As used herein, "recombinant nucleic acid sequence" refers to a nucleic acid sequence constructed by recombinant DNA methods well known in the art and described herein.

Preferably, in the inventive methods and compositions, the cytokine-coated cell is substantially unable to divide in vitro. "Substantially unable to divide *in vitro*" means that the cytokine-coated cells divide at a rate that is less than about 50% than the rate of division of corresponding cells which are not treated to prevent cell division. In a preferred embodiment, substantially unable to divide *in vitro* means that the cytokine-coated cells divide at a rate that is less than about 30-50% of the rate of division of corresponding cells which are not treated to prevent cell division.

It also is preferred that the vaccine composition is attenuated, and therefore is unable to cause a disease that the cells cause in their pathogenic form. As used herein, "attenuated" refers to a state wherein the cells comprising the vaccine are unable to cause disease, e.g., by exposure to ionizing radiation, antiproliferative agents, or by killing with or without fixation.

In one embodiment of the invention, it is preferred that a cytokine comprised by a composition of the invention promote a Th1 immune response, i.e., the generation of T cells that express Th 1 cytokines such as IL-2 and IFN-γ. In another embodiment, it is preferred that a cytokine comprised by a composition of the invention promote a Th2 immune response, i.e., the generation of T cells that express Th2 cytokines such as IL-4 and IL-10.

The cytokine is an engineered cytokine. It is preferred that the cytokine does not naturally occur in a membrane-associated form, or that it not naturally occur in a lipid-linked form. It is preferred that the cytokine be bioactive. It is further preferred that the cytokine be highly bioactive. It is even more preferred that the cytokine be extremely bioactive. It is most preferred that the cytokine be natively bioactive or suprabioactive.

It is preferred that the cytokine be an engineered cytokine comprising a GPI moiety at its carboxyl terminus, and that the carboxyl terminus contribute to the binding to and/or signalling through a receptor for the cytokine. The contribution of the carboxyl terminus can be delineated by constructing recombinant mutants in which the native or naturally occurring form (i.e., non-engineered) of the cytokine is truncated at its carboxyl terminus by about five to fifty amino acids. If binding to a receptor or readout in an assay of bioactivity is decreased by at least about 20% as compared to the non-truncated native, or naturally occurring form of the cytokine, then the carboxyl terminus contributes to the binding to and/or signalling through a receptor for the cytokine. Alternatively, a monoclonal antibody specific for the carboxyl terminus (i.e., up to about 50 amino acids) is admixed with the cytokine in a receptor-binding or bio-assay. If binding to a receptor or readout in an assay of bioactivity is decreased by at least about 20% as compared to the cytokine in the absence of the antibody, then the carboxyl terminus contributes to the binding to and/or signalling through a receptor for the cytokine. Both methodologies are well-known to one skilled in the art.

Preferably, the opsonin of an opsonin-enhanced cell is one of alpha' chain of C3b or mannose binding protein.

If the opsonin is a fragment of C3, it is preferred that it bind to CR1 with a greater affinity than to CR2. It is further preferred that the fragment of C3 not be a ligand for CR2. Preferably, the opsonin is neither C3bi nor C3d nor C3dg.

Preferably, the antigen of a composition of the invention is a pathogenic cell, which maybe a tumor cell which is malignant. As used herein, "malignant" refers to derived from a tumor comprising cells that have the ability to invade surrounding tissue.

Tumor cells that are useful according to the invention include but are not limited to B16 cells, and CMS-5 murine fibrosarcoma cells, and cells derived from the tumors included in the section entitled tumors for which the invention is useful.

The invention also encompasses a pathogenic cytokine-coated cell containing a selected antigen and a recombinant nucleic acid encoding an opsonin, wherein the cell expresses the encoded opsonin, wherein the opsonin is selected from the group consisting of: vitronectin, fibronectin, complement components C1q, ClqA chain, C1qB chain, C1qC chain, complement fragments C3b, C3bi, C3d, C3dg and C4b, mannose binding protein, conglutinin, surfactant proteins A and D, alpha-2-macroglobulin, and immunoglobulins and engineered opsonins.

Preferably, the pathogenic cell is a tumor cell which may be malignant.

It is preferred that the pathogenic cell is selected from the group consisting of: a pathogenic bacterium, a pathogenic fungus, a pathogenic virus, a cell of a pathogenic parasite, a cell of a pathogenic arthropod. Where the invention comprises a vaccine composition comprising an opsonin-enhanced bacterial pathogen, it is preferred that the administered cells comprise opsonin-enhanced pathogenic cells.

Preferably, the host cell is any cytokine-coated cell which is able to act as a carrier for the antigen, and thus may be a nucleated cell or a procaryotic cell. In this aspect of the invention, the host cell need not be pathogenic, but may be any cell into which nucleic acid is introduced artificially. Such cells include but are not limited to a fibroblast, including a specialized mesenchymal cell such as a synoviocyte; a keratinocyte, an epithelial cell, an endothelial cell, a leukocyte, a tumor cell, a bacterial cell, a cell of a fungus, a cell of a parasite.

The invention encompasses a composition comprising a cell admixed with an engineered cytokine.

Preferably, the cell is a pathogenic cell.

It is preferred that the compositions of the invention further comprise opsonin-enhanced cells, as described in Segal, WO 9735619.

Preferably, the composition is substantially free of culture medium. As used herein, "culture medium" refers to medium that is used in cell culture containing at least 2% animal serum, such as fetal calf serum.

The invention also encompasses a composition comprising a cytokine-coated cell, admixed with an opsonin, wherein the cell is substantially unable to divide in vitro.

Preferably, the composition is substantially free of culture medium.

The invention also encompasses a composition comprising a cytokine-coated cell comprising a heterologous antigen, admixed with an exogenous opsonin which is able to bind to the cytokine-coated cell either via covalent binding or via a receptor-ligand binding interaction, e.g., an interaction between a lectin domain of a collectin opsonin and a carbohydrate on the surface of an antigen-containing cell (e.g.,a cytokine-coated cell).

Preferably, the cell is unable to divide in a mammalian host.

Compositions of the invention also may further include a second cytokine, which is preferably admixed with the cell or is expressed by the cell. It is also preferred that the second cytokine be an engineered cytokine.

The invention also encompasses a vaccine composition comprising cytokine-coated cells and a pharmaceutically acceptable carrier. Preferably, the composition further comprises opsonin-enhanced cells.

As used herein, the term "vaccinating" refers to modulating an immune response to a selected antigen such that the response is more or less efficient, more or less rapid, greater or lesser in magnitude, and/or more or less easily induced than the response obtained from administration of a replicate sample of corresponding cells which are identical in every respect except that they are not cells of the present invention for example, cytokine-coated and/or opsonin-enhanced cells or APCs that have internalized a cytokine-coated cell. In a preferred embodiment, vaccinating refers to modulating an immune response to a selected antigen such that the response is between about 5 and 100%, or preferably between about 5 and 50% or more preferably between about 5 and 25% more or less efficient, more or less rapid, greater or lesser in magnitude, and/or more or less easily induced than the response obtained from administration of a replicate sample of corresponding cells which are identical in every respect except that they are not cells of the present invention.

As used herein, the term "vaccine compositions" refers to a composition the administration of which constitutes vaccinating (as defined herein) the subject. In a preferred embodiment, a vaccine composition refers to a composition comprising a cytokine coated cell; a cytokine-coated, opsonin-enhanced cell; an APC cell that has been contacted with either a cytokine-coated cell or a cytokine-coated, opsonin-enhanced cell capable of modulating an immune response to a selected antigen such that the response is about between 5 and 100%, or preferably about between 5 and 50%, or more preferably about between 5 and 25% more or less efficient, more or less rapid, greater or lesser in magnitude, and/or more easily induced than the response obtained from administration of corresponding cells which are identical in every respect except that they are not cytokine coated cells; cytokine-coated, opsonin-enhanced cells; or APC cells that has been contacted with either a cytokine-coated cell or cytokine-coated, opsonin-enhanced cells.

The term "modulate the immune response" may refer to stimulation/activation of an immune response to a selected antigen or it may refer to suppression, elimination, or attenuation of an immune response to a selected antigen. In a preferred embodiment, modulate the immune response refers to stimulation/activation of an immune response to a selected antigen by about at least 5%, or preferably between 5 and 50% or more preferably between 50 and 100%, as compared to an immune response in the absence of vaccination, or it may refer to suppression, elimination, or attenuation of an immune response to a selected antigen by about at least 5%, or preferably between 5 and 50% or more preferably between 50 and 100%, as compared to an immune response in the absence of vaccination.

A "pathogenic" cell is a cell that, in a naturally occuring form, can cause disease, e.g., a tumor cell, an autoreactive T cell, a pathogenic bacterium, a pathogenic fungus, or a cell of a pathogenic parasite. For the purposes of the invention, a pathogenic virus is a pathogenic cell. A pathogenic cell may be modified by attenuating, inactivating, or killing the pathogenic bacterium, fungus, parasite, or virus.

"Exogenous opsonin", "antigen" or "cytokine", refers to an opsonin, antigen or cytokine, which is introduced from or produced outside the cell.

"Endogenous opsonin", "antigen" or "cytokine", refers to an opsonin, antigen or cytokine, which is the native form of an opsonin, antigen, or cytokine which is expressed or present naturally in a cell.

"Heterologous opsonin", "antigen" or "cytokine", refers to an opsonin, antigen, or cytokine, which is not naturally expressed in a cell.

An opsonin of an opsonin-enhanced cell may be expressed as a cytoplasmic, cell surface, or secreted molecule. It is preferred, however, that the opsonin be expressed as a cell surface or secreted molecule.

It is preferred that the antigen and the opsonin are nonidentical. It is also preferred that the antigen and the cytokine are nonidentical. It is further preferred that the cytokine and the opsonin are nonidentical.

In another preferred embodiment, the cytokine is an engineered cytokine which contains a transmembrane segment. As used herein, "transmembrane segment" refers to the hydrophobic region of a transmembrane protein that passes through the cell membrane and interacts with the hydrophobic tails of the lipid molecule in the interior of the cell membrane bilayer.

In another preferred embodiment, the cytokine is an engineered cytokine and comprises a lipid. In yet another preferred embodiment, the lipid is linked to the cytokine via a glycosylphosphatidylinositol moiety.

In one preferred embodiment, the opsonin is an engineered opsonin and comprises a lipid. In a further preferred embodiment, the opsonin, e.g.,a collectin, can bind to the cells noncovalently. The cells can be processed, e.g.,by treatment with a glycosyltransferase or a glycosidase, to facilitate such noncovalent binding.

If the opsonin can be inactivated by a molecule comprised by the cell, as, for example, C3b can be inactivated by CD46, an inhibitor of the inactivator, e.g.,an antibody, can be included in the composition.

"Cell-like" structures, i.e.,structures that comprise a lipophilic membrane that sequesters an internal hydrophilic compartment, can be used in lieu of, i.e.,as the equivalent of, cells in the methods and compositions of the invention. Liposomes are particularly useful cell-like structures. Thus, the invention encompasses a liposome which contains an antigen and which is admixed with a lipid-linked engineered cytokine.

The term "cytokine" as used herein refers to a polypeptide molecule that is naturally secreted by mammalian cells and that binds to a cell surface receptor on a leukocyte, inducing a change (e.g., a change in the proliferative state, a change in the transcriptional profile or a change in the propensity to migrate) in the leukocyte (other than mere occupancy of the leukocyte's receptors for the cytokine). "Change" refers to at least about a 5% increase or decrease as compared to in the absence of a cytokine. The term "cytokine" also refers herein to a polypeptide molecule that is a ligand for a receptor for a naturally occurring cytokine. Unlike an opsonin, a cytokine does not naturally contemporaneously bind an antigen and a cell-surface receptor.

Examples of cytokines which are useful in the methods and compositions of the invention include, but are not limited to the following: GM-CSF, Il-2, IL-4, IL-6, IL-12, ligands for hematopoietin receptors, ligands for immunoglobulin superfamily receptors, ligands for interferon receptors, ligands for TNF receptors, and ligands for chemokine receptors. An antibody against a cytokine receptor can also be a cytokine.

"Engineered cytokines" as described herein are cytokines which comprise a heterologous cell membrane binding moiety.

The term "opsonin" as used herein refers to naturally occurring and non-naturally occurring molecules which are capable, by virtue of being contemporaneously bound or attached to both an antigen-containing cell and an antigen-presenting cell (APC), of acting as a link or coupling agent (an adapter) between the antigen and the APC to allow more efficient binding, engulfment, and internalization of the antigen-containing cell by the APC. An opsonin useful according to the invention, also includes non-naturally occurring opsonins capable of binding to APCs via receptors that can bind naturally occurring opsonins.

The term "opsonin" as used herein can also refer to molecules which can be processed such that at least one product of the processing step or steps is capable of, by virtue of being contemporaneously bound or attached to both an antigen-containing cell and an APC, acting as a link or coupling agent to allow more efficient binding, engulfment, and internalization of other antigen-containing cells by the APC. An opsonin can also be any polypeptide chain of a multichain opsonin.

Examples of opsonins which are useful in the methods and compositions of the invention include the following: vitronectin, fibronectin, complement components such as C1q (including any of its component polypeptide chains A, B and C), complement fragments such as C3d, C3b and C4b, mannose binding protein, conglutinin, surfactant proteins A and D, C-reactive protein (CRP), alpha-2-macroglobulin, and immunoglobulins, for example, the Fc portion of an immunoglobulin.

"Innate opsonins" are opsonins of the innate immune system and are known in the art as secreted polypeptide molecules of the innate immune system and are believed to bind contemporaneously to an antigen and to the surface of an APC. They can thus act as "bridges", and are thought, by virtue of this property, to promote internalization of antigens by APCs. The mode in which opsonins bind to antigens varies among opsonins, and can be covalent or noncovalent. In general, the antigen-binding moieties of innate opsonins differ from the antigen-binding moieties of immunoglobulins in that the former are relatively invariant among members of the same species, and do not undergo diversification during the ontogeny of an individual.

A molecule containing a naturally occurring APC-binding moiety shall be considered an opsonin if it contains a moiety through which it can be stably bound or attached to a cell such that the APC-binding moiety is located in the extracellular space, whether or not the opsonin molecule contains its natural antigen-binding domain.

"Engineered opsonins", as described herein, include molecules in which a cell membrane binding moiety is substituted for the natural antigen-binding domain of an opsonin or where a cell membrane binding moiety is linked to the opsonin without modification or removal of the natural antigen-binding domain of the opsonin.

A "cell membrane binding moiety" of an engineered opsonin, an engineered cytokine, i.e., a moiety through which a molecule can be stably bound to a cell, includes but is not limited to crosslinking moieties and lipid moieties. It is preferred that the cell membrane binding moiety bind to a cell by a means other than interaction of a polypeptide with its cognate cell-surface polypeptide. It is further preferred that the cell membrane binding moiety be a non-polypeptide moiety. In a preferred embodiment, a lipid moiety is linked to the engineered molecule via a glycosylphosphatidylinositol (GPI) moiety. In another preferred embodiment, the lipid comprises a fatty acid, e.g.,palmitate. In yet another preferred embodiment of the invention, the cell membrane binding moiety is linked to the opsonin or the antigen-binding domain-truncated opsonin at the antigen-binding end of the opsonin. In another preferred embodiment, the engineered opsonin comprises an idiotypic portion of an immunoglobulin which can bind to an APC.

It is preferred that the opsonins bind to receptors that trigger phagocytosis and that are non-clonotypic and thus do not vary from cell to cell as, for example, clonotypic receptors do. Non-clonotypic receptors are present on cells which play a role in innate immunity, and include, e.g., non-idiotypic receptors. Examples of such receptors include CR1, CR2, CR3, CR4, and C1q receptor, receptors containing a component of the C1q receptor, collectin receptors, receptors for α2m, receptors for CRP, and Fc receptors for immunoglobulins.

The term "antigen" as used herein refers to a molecule which can initiate a humoral and/or cellular immune response in a recipient of the antigen. Antigens can be any type of biologic molecule including, for example, simple intermediary metabolites, sugars, lipids, and hormones as well as macromolecules such as complex carbohydrates, phospholipids, nucleic acids and proteins. Common categories of antigens include, but are not limited to, viral antigens, bacterial antigens, fungal antigens, protozoa and other parasitic antigens, tumor antigens, antigens involved in autoimmune disease, allergy and graft rejection, and other miscellaneous antigens. In the compositions and methods of the invention, it is preferred that the antigen is a polypeptide, e.g., one comprising at least seven amino acids.

The cytokine-coated cells of the invention can be used, for example, to modulate an immune response in a human to an antigen or antigens contained in the cells. The cells are administered to a mammal, preferably a human, and are taken up (i.e., ingested or phagocytosed) by antigen presenting cells or contacted by other leukocytes, e.g.,lymphocytes or granulocytes. Alternatively, the cells are contacted with antigen presenting cells in vitro under conditions which allow phagocytosis.

As used herein, "antigen presenting cell" or "APC" refers to cells that ingest and present antigen to T cells. These cells include phagocytic leukocytes, monocytes, macrophages, and dendritic cells (for example, Langerhans cells of the skin), B lymphocytes, and endothelial cells. A "professional APC" is an APC that is constitutively able to activate a T lymphocyte. Professional APCs typically constitutively express class II major histocompatibility molecules and costimulatory molecules such as B7-1 and/or B7-2.

The vaccine compositions of the invention can be used, for example, to modulate an immune response in a mammal such as a human.

### DESCRIPTION

The invention is also based, in part, on the surprising and unexpected discovery that cells which bear a cell-surface cytokine can, when administered to a subject, modulate the immune response in the recipient to an antigen or antigens contained in or attached to the cells. Thus the invention relates to membrane-bound versions of such molecules. Furthermore, such cells offer surprising advantages over cells that express the same cytokine in a soluble form or cells that are admixed with the same cytokine in soluble form. The invention provides unexpected results in that the prior art teaches that the carboxy-terminus of some cytokines, such as GM-CSF, is important for interaction with a cognate receptor, and that GPI moieties attach to the carboxy-termini of proteins, and thus the expectation that a heterologous GPI-linked protein would interfere with the binding of a cytokine such as GM-CSF to its receptor. Furthermore, the prior art teaches that many cytokines, such as chemokines, exercise their activities via concentration gradients.

Membrane-bound cytokines offer several advantages over soluble cytokines. Membrane-bound cytokines do not diffuse into the systemic circulation to as great a degree as soluble cytokines, and therefore pose less risk of systemic toxicity. In addition, the close juxtaposition of a membrane-bound cytokine to a cell, compared to an admixed soluble cytokine, permits greater modulation of an immune response to an antigen comprised by the cell. Furthermore, cytokine-coated cells comprising exogenous engineered cytokine can be prepared more quickly and with less labor than cells transduced with a heterologous cytokine gene.

### CYTOKINES USEFUL ACCORDING TO THE INVENTION

The term "cytokine" as defined hereinabove refers to a polypeptide molecule that is naturally secreted by mammalian cells and that binds to a cell surface receptor on a leukocyte. The term "cytokine" also refers herein to a polypeptide molecule that is a ligand for a receptor for a naturally occurring cytokine. Unlike an opsonin, a cytokine does not naturally contemporaneously bind an antigen and a cell-surface receptor.

Leukocytes which bear receptors for cytokines include, for example, monocytes, macrophages, dendritic cells, neutrophils, eosinophils, basophils, platelets, lymphocytes, T lymphocytes, B lymphocytes, NK cells, myeloma cells, lymphoma cells, and leukemic cells.

Without being bound by any one mechanism, it is believed that cell-surface associated cytokines provide an advantage over freely diffusible cytokines by allowing stable juxtaposition of the cytokine to the cell, thus increasing the concentration of cytokine in the vicinity of the cell.

Preferred cytokines are non-rodent cytokines, e.g primate, e.g.,human cytokines.

Some cytokines can be regarded as belonging to one or more families of cytokines based on structural and/or functional properties. One such family consists of the interleukins. Interleukins are structurally diverse, but share the property of both being expressed by and acting on leukocytes. Examples of interleukins include IL-1 (e.g.,polypeptides encoded by Genbank Accession No. M15330, M28983 , E04743, M15131) IL-2 (e.g.,polypeptides encoded by Genbank Accession No. E01108, K02797), IL-3 (e.g.,polypeptides encoded by Genbank Accession No. A02046, M14743), IL-4 (e.g.,polypeptides encoded by M13982, M25892), IL-5 (e.g.,polypeptides encoded by X06270, J03478), IL-6 (e.g.,polypeptides encoded by E02772, M20572), IL-7 (e.g.,polypeptides encoded by J04156, M29054-29057), IL-8 (e.g.,polypeptides encoded by M28130), IL-9 (e.g.,sequences disclosed in Kelleher et al, Blood. 1991; 77: 1436-1441, Immunogenetics 1990;31(4):265-270), IL-10 (e.g.,polypeptides encoded by M84340, U16720), IL-11 (e.g.,sequences disclosed in Paul et al, Proc Natl Acad Sci USA. 1990; 87: 7512-7516, Morris et al, Exp Hematol. 1996; 24: 1369-1376), IL-12 (e.g.,polypeptides encoded by Genbank Accession No. M86671, S82412; Genbank protein P29459, P29460), IL-13 (e.g.,polypeptides encoded by U31120, L13028), 1L-14 (e.g.,sequences disclosed in Ambrus et al, Proceedings of the National Academy of Science (USA) 1993; 90: 6330-4), IL-15 (e.g.,polypeptides encoded by AF031167, U22339), 1L-16 (e.g.,polypeptides encoded by AF006001, M90391), IL-17 (e.g.,polypeptides encoded by U32659, U43088), IL-18 (e.g.,polypeptides encoded by D49949, D49950), TNF-α (e.g.,polypeptides encoded by M16441, Y00467), and GM-CSF (e.g.,polypeptides encoded by X03019, M11220) and their homologues among species. Nucleotide sequences encoding homologues will hybridize to each other under moderate- to high-stringency conditions.

Another family consists of the hematopoietins. Members of this family comprise four α-helical regions, known as helices A, B, C, and D. Helices A and B and helices C and D run roughly parallel to each other, respectively. Examples of hematopoietins include IL-2, IL-3, IL-4, IL-5, IL-6, IL-7, IL-9, IL-11, IL-12, IL-13, 1L-15, GM-CSF, G-CSF (e.g.,polypeptides encoded by Genbank Accession No. E01219, M13926), oncostatin M (e.g.,polypeptides encoded by Genbank Accession No. D31942, sequences disclosed in Malik et al, Mol Cell Biol 1989;9:2847-2853)*,* LIF (e.g.,polypeptides encoded by Genbank Accession No. X13967, X06381), CNTF (e.g.,polypeptides encoded by Genbank Accession No. U05342, X60542), and their homologues among species. Nucleotide sequences encoding homologues will hybridize to each other under moderate- to high-stringency conditions.

Human IL2 is a protein of 133 amino acids (15.4 kDa) with a slightly basic pI. Murine and human IL2 display a homology of approximately 65%. IL2 is synthesized as a precursor protein of 153 amino acids with the first 20 amino-terminal amino acids functioning as a hydrophobic secretory signal sequence. The protein contains a single disulfide bond (positions Cys58/105) essential for biological activity.

IL2 is O-glycosylated at threonine at position 3. Variants with different molecular masses and charges are due to variable glycosylation. Non-glycosylated IL2 is also biologically active. Glycosylation appears to promote elimination of the factor by hepatocytes.

A dimeric form of human IL2, produced by the action of a transglutaminase isolated from regenerating fish optic nerves, has been shown to be a cytotoxic factor for rat brain oligodendrocytes in culture.

The human IL2 gene contains four exons. The IL2 gene maps to human chromosome 4q26-28 (murine chromosome 3). The homology of murine and human IL2 is 72% at the nucleotide level in the coding region.

The biological activities of IL2 are mediated by a membrane receptor that is expressed almost exclusively on activated, but not on resting, T-cells at densities of 4-12x10³ receptors/cell. Activated B-cells and resting mononuclear leukocytes rarely express this receptor. The expression of the IL2 receptor is modulated by IL5 and IL6. Three different types of IL2 receptors are distinguished that are expressed differentially and independently. The high affinity IL2 receptor (Kdis ∼10 pM) constitutes approximately 10% of all IL2 receptors expressed by a cells. This receptor is a membrane receptor complex consisting of the two subunits IL2R-alpha (TAC antigen = T-cell activation antigen; p55) and IL2R-beta (p75; CD122) as the ligand binding domains and a gamma chain as a signaling component. p75 is expressed constitutively on resting T-lymphocytes, NK-cells, and a number of other cell types while the expression of p55 is usually observed only after cell activation. p55 is, however, synthesized constitutively by a number of tumor cells and by HTLV-1-infected cells.

IL2 receptor expression of monocytes is induced by IFN-gamma , so that these cells become tumor-cytotoxic. In T-cells the expression of p75 can be reduced by IL3 . An intermediate affinity IL2 receptor (Kdis =100 pM) consists of the p75 subunit and a gamma chain (see below) while a low affinity receptor (Kdis =10 nM) is formed by p55 alone.

p55 (e.g.,polypeptides encoded by Genbank Accession No. X01057) has a length of 251 amino acids with an extracellular domain of 219 amino acids an a very short cytoplasmic domain of 13 amino acids. The p55 gene maps to human chromosome 10p14-p15.

p75 (e.g.,polypeptides encoded by Genbank Accession No. M26062, M28052) has a length of 525 amino acids with an extracellular domain of 214 amino acids and a cytoplasmic domain of 286 amino acids. The p75 gene contains 10 exons and has a length of approximately 24 kb. It maps to human chromosome 22q11. 2-q12 and to murine chromosome 15 (band E).

A third 64 kDa subunit of the IL2 receptor, designated gamma, has been described (e.g.,polypeptides encoded by Genbank Accession No. D13821, D11086). Murine and human gamma subunits of the receptor have approximately 70% sequence identity at the nucleotide and amino acid levels. This subunit is required for the generation of high and intermediate affinity IL2 receptors but does not bind IL2 by itself. These two receptor types consist of a alpha-beta-gamma heterotrimer and a beta-gamma heterodimer, respectively. The gene encoding the gamma subunit of the IL2 receptor maps to human chromosome Xq13, spans approximately 4.2 kb and contains eight exons. The gamma subunit of the IL2 receptor has been shown recently to be a component of the receptors for IL4 and IL7. It is also believed to be a component of the IL13 receptor.

The amino acids at positions 267-317 lying directly adjacent to the transmembrane region of p75 are involved in IL2-mediated signal transduction. In addition the IL2 receptor is associated with a number of other proteins (p22, p40 , p100) which are thought to be involved in mediating conformational changes in the receptor chains, receptor-mediated endocytosis, and further signal transduction processes. One of the identified proteins is the 95 kDa cell adhesion molecule ICAM-1 which probably focuses IL2 receptors at regions of cell-to-cell contacts and thus may mediate paracrine activities, for example, during IL2-mediated stimulation of T-cells. Another protein associated with p75 is a tyrosine-specific protein kinase called lck . The observation that proliferation of cells induced by IL2 is inhibited by specific inhibitors of protein tyrosine kinases in an lck negative cell line suggests that other kinases may also be associated with IL2 receptors. Two such kinases, called fyn and lyn , have been identified. In addition, IL2 receptor signaling may also be mediated by vav .

Activated lymphocytes continuously secrete a 42 kDa fragment of the TAC antigen. This fragment circulates in the serum and plasma and functions as a soluble IL2 receptor (sIL2R). The concentrations of this soluble receptor vary markedly in different pathological situations, for example, infections, autoimmune diseases, leukemias, or after organ transplantation. Levels may increase up to 100-fold. The levels of sIL2R appear to correlate with the severity of HIV-induces diseases and may be of diagnostic value also in other settings.

Mouse and human IL2 both cause proliferation of T-cells of the homologous species at high efficiency. Human IL2 also stimulates proliferation of mouse T-cells at similar concentrations, whereas mouse IL2 stimulates human T-cells at a lower (sixfold to 170-fold) efficiency.

IL2 is a growth factor for all subpopulations of T-lymphocytes. It is an antigen-unspecific proliferation factor for T-cells that induces cell cycle progression in resting cells and thus allows clonal expansion of activated T-lymphocytes. This effect is modulated by hormones such as prolactin.

IL2 also promotes the proliferation of activated B-cells also this requires the presence of additional factors, for example, IL4.

Due to its effects on T-cells and B-cells IL2 is a central regulator of immune responses. It also plays a role in anti-inflammatory reactions, in hematopoiesis and in tumor surveillance. IL2 stimulates the synthesis of IFN-gamma in peripheral leukocytes and also induces the secretion of IL1, TNF-alpha and TNF-beta.

It is believed that he induction of the secretion of tumoricidal cytokines apart from the activity in the expansion of LAK cells (lymphokine-activated killer cells) are probably the main factors responsible for the antitumor activity of IL2.

IL2 can be assayed in bioassays employing cell lines that respond to the factor (e.g., ATH8, CT6, CTLL-2, FDCPmix, HT-2, NKC3, TALL-103). Specific ELISA assays for IL2 and enzyme immunoassays for the soluble receptor are also available. The soluble receptor can be detected also by employing biotinylated IL2 and flow-through cytometry or ELISA assays.

IL2 displays significant anti-tumor activity for a variety of tumor cell types since it supports the proliferation and clonal expansion of T-cells that specifically attack certain tumors. IL2 is increasingly used to treat patients with cancers refractory to conventional treatment. Combination therapy with systemically administered IL2 has resulted in long-term remissions in 30% of patients with metastatic renal cell carcinoma, for which there is no standard treatment. Objective and long-lived clinical responses have been documented also in a proportion of patients with melanoma or acute myeloid leukemia.

High dose systemic IL2 therapy is also associated with a great number of unwanted toxic side-effects. IL2 has additional effects on other components of the cellular immune system, including B-cells and macrophages, and induces the secretion of other soluble mediators, including TNF-alpha , TNF-beta, and IFN-gamma. These effects may contribute to the antitumor activity of IL2 as well as to its dose-related toxicity.

The transduction of murine tumor cells with a functional IL2 gene has been shown to lead to the rejection of the genetically modified cells by syngeneic hosts. Altered tumor cells expressing IL2 also increase systemic immunity.

Human IL4 is a protein of 129 amino acids (20 kDa) that is synthesized as a precursor containing a hydrophobic secretory signal sequence of 24 amino acids. IL4 is glycosylated at two arginine residues (positions 38 and 105) and contains six cysteine residues involved in disulfide bond formation. The disulfide bonds are essential for biological activity. Some glycosylation variants of IL4 have been described that differ in their biological activities. A comparison of murine and human IL4 shows that both proteins only diverge at positions 91-128.

An IL4 variant, Y124D, in which Tyr124 of the recombinant human protein is substituted by an aspartic acid residue, binds with high affinity to the IL4 receptor (Kd =310 pM ). This variant is a powerful antagonist for the IL4 receptor system. It retains no detectable proliferative activity for T-cells and competitively inhibits IL4-dependent T-cell proliferation (K(i) = 620 pM). The existence of this mutant demonstrates that high affinity binding and signal generation can be uncoupled efficiently in a ligand. Y124D also acts as a powerful antagonist for the IL13 receptor.

The human IL4 gene contains four exons and has a length of approximately 10 kb. It maps to chromosome 5q23-31. The murine gene maps to chromosome 11. The IL4 gene is in close proximity to other genes encoding hematopoietic growth factors (e.g., GM-CSF , M-CSF , IL3 , IL5). The distance between the IL4 and the IL5 gene is approximately 90-240 kb.

At the nucleotide level the human and the murine IL4 gene display approximately 70% homology. The 5' region of the IL4 contains several sequence elements, designated CLE (conserved lymphokine element), that are binding sites for transcription factors controlling the expression of this and other genes. A sequence motif, called P sequence (CGAAAATTTCC) in the 5' region of the human IL4 gene (positions -79 - - 69) is the binding site for a nuclear factor, called NF(P), mediating the response to T-cell activation signals.
The biological activities of IL4 are mediated by a specific receptor (Kdis =20-100 pM) which is expressed at densities of 100-5000 copies/cell (e.g.,polypeptides encoded by Genbank Accession No. M29854, X52425). The extracellular domain of the IL4 receptor is related to the receptors for erythropoietin (Epo), IL6, and the beta chain of the IL2 receptor. It has been given the name CD124.

The cDNA for the murine IL4 receptor encodes a transmembrane protein of 810 amino acids (including a secretory signal sequence ). This receptor has a large intracellular domain of 553 amino acids. The human receptor has an extracellular domain of 207 amino acids, a transmembrane domain of 24 residues, and a large intracellular domain of 569 amino acids.

The IL4 receptor has been shown recently to contain the gamma subunit of the IL2 receptor as a signaling component. This gamma subunit is also associated with the receptors for IL4 and IL7 and probably also of IL13. Two forms of the receptor have been described, one of which is secreted. The secreted receptor only contains the extracellular IL4 binding domain and is capable of blocking IL4 activities. An IL4 binding protein (IL4-BP ) that binds IL4 with the same affinity as the IL4 receptor has been shown also to be a soluble IL4 receptor variant. These soluble receptors probably function as physiological regulators of cytokine activities by inhibiting receptor binding or act as transport proteins. Soluble receptors or binding proteins have been described also for IL1 (IL1 receptor antagonist), IL2, IL6 , IL7 , TNF-alpha , IGF , and IFN-gamma.

The biological activities of IL4 are species-specific; mouse IL4 is inactive on human cells and human IL4 is inactive on murine cells. IL4 promotes the proliferation and differentiation of activated B-cells, the expression of class II MHC antigens, and of low-affinity IgE receptors in resting B-cells. IL4 enhances expression of class II MHC antigens on B-cells. It can promote their capacity to respond to other B-cell stimuli and to present antigens for T-cells. This may be one way to promote the clonal expansion of specific B-cells and the immune system may thus be able to respond to very low concentrations of antigens. The production of IL4 by non-B non-T-cells is stimulated if these cells interact with other cells via their Fc receptors for IgE or IgG. This effect can be enhanced by IL3. IL2 and PAF (platelet activating factor ) induce the synthesis of IL4 while TGF-beta inhibits it.

IL3 antagonizes the IL2-induced effects in B-cells and causes a slow decrease of the expression of IL2 receptors, thus inhibiting the proliferation of human B-cells stimulated by IL2 . In activated B-cells IL4 stimulates the synthesis of IgG1 and IgE and inhibits the synthesis of IgM, IgG3, IgG2a and IgG2b. This isotype switching induced by IL4 in B-cells is antagonized by IFN-gamma. The growth of multiple myelomas can be suppressed by IL4 which inhibits the synthesis of IL6 , a myeloma growth factor. IL4 also inhibits the synthesis of IL6 in human alveolar macrophages.

Pretreatment of macrophages with IL4 prevents the production of IL1, TNF-alpha and prostaglandins in response to activation of the cells by bacterial endotoxins or IFN-gamma.

IL4 synergises with Epo and G-CSF/Epo in the generation of colonies containing granulocytes or erythroid progenitor cells in a colony formation assay.

The classical detection method for IL4 is a B-cell costimulation assay measuring the enhanced proliferation of stimulated purified B-cells. IL4 can be detected also in bioassays , employing IL4-responsive cells (e.g., BALM-4 ; BCL1 ; CT.4S ; CTL44 ; CTLL-2 ; Da ; FDCPmix ; HT-2 ; L4 ; L138.8A; MO7E ; MC/9 ; NFS-60 ; Ramos , Sez627 , TF-1 ; TS1). A specific detection method for human IL4 is the induction of CD23 in a number of B-cell lines with CD23 detected either by flow-through cytometry or by a fluorescence immunoassay. An immunoassay that allows rapid determination of the rate of IL4 production under conditions preventing consumption/degradation is cytokine immunotrapping.

IL4 inhibits the growth of colon and mammary carcinomas. It has been shown to augment the development of LAK cells. The transduction of murine tumor cells with a functional IL4 gene has been shown to lead to the rejection of the genetically modified cells by syngeneic hosts. Altered tumor cells expressing IL4 also increase systemic immunity. Mice vaccinated with transduced cells reject a subsequent challenge of non-transduced cells, and, in some cases, a pre-existing tumor.

Human IL6 is a protein of 185 amino acids glycosylated at positions 73 and 172. It is synthesized as a precursor protein of 212 amino acids. Monocytes express at least five different molecular forms of IL6 with molecular masses of 21.5-28 kDa. They mainly differ by post-translational alterations such as glycosylation and phosphorylation.

IL6 isolated from various cell types shows some microheterogeneity in its N terminus. A 42-45 kDa form has been observed in plasma that is probably complexed with a carrier protein, alpha-2-macroglobulin (α2M). Murine and human IL6 show 65% sequence homology at the DNA level and 42% homology at the protein level. IL6 is a member of a family of cytokines which also includes LIF , CNTF , Oncostatin M, IL11 , and CT-1 . All known members of the IL6 cytokine family induce hepatic expression of acute phase proteins.

A stable and highly bioactive designer cytokine consisting of a fusion protein between IL6 and a soluble IL6 receptor, designated H-IL6 , has been used for human hematopoietic progenitor cell expansion and is useful in cases in which cells do not respond to IL6 but require a stable complex consisting of IL6 and a soluble IL6 receptor.

The human IL6 gene has a length of approximately 5 kb and contains five exons. It maps to human chromosome 7p21-p14 between the markers D7S135 and D7S370. The murine gene maps to chromosome 5. The nucleotide sequences of IL6 and G-CSF genes resemble each other in a way suggesting a possible evolutionary relationship.

The IL6 receptor (e.g.,polypeptides encoded by Genbank Accession No. M20566, E03515) is expressed on T-cells, mitogen-activated B-cells, peripheral monocytes and some macrophage-and B-cell-derived tumor cell types. It is not expressed in resting B-cells but is in resting T-cells. In hepatocytes the IL6 receptor expression is enhanced after treatment with IL6 or IL1 . In several cell types the expression of the IL6 receptor is also enhanced by glucocorticoids. The IL6 receptor gene maps to human chromosome 1q21.

The IL6 receptor is a strongly glycosylated protein of 80 kDa and a length of 449 amino acids. It has been designated CD 126 . It is synthesized as a precursor of 468 amino acids. The molecular structure resembles that of receptors for M-CSF , PDGF and IL1 in that the receptor contains an immunoglobulin-like sequence domain in the aminoterminal region of the extracellular receptor domain.

The intracellular domain of the IL6 receptor has a length of approximately 82 amino acids and does not show any homology to other proteins involved in intracellular signal transduction. Two different forms of the receptor have been described that bind IL6 with different affinities (Kdis =10⁻⁹ and 10⁻¹¹ M) and most likely arise by post-translational modification of the same receptor protein. Biological activities of IL6 have been found also at concentrations of 10⁻¹³ -10⁻¹⁵ M suggesting either the existence of other high-affinity receptor conformations or the existence of further receptor molecules with higher affinities.

IL6 receptor-mediated signal transduction involves protein kinase C and also adenylate cyclase.

The complex formed between IL6 and its receptor associates with a transmembrane glycoprotein, gp130 (918 amino acids; cytoplasmic domain of 277 amino acids), that is involved in signal transduction. Binding of IL6 to its receptor leads to disulfide-linked homodimerization of gp130 and the associated activation of a tyrosine kinase as the first step of signal transduction gp130 is expressed also in cells that do not express IL6 receptors. It has been found to be a component of other receptors, including those for IL11 , LIF , Oncostatin M , and CNTF, and CT-1 This explains why LIF , CNTF, and IL6 share many biological activities although the factors themselves are not related to each other. A factor resembling STAT proteins , termed LIL factor, has been found to be involved in signaling pathways of IL6, and also of IL1 and bacterial lipopolysaccharides.

A soluble form of the IL6 receptor (IL6R-SUP (IL6 receptor soluble urinary protein )) has been described also that also interacts with gp130. These soluble receptors probably function as physiological regulators of cytokine activities by inhibiting receptor binding or act as transport proteins. Similar soluble receptors or binding proteins have been described also for IL1 (IL1ra, IL1 receptor antagonist), IL2, IL4, IL7, TNF-alpha, IGF, and IFN-gamma.

Some cells, including hematopoietic progenitor cells and neuronal cells, are only responsive towards a combination of IL6 and soluble IL6 receptor but not to IL6 alone.

Human IL6 is biologically active in monkeys, rats, and mice. Murine IL6 is not active in human cells. The plethora of biological activities is exemplified by the many different acronyms under which IL6 has been described. IL6 is a pleiotropic cytokine influencing antigen-specific immune responses and inflammatory reactions. It is one of the major physiological mediators of cute phase reaction. In hepatocytes IL6 in combination with glucocorticoids induces the synthesis of metallothioneins and increases intracellular zinc levels, thus preventing CCL4-induced hepatotoxicity.

IL6 is a neurotrophic factor for cholinergic neurons that promotes their survival in culture. Some neuronal cell lines can be induced to differentiate by IL6.

IL6, like IL1, stimulates the synthesis of ACTH (Corticotropin) in the pituitary. Glucocorticoids synthesized in response to ACTH inhibit the production of IL6, IL1 and TNF in vivo, thus establishing a sort of negative feedback loop between the immune system and neuroendocrine functions. In astrocytes IL6 induces the synthesis of Nerve Growth Factor (NGF).

IL6 is a B-cell differentiation factor in vivo and in vitro and an activation factor for T-cells. In the presence of IL2 IL6 induces the differentiation of mature and immature T-cells into cytotoxic T-cells. IL6 also induces the proliferation of thymocytes and probably plays a role in the development of thymic T-cells.

IL6 is capable of inducing the final maturation of B-cells into immunoglobulin-secreting plasma cells if the cells have been pre-activated by IL4 . In B-cells IL6 stimulates the secretion of antibodies to such a degree that serum IgG1 levels can rise 120-400-fold.

IL6 at concentrations of only 0.002 ng/mL is one of the major autocrine growth modulator for many human myelomas. The growth of these cells can be inhibited by monoclonal antibodies directed against IL6. It can be inhibited also by the introduction of antisense oligonucleotides against IL6 or by IL4. The growth-inhibitory effects of corticosteroids on myeloma cells is probably due to the steroid-induced reduction in the expression of IL6. The growth of human IL6 dependent myeloma cells can be inhibited also by IFN-gamma. IL6 may also function as an autocrine growth modulator for other tumor types, some of which have been found to secrete IL6 constitutively. IL6 has been shown to be an autocrine modulator of growth for in vitro cervical tumor cell growth. On the other hand IL6 blocks the growth of some solid tumors such as mammary carcinomas, cervical carcinomas, human lung cancer cell lines, histiocytic lymphomas, and melanomas.

IL6 and IL3 synergise in vitro in promoting the proliferation of multipotent hematopoietic progenitor cells. IL6 is also a thrombopoietin that induces the maturation of megakaryocytes in vitro and increases platelet counts in vivo. In murine, but not in human bone marrow cultures IL6 shows activities resembling those of GM-CSF.

Plasmacytoma cells produce IL6 and also the IL6 receptor. It has been suggested that these cells are stimulated in an autocrine fashion. A paracrine mechanism involving the presence of two different cell populations, one producing the factor and the other expressing the receptor, has been described also.

IL6 can be detected in bioassays employing IL6 responsive cell lines (e.g., 7TD1 ; B9 ; CESS , KPMM2 , KT-3 ; M1 , MH60-BSF-2 , MO7E ; Mono Mac 6 ; NFS-60 ; PIL-6 ; SKW6-C14 ; T1165 ; XG-1). IL6 can be assayed also by its activity as a hybridoma growth factor. Sensitive immunoassays and colorimetric tests are also available. An ELISA assay exists for detecting the receptor-associated gp130 protein.

In combination with other cytokines (for example, IL2) IL6 may be useful in the treatment of some tumor types. The transduction of murine tumor cells with a functional IL6 gene has been shown to lead to the rejection of the genetically modified cells by syngeneic hosts. Altered tumor cells expressing IL6 also increase systemic immunity. Mice vaccinated with transduced cells reject a subsequent challenge of non-transduced cells, and, in some cases, a pre-existing tumor.

Human IL10 is a homodimeric protein with subunits having a length of 160 amino acids. Human IL10 shows 73% amino acid homology with murine IL10. The human IL10 contains four exons. It is closely related to the product of the BCRF-1 gene (Bam HI C fragment rightward reading frame) of Epstein-Barr virus (84% homology at the protein level). These two proteins are more closely related to each other than human and murine IL10. BCRF-1 has therefore also been called viral IL10 (vIL10). The human IL10 gene maps to chromosome 1. The human IL10 shows 81% homology with murine IL10 at the nucleotide level.

A receptor has been identified on murine and human cells by using radiolabeled IL10 (e.g.,polypeptides encoded by Genbank Accession No. L12120, U00672). Mouse IL10 is capable of blocking binding of human IL10 to mouse but not human cells. The murine IL10 receptor has been cloned. This receptor is a protein of approximately 110 kDa that binds murine IL10 specifically. This receptor is structurally related to receptors for IFN .

IL10 inhibits the synthesis of a number of cytokines such as IFN-gamma , IL2 and TNF-beta in Th1 subpopulations of T-cells but not of Th2 cells. This activity is antagonized by IL4 . The inhibitory effect on IFN-gamma production is indirect and appears to be the result of a suppression of IL12 synthesis by accessory cells. In the human system, IL10 is produced by, and down-regulates the function of, Th1 and Th2 cells. In macrophages stimulated by bacterial lipopolysaccharides IL10 inhibits the synthesis of IL1 , IL6 and TNF-alpha by promoting, among other things, the degradation of cytokine mRNA. It also leads to an inhibition of antigen presentation. In human monocytes IFN-gamma and IL10 antagonize each other's production and function. IL10 has been shown also to be a physiologic antagonist of IL12.

IL10 also inhibits mitogen- or anti-CD3-induced proliferation of T-cells in the presence of accessory cells and reduces the production of IFN-gamma and IL2 . Exogenous IL2 and IL4 inhibit the proliferation-inhibitory effect but do not influence the production of IFN-gamma . In LPS-stimulated macrophages IFN-gamma increases the synthesis of IL6 by inhibiting the production of IL10. IL10 appears to be responsible for most or all of the ability of Th2 supernatants to inhibit cytokine synthesis by The cells.

IL10 inhibits secretion of Ig by T-cell-independent antigens induced by IL5 but not that induced by IL2.

Murine Ly-1 B cells are the principal source of IL10. In contrast to other B-cells, Ly-1 B-cells express greatly elevated constitutive and inducible levels of IL10. These cells also have the distinctive property of continuous self-replenishment. The continuous treatment of newborn mice with anti-IL10 antibodies leads to a depletion of the Ly-1 B-cells while maintaining a normal population of splenic B-cells. These mice also contain greatly reduced serum immunoglobulin M levels and are also impaired in their antibody responses to specific antigens. IL10 is therefore a regulator of Ly-1 B-cell development. The mechanism of Ly-1 B-cell depletion appears to involve the increased production of IFN-gamma since coadministration of neutralizing anti-IFN-gamma antibodies substantially restores the number of peritoneal-resident Ly-1 B-cells in these mice.

IL10 is also a costimulator for the growth of mature and immature thymocytes (together with IL2 , IL4 and IL7) and functions as a cytotoxic T-cell differentiation factor, promoting a higher number of IL2-activated cytotoxic T-lymphocyte precursors to proliferate and differentiate into cytotoxic effector cells. IL10 sustains viability of B-cells in vitro and also stimulates B-cells and promotes their differentiation. It enhances the expression of MHC class II antigens on B-cells whereas it inhibits MHC class II expression on monocytes. In B-cells activated via their antigen receptors or via CD40 IL10 induces the secretion of IgG, IgA and IgM. This effect is synergised by IL4 while the synthesis of immunoglobulins induced by IL10 is antagonized by TGF-beta. The activation of macrophages can be prevented by IL10.

It has been shown that human IL10 is a potent and specific chemoattractant for human T-lymphocytes. The chemotactic activity is directed towards cells expressing CD8 and not towards CD4 (+)cells. IL10 also inhibits the chemotactic response of CD4 (+)cells, but not of CD8 (+)cells, towards IL8.

IL10 can be detected with a sensitive ELISA assay. The murine mast cell line D36 can be used to bioassay human IL10. The intracellular factor can be detected also by flow cytometry.

The introduction of an IL10 expression vector into CHO cells has been used to analyze the consequences of local IL10 production in vivo. These altered cells were no longer tumorigenic in nude mice or severe combined immunodeficient SCID mice and also suppressed the growth of equal numbers of co-injected normal CHO cells. While normal CHO tumors are usually substantially infiltrated by macrophages, theese were virtually absent within CHO-IL10 tumor tissues, suggesting that IL10 indirectly suppresses tumor growth of certain tumors by inhibiting infiltration of macrophages which may provide tumor growth promoting activity.

Human IL12 is a heterodimeric 70 kDa glycoprotein consisting of a 40 kDa subunit (p40, 306 amino acids; 10% carbohydrate) and a 35 kDa subunit (p35, 197 amino acids; 20% carbohydrate) linked by disulfide bonds that are essential for the biological activity of IL12. p40 contains 10 cysteines and a binding site for heparin; p35 contains 7 cysteines.

The two subunits of IL12 are not related to any other known proteins. p40 shows some homology with the extracellular domain of the receptor for IL6 , and p35 appears to be a homologue of IL6.

Bioactive murine and human IL12 fusion proteins combining the two IL12 subunits in a single molecule have been described. This designer cytokine retains antitumor activity in vivo. Flexi 12 , a single chain protein retaining all of the biological characteristics of the dimeric recombinant IL12, has also been described.

The gene encoding the p40 subunit of IL12 (IL12B) maps to human chromosome 5q31-q33 in the same region that also harbors other cytokine genes. The gene encoding the p35 subunit of IL12 (IL12A) maps to human chromosome 3p12-q13.2. The expression of the two genes is regulated independently of each other.

The IL12 receptor appears to be a single protein of approximately 110 kDa (e.g.,polypeptides encoded by Genbank Accession No. U03187, U23922, U64198, U64199). Up to 1000-9000 high affinity IL12 receptors/cell are expressed on peripheral blood mononuclear cells activated by various T-cell mitogens or by IL2. IL12 receptors are present on activated T-cells expressing CD4 and CD8 and on activated CD56 positive natural killer cells. Resting peripheral blood mononuclear cells, tonsillar B-cells, or tonsillar B-cells activated by anti-IgM/Dx, anti-IgM/Dx +IL2 , or SAC +IL2 do not express the receptor. High affinity IL12 receptors are expressed constitutively on a transformed marmoset NK-like cell line, HVS.SILVA 40.

Binding of IL12 to its receptor can be prevented by monoclonal antibodies directed against the p40 subunit which therefore contains the binding site. The p40 subunit of IL12 shows homology with the extracellular domain of the IL6 receptor. A virus-encoded homologue of the p40 subunit is EBV-induced gene-3.

Human IL12 is not active in murine lymphocytes. Hybrid heterodimers consisting of murine p35 and human p40 subunits retain bioactivity on murine cells; however, the combination of human p35 and murine p40 is completely inactive on murine cells. Murine IL12 is active on both murine and human lymphocytes. The p40 subunit of murine IL12 subunit p40 (IL12p40) has been shown to specifically antagonize the effects of the IL12 heterodimer in different assay systems and to function as an endogenous specific inhibitor for the IL12 heterodimer.

IL12 stimulates the proliferation of human lymphoblasts activated by phytohemagglutinin IL12 activates NK-cells positive for CD56, and this activity is blocked by antibodies specific for TNF-alpha. IL12 promotes specific allogenic CTL reactions. IL12 synergizes also with anti-CD3 antibodies and with allogeneic stimulation in mixed lymphocyte cultures in inducing T-cell proliferation.

In peripheral lymphocytes of the Th1 type IL12 induces the synthesis of IFN-gamma and IL2 , and TNF . TNF-alpha also appears to be involved in mediating the effects of IL12 on natural killer cells since the effects of IL12 are inhibited by an antibody directed against TNF-alpha. IL12 and TNF-alpha are costimulators for IFN-gamma production with IL12 maximizing the IFN-gamma response; the production of IL12, TNF , and IFN-gamma is inhibited by IL10 . In Th2 helper cells IL12 reduces the synthesis of IL4 , IL5 , and IL10.

IL12 synergises with suboptimal amounts of IL2 in promoting the proliferation of mononuclear cells in the peripheral blood and in promoting the generation of LAK cells (lymphokine activated killer cells). Picomolar concentrations of IL12 are as effective as nanomolar concentrations of IL2 in augmenting the cytolytic activity of natural killer cells expanded in vivo by IL2 . IL12 also acts as a co-mitogen and potentiates the proliferation of resting peripheral cells induced by IL2.

IL12 enhances myelopoiesis of primitive bone marrow progenitor cells induced by SCF (stem cell factor) and synergizes with colony stimulating factors to induce proliferation. IL12 also has synergistic effects on more committed bone marrow progenitors, synergising with IL3 , IL11 , or IL3 plus SCF.

IL12 is of potential clinical interest since it allows the reduction of doses of IL2 required for the generation of LAK cells (lymphokine-activated killer cells). IL12 has been shown to inhibit the growth of a variety of experimental tumors in vivo and to have antiangiogenic effects in vivo, which are, at least in part, mediated by IFN-gamma. IL12 therefore seems to be a potential candidate also for the treatment of angiogenesis-dependent malignancies.

The members of the TNF ligand superfamily (TNFalpha, TNF-beta , LT beta, CD27 ligand , CD30 ligand , CD40 ligand , CD95 ligand, 4 1BB, OX40 ligand , TRAIL) share common biological activities, but some properties are shared by only some ligands, while others are unique. Human TNF-alpha is a non-glycosylated protein of 17 kDa and a length of 157 amino acids. Murine TNF-alpha is N-glycosylated. Homology with TNF-beta is approximately 30%. TNF-alpha forms dimers and trimers. The 17 kDa form of the factor is produced by processing of a precursor protein of 233 amino acids. A TNF-alpha converting enzyme has been shown to mediate this conversion. A transmembrane form of 26 kDa has been described also.

TNF-alpha contains a single disulfide bond that can be destroyed without altering the biological activity of the factor. Mutations Ala84 to Val and Val91 to Ala reduce the cytotoxic activity of the factor almost completely. These sites are involved in receptor binding. The deletion of 7 N-terminal amino acids and the replacement of Pro8Ser9Asp10 by ArgLysArg yields a mutated factor with an approximately 10-fold enhanced antitumor activity and increased receptor binding, as demonstrated by the L-M cell assay, while at the same time reducing the toxicity.

The gene has a length of approximately 3.6 kb and contains four exons. The primary transcript has a length of 2762 nucleotides and encodes a precursor protein of 233 amino acids. The aminoterminal 78 amino acids function as a presequence. The human gene maps to chromosome 6p23-6q12. It is located between class I HLA region for HLA-B and the gene encoding complement factor C. The gene encoding TNF-beta is approximately 1.2 kb downstream of the TNF-alpha gene. However, both genes are regulated independently. The two genes also lie close to each other on murine chromosome 17.

Approximately 500-10000 high-affinity receptors (Ka =2.5x 10⁻⁹ M) for TNF-alpha are expressed on all somatic cell types with the exception of erythrocytes. Two receptors of 55 kDa (TNF-R1; new designation: CD120a) (e.g.,polypeptides encoded by Genbank Accession No. X55313) and 75 kDa (TNF-R2; new designation: CD120b) (e.g.,as described in Goodwin RG et al (1991) Molecular Cellular Biology 11: 3020-6) have been described. One receptor is a glycosylated protein of 455 amino acids that contains an extracellular domain of 171 and a cytoplasmic domain of 221 amino acids. Sequence homologies in the cysteine-rich domains of the extracellular portion reveal that the receptor is related to the low-affinity receptor of NGF and to human cell surface antigen CD40.

Deletion analysis in the C-terminal intracellular region of the 55 kDa receptor, TNF-R1 has revealed the existence of a so-called death domain, which is involved in signaling processes leading to programmed cell death. The death domain of TNF-R1 interacts with a variety of other signaling adaptor molecules, including TRADD , and RIP.

The two known receptors bind both TNF-alpha and TNF-beta. p55 is expressed particularly on cells susceptible to the cytotoxic action of TNF. p75 is also present on many cell types, especially those of myeloid origin (a virus-encoded homologue of the receptor subunit is EBV-induced gene-6 ). It is strongly expressed on stimulated T-cells and B-lymphocytes. The differential activities of TNF on various cell types, i. e. growth-promoting and growth-inhibiting activities, are probably mediated by the differential expression and/or regulation of multiple receptors in combination with other distinct receptor-associated proteins. p55 appears to play a critical role in host defenses against microorganisms and their pathogenic factors.

A third receptor subtype is expressed in normal human liver. It binds TNF-alpha but not TNF-beta. Some viruses contain genes encoding secreted proteins with TNF binding properties that are closely homologous to the p55 and p75 TNF receptors.
Differential effects of the two receptor subtypes have been found also in TNF-mediated adhesion of leukocytes to the endothelium. It appears that engagement of the p55 receptor specifically leads to the induction of the cellular adhesion molecules ICAM-1, E-selectin, V-CAM-1, and CD44, while engagement of both the p55 and the p75 receptor induces expression of alpha-2 integrin.

Truncated soluble forms of the receptor have been found also. The soluble forms, in particular the soluble extracellular domain of the p60 receptor, block the antiproliferative effects of TNF and, therefore, may modulate the harmful effects of TNF.

Receptor densities are reduced by IL1 and tumor promoters such as phorbol esters . The expression of TNF-alpha receptor density is induced by IFN-alpha , IFN-beta, and IFN-gamma.

Signal transducers that associate with the cytoplasmic domains of members of the TNF receptor superfamily comprise TRAF (Tumor necrosis factor receptor-associated factors).

Human TNF-alpha is active on murine cells with a slightly reduced specific activity. In general, TNF-alpha and TNF-beta display similar spectra of biological activities in in vitro systems, although TNF-beta is often less potent or displays apparent partial agonist activity.

TNF-alpha shows a wide spectrum of biological activities. It causes cytolysis and cytostasis of many tumor cell lines in vitro. Sensitive cells die within hours after exposure to picomolar concentrations of the factor and this involves, at least in part, mitochondria-derived second messenger molecules serving as common mediators of TNF cytotoxic and gene-regulatory signaling pathways. The factor induces hemorrhagic necrosis of transplanted tumors. Within hours after injection TNF-alpha leads to the destruction of small blood vessels within malignant tumors. The factor also enhances phagocytosis and cytotoxicity in neutrophilic granulocytes and also modulates the expression of many other proteins, including fos , myc , IL1 and IL6.

The 26 kDa form of TNF is found predominantly on activated monocytes and T-cells. It is also biologically active and mediates cell destruction by direct cell-to-cell contacts.

The chemotactic properties of fMLP (Formyl-Met-Leu-Phe) for neutrophils are enhanced by TNF-alpha. TNF-alpha induces the synthesis of a number of chemoattractant cytokines, including IP-10 , JE , KC , in a cell-type and tissue-specific manner.

TNF-alpha is a growth factor for normal human diploid fibroblasts. It promotes the synthesis of collagenase and prostaglandin E2 in fibroblasts. It may also function as an autocrine growth modulator for human chronic lymphocytic leukemia cells in vivo and has been described to be an autocrine growth modulator for neuroblastoma cells. The autocrine growth-promoting activity is inhibited by IL4.

In resting macrophages TNF induces the synthesis of IL1 and prostaglandin E2. It also stimulates phagocytosis and the synthesis of superoxide dismutase in macrophages. TNF activates osteoclasts and thus induces bone resorption.

In leukocyte and lymphocyte progenitors TNF stimulates the expression of class I and II HLA and differentiation antigens , and the production of IL1, colony stimulating factors, IFN-gamma, and arachidonic acid metabolism. It also stimulates the biosynthesis of collagenases in endothelial cells and synovial cells.

IL6 suppresses the synthesis of IL1 induced by bacterial endotoxins and TNF , and the synthesis of TNF induced by endotoxins.

The neurotransmitter SP (substance P) induces the synthesis of TNF and IL1 in macrophages. IL1 , like IL6 , stimulates the synthesis of ACTH (corticotropin) in the pituitary. Glucocorticoids synthesized in response to ACTH in turn inhibit the synthesis of IL6 , IL1 and TNF in vivo, thus establishing a negative feedback loop between the immune system and neuroendocrine functions.

TNF-alpha enhances the proliferation of T-cells induced by various stimuli in the absence of IL2. Some subpopulations of T-cells only respond to IL2 in the presence of TNF-alpha. In The presence of IL2 TNF-alpha promotes the proliferation and differentiation of B-cells.

The functional capacities of skin Langerhans cells are also influenced by TNF-alpha. These cells are not capable of initiating primary immune responses such as contact sensibilisation. They are converted into immunostimulatory dendritic cells by GM-CSF and also IL1 . These cells therefore are a reservoir for immunologically immature lymphoid dendritic cells. The enhanced ability of maturated Langerhans cells to process antigens is significantly reduced by TNF-alpha.

Although TNF-alpha is also required for normal immune responses the overexpression has severe pathological consequences. TNF-alpha is the major mediator of cachexia observed in tumor patients (hence its name, cachectin). TNF is also responsible for some of the severe effects during Gram-negative sepsis .

TNF-alpha can be detected in bioassays involving cell lines that respond to it (e.g., BT-20 , CT6 , EL4 ; PK15 ; L929 ; L-M ; MO7E ; T1165 ; WEHI-3B). TNF-alpha can be detected also by a sensitive sandwich enzyme immunoassay, ELISA , an immunoradiometric assay (IRMA), and by an assay designated RELAY (receptor-mediated label-transfer assay). Intracellular factor is detected by two color immunofluorescence flow cytometry. Higuchi et al have described an assay based on the release of tritiated thymidine from cells undergoing apoptosis after treatment with either TNF-alpha or TNF-beta . IFN-alpha, IFN-beta, IFN-gamma, TGF-beta, IL4, LIF and GM-CSF have been shown not to interfere with this assay.

In contrast to chemotherapeutic drugs TNF specifically attacks malignant cells. Extensive preclinical studies have documented a direct cytostatic and cytotoxic effect of TNF-alpha against subcutaneous human xenografts and lymph node metastases in nude mice, as well as a variety of immunomodulatory effects on various immune effector cells, including neutrophils, macrophages, and T-cells. Single- and multiple-dose phase I studies have confirmed that TNF can be administered safely to patients with advanced malignancies in a dose range associated with anticancer effect without concomitant serious toxicities such as shock and cachexia. However, clinical trials on the whole have unfortunately so far failed to demonstrate significant improvements in cancer treatment, with TNF-induced systemic toxicity being a major limitation for the use of TNF as an antineoplastic agent in most cases. The combined use of TNF and cytotoxic or immune modulatory agents, particularly IFN-gamma and possibly IL2 , may be of advantage in the treatment of some tumors. In some cases intratumoral application of TNF has been found to be of advantage in tumor control.

Some mutant forms of TNF-beta with selective activity on the p55 receptor have been described recently. It has been shown that activation of the p55 receptor is sufficient to trigger cytotoxic activity towards transformed cells. Some of these mutants have been described to retain their antitumor activity in nude mice carrying transplanted human tumors.

TNF can also be used to increase the aggressiveness of lymphokine-activated killer cells.

Studies with an experimental fibrosarcoma metastasis model have shown that TNF induces significant enhancement of the number of metastases in the lung. It has been suggested that low doses of endogenous TNF or administration of TNF during cytokine therapy may enhance the metastatic potential of circulating tumor cells. The transduction of murine tumor cells with a functional TNF-alpha gene has been shown to lead to the rejection of the genetically modified cells by syngeneic hosts.

The interferons are a family of cytokines that induce a virus-nonspecific antiviral state in target cells. Binding of an interferon to its receptor induces new protein synthesis which, in turn, results in the inactivation of initiation factor eIF-2. The inactivation is thought to contribute to the antiviral state induced by the interferons. Interferons also induce pathways that activate intracellular endonucleases which degrade viral mRNA. Many interferons also possess immunomodulatory activities, such as activation of macrophages and lymphocytes. Examples of interferons include IFN-α (e.g.,polypeptides encoded by Genbank Accession No. K01900, J00209, M12350, J00213, J00216, J00214, M11003, M11026, M34913, M54886, X01974, L38698, M13710, K01238, M13660, M68944, X01972, X01971, X01973, X01969), IFN-β (e.g.,polypeptides encoded by Genbank Accession No. M28622, X14029, X14455, K00020, J00218, E00171, X04430, A09363, M27327, M16656, M25460, K03196), IFN-γ (e.g.,polypeptides encoded by Genbank Accession No. A34532, X87308, E00756, K00083), IFN-ω ( e.g.,polypeptides encoded by Genbank Accession No. X58822, A12140), bovine trophoblast protein-1 (IFN-τ) (e.g.,polypeptides encoded by Genbank Accession No. M31556, M31557, M31558), and their homologues among species. Human IFN-α and IFN-β are thought to bind to a common receptor (e.g.,polypeptides encoded by Genbank Accession No. X60459, M89641) which is distinct from the receptor for IFN-γ (e.g.,polypeptides encoded by Genbank Accession No. J03143, M28233).

At least 23 different variants of IFN-alpha are known. The individual proteins have molecular masses between 19-26 kDa and consist of proteins with lengths of 156-166 and 172 amino acids. All IFN-alpha subtypes possess a common conserved sequence region between amino acid positions 115-151 while the amino-terminal ends are variable. Many IFN-alpha subtypes differ in their sequences at only one or two positions. Naturally occurring variants also include proteins truncated by 10 amino acids at the carboxy-terminal end. Disulfide bonds are formed between cysteines at positions 1/98 and 29/138. The disulfide bond 29/138 is essential for biological activity while the 1/98 bond can be reduces without affecting bioactivity.

Human IFN-beta is a glycoprotein (approximately 20% sugar moiety) of 20 kDa and has a length of 166 amino acids. Glycosylation is not required for biological activity in vitro. The protein contains a disulfide bond Cys31/141) required for biological activity. At the DNA level IFN-beta displays 34% sequence homology with IFN-beta-2 and approximately 30% homology with other IFN-alpha subtypes. In contrast to IFN-gamma IFN-beta is stable at pH2.

Human IFN-gamma is a dimeric protein with subunits of 146 amino acids. The protein is glycosylated at two sites. The pI is 8.3-8.5. IFN-gamma is synthesized as a precursor protein of 166 amino acids including a secretory signal sequence of 23 amino acids. Two molecular forms of the biologically active protein of 20 and 25 kDa have been described. Both of them are glycosylated at position 25. The 25 kDa form is also glycosylated at position 97. The observed differences of natural IFN-gamma with respect to molecular mass and charge are due to variable glycosylation patterns. 40-60 kDa forms observed under non-denaturing conditions are dimers and tetramers of IFN-gamma.

Members of the CSF family of cytokines allow the growth and differentiation of bone marrow cells immobilized on soft agar or methylcellulose. While hematopoietic progenitor cells can be maintained only for short periods of time in the absence of such factors, their presence allows the development of colonies containing erythroid cells, neutrophils, eosinophils, macrophages, and/or megakaryocytes, depending on the particular factor. The biochemical analysis of various activities stimulating colony formation supporting the growth and development of these cell types revealed that there existed many different and distinct factors of this sort.

Many of these factors are either N - or O-glycosylated. Glycosylation has been shown to enhance the solubility, stability and resistance to proteolytic enzymes. It does not appear to be required for the full spectrum of biological activities of these factors. The genes encoding many of the human colony stimulating factors have been cloned and mapped. Some of the genes are in close vicinity but they do not show great homology among each other with the exception of some conserved regions.

Colony stimulating factors are produced by many different cell types, including, for example, B-lymphocytes, epithelial cells, fibroblasts, endothelial cells, macrophages, Stromal cell line , T-lymphocytes. They are synthesized as precursor molecules containing a classical hydrophobic secretory signal sequence of approximately 25-32 amino acids. The secreted factors have an extremely high specific biological activity are active at very low concentrations (1-100 pM ). These factors are absolutely required for the proliferation of hematopoietic progenitor cells. The concentrations required for mere maintenance of viability are usually orders of magnitude lower than those required to induce cell proliferation or to elicit specific functional activities of the cells.

The names of the individual factors usually indicate the cell types that respond to these factors. The classical colony stimulating factors include M-CSF (e.g.,polypeptides encoded by Genbank Accession No. E03235, M64592, U22386, X05010) (macrophage-specific), G-CSF (granulocyte-specific), GM-CSF (macrophage/granulocyte-specific), IL3 (multifunctional) and MEG-CSF (e.g.,polypeptides encoded by Genbank Accession No.D86370, U70136) (megakaryocyte-specific). G-CSF and M-CSF are lineage-specific while GM-CSF and IL3 are multifunctional hematopoietic growth factors acting on earlier stages of differentiation of hematopoietic progenitor cells.

Human GM-CSF is a monomeric protein of 127 amino acids with two glycosylation sites. The protein is synthesized as a precursor of 144 amino acids, which included a hydrophobic secretory signal sequence at the aminoterminal end. The sugar moiety is not required for the full spectrum of biological activities. Non-glycosylated and glycosylated GM-CSF show the same activities in vitro. Fully glycosylated GM-CSF is biologically more active in vivo than the non-glycosylated protein. The different molecular weight forms of GM-CSF (14 kDa, 35 kDa) described in the literature are the result of varying degrees of glycosylation. GM-CSF contains four cysteine residues (positions 54/96 and 88/121).

A comparison of the protein sequence of GM-CSF with those of the other colony stimulating factors reveals that they are not strongly homologous to each other. Human and murine GM-CSF display 60% homology at the protein level and 70% at the nucleotide level. The two factors do not, however, cross-react immunologically. GM-CSF can be associated with the extracellular matrix of cells as a complex with heparan sulfate proteoglycans . This allows storage of the factor in a biologically inactive form. The exact mechanism by which the factor is eventually released from these depots is not known.

The human gene has a length of approximately 2. 5 kb and contains four exons. The distance between the GM-CSF gene and the IL3 gene is approximately 9 kb. The human GM-CSF gene maps to chromosome 5q22-31 in the vicinity of other genes encoding hematopoietic growth factors (M-CSF , IL3 , IL4 , IL5) and the gene encoding the M-CSF receptor. The 5' region of the GM-CSF gene contains several sequence elements known as CLE (conserved lymphokine element). They function as binding sites for transcription factors , modulating the expression of the GM-CSF gene.

GM-CSF receptors are expressed at densities of several 100 to several 1000 copies/cell on the cell surface of myeloid cells. The receptor is expressed also on non-hematopoietic cells such as endothelial cells and small cell lung carcinoma cells. In receptor-positive cell lineages the receptor density decreases with increasing degrees of maturation.

The receptor shows significant homologies with other receptors for hematopoietic growth factors , including IL2-beta, IL3 , IL6 , IL7 , Epo and the prolactin receptors. One cloned subunit of the GM-CSF receptor (GM-R alpha, 45 kDa) binds GM-CSF with low affinity (e.g.,polypeptides encoded by Genbank Accession No. SEG_HUMGRAS). The second subunit (GM-R beta, 120 kDa) does not bind GM-CSF. GM-R alpha is a protein of 400 amino acids that contains only a short cytoplasmic domain of 54 amino acids. The high affinity GM-CSF receptor is formed by the aggregation of the two receptor subunits. The GM-R beta subunit of the receptor (e.g.,polypeptides encoded by Genbank Accession No. SEG_MUSAIC2B, M59941) is also a constituent of other cytokine receptor systems. It is a component of the high affinity receptors for IL3 and IL5 , both of which also contain a cytokine-specific subunit (AIC2A).

Human GM-CSF is not active on murine cells and vice versa. GM-CSF was isolated initially as a factor stimulating the growth of macrophage/granulocyte-containing colonies in soft agar cultures (colony formation assay). GM-CSF is indispensable for the growth and development of granulocyte and macrophage progenitor cells. It stimulates myeloblasts and monoblasts and triggers irreversible differentiation of these cells. GM-CSF synergises with Epo in the proliferation of erythroid and megakaryocytic progenitor cells. In combination with another colony stimulating factor, M-CSF , one observes the phenomenon of synergistic suppression, i.e., the combination of these two factors leads to a partial suppression of the generation of macrophage-containing cell colonies.

For some types of blast cells from patients with acute myeloid leukemia GM-CSF acts as an autocrine mediator of growth. GM-CSF is a strong chemoattractant for neutrophils. It enhances microbicidal activity, oxidative metabolism, and phagocytotic activity of neutrophils and macrophages. It also improves the cytotoxicity of these cells.

GM-CSF displays a less pronounced specificity than, for example, G-CSF . It stimulates the proliferation and differentiation of neutrophilic, eosinophilic, and monocytic lineages. It also functionally activates the corresponding mature forms, enhancing, for example, the expression of certain cell surface adhesion proteins (CD-11A, CD-11C). The overexpression of these proteins could be one explanation for the observed local accumulation of granulocytes at sites of inflammation . In addition, GM-CSF also enhances expression of receptors for fMLP (Formyl-Met-Leu-Phe) which is a stimulator of neutrophil activity.

At pico- to nanomolar concentrations GM-CSF is chemotactic for eosinophils and also influences the chemotactic behavior of these cells in response to other chemotactic factors.

In granulocytes GM-CSF stimulates the release of arachidonic acid metabolites and the increased generation of reactive oxygen species. The activation of the Na+/H+ antiport system leads to a rapid alkalization of the cytosol. Phagocytotic activities of neutrophil granulocytes and the cytotoxicity of eosinophils is also enhanced considerably by GM-CSF. Since GM-CSF is produced by cells (T-lymphocytes, tissue macrophages, endothelial cells, mast cells) present at sites of inflammatory responses it can be assumed that it is an important mediator for inflammatory reactions.

The functional state of Langerhans cells of the skin is also influenced by GM-CSF. These cells are not capable of initiating primary immune responses, for example, contact sensibilization. They are converted to highly potent immunostimulatory dendritic cells by GM-CSF (and also IL1 ). Langerhans cells therefore form an in situ reservoir for immunologically immature lymphoid dendritic cells. The maturation of these cells which is seen as an increased ability to process antigens, can be down-regulated by TNF-alpha.

At nanomolar concentrations GM-CSF induces the expression of complement C3a receptors on basophils. Cells which normally do not respond to C3a and which have been activated by GM-CSF degranulate in response to the C3a stimulus. This is accompanied by the release of histamine and leukotriene C4. This process may be of significance in hypersensitivity reactions associated with inflammatory responses (T-lymphocytes, tissue macrophages, endothelial cells, mast cells). GM-CSF has been shown also to be a potent inducer of trophoblast interferon (TP-1 ).

GM-CSF synergises with some other cytokines , including IL1 , IL3 and G-CSF . GM-CSF and G-CSF must act in concert to allow the development of neutrophil-containing colonies in vitro.

IL3 by itself only negligibly expands the number of circulating blood cells; a subsequent dose of GM-CSF, however, significantly increases cell numbers, probably because IL3 first leads to an expansion of those cells capable of responding to GM-CSF.
The observations that most IL3-dependent cell lines can also grow in the presence of GM-CSF and IL4 and that several synergistic effects are observed between GM-CSF and IL4 suggest that these three factors perform similar functions in controlling the growth of cells. There are some indications that the mechanism of signal transduction contains at least some common factors.

Experiments with tyrosine-specific protein kinases encoded by an oncogene have shown that the expression of this kinase activity in factor-dependent cells abolishes their dependence on GM-CSF, IL3 and IL4. The exact mechanism by which these factors regulate the proliferation and differentiation of cells is still unknown.

The consequences of a deregulated expression of GM-CSF have been studied in transgenic mice harboring a constitutively expressed GM-CSF gene. The overexpression of the transgene encoding GM-CSF leads to pathological alterations in the retina and causes blindness and also causes muscle deterioration. These mice are characterized by a very pronounced increase in activated macrophages. In addition, the overexpression of GM-CSF leads to the activation of mature macrophages secreting large amounts of IL1 and TNF, suggesting that these cytokines may be responsible for some aspects of the transgenic mouse disease.

Histopathological examination demonstrates a pronounced increase in the progenitor cell population of the monocytic lineage. GM-CSF-transgenic animals usually die within months from the massive tissue damage resulting from the overexpression of these factors. Similar results have been obtained with mice possessing a bone marrow manipulated to overexpress GM-CSF by transformation with suitable retrovirus vectors. These findings do not seem to be of clinical significance, though. The long-term treatment of primates and mice with GM-CSF has shown that life-threatening complications do not occur.

The biological consequences of GM-CSF gene disruption have been studied in mice generated from ES cells carrying a targeted deletion of the gene. Mice homozygous for a targeted disruption of the GM-CSF gene are characterized by an unimpaired steady-state hematopoiesis , demonstrating that GM-CSF is not essential for maintaining normal levels of the major types of mature hematopoietic cells and their precursors in blood, marrow, and spleen.

Most GM-CSF-deficient mice are superficially healthy and fertile but develop abnormal lungs. GM-CSF-deficient mice develop a progressive accumulation of surfactant lipids and proteins in the alveolar space, the defining characteristics of the idiopathic human disorder pulmonary alveolar proteinosis. Extensive lymphoid hyperplasia associated with lung airways and blood vessels is found also. These results demonstrate an unexpected, critical role for GM-CSF in pulmonary homeostasis.

Transgenic mice homozygous for null mutations of the gene encoding the common beta subunit (beta C) of the GM-CSF, IL3, and IL5 receptor complexes exhibit normal development and survive to young adult life. They develop pulmonary peribronchovascular lymphoid infiltrates and areas resembling alveolar proteinosis. Eosinophil numbers in peripheral blood and bone marrow of homozygous deletion mutants are reduced, while other hematological parameters are normal. Bone marrow cells from homozygous deletion mutants do not show high-affinity binding of GM-CSF, while cells from heterozygous animals show an intermediate number of high-affinity receptors. In clonal cultures of bone marrow cells derived from homozygous deletion mutants, even high concentrations of GM-CSF and IL5 do not stimulate colony formation in the colony formation assay. Differences in the systemic clearance and distribution of GM-CSF between mutant and wild-type littermates are not observed.

Nishinakamura et al have crossed beta-c mutant mice with mice deficient for IL3 . The double-mutant mice lacking all IL3, GM-CSF, and IL5 functions are apparently normally fertile. The animals show the same reduced numbers of eosinophils and a lack of eosinophilic response to parasites as beta-c mutant mice. The immune response of the double mutant mice to Listeria monocytogenes is normal. Hematopoietic recovery after treatment with fluorouracil is also normal. These findings suggest the existence of alternative mechanism to produce blood cells that do not depend on the presence of IL3, GM-CSF, and IL5.

GM-CSF can be assayed in a colony formation assay by the development of colonies containing macrophages, neutrophils, eosinophils, and megakaryocytes. GM-CSF is also detected in specific bioassays with cells lines that depend in their growth on the presence of GM-CSF or that respond to this factor (e.g., AML-193 ; B6SUt-A ; BAC1.2F5 ; BCL1 ; Da; FDCP1 ; GF-D8 ; GM/SO; IC-2 ; MO7E ; NFS-60 ; PT-18 ; TALL-103 ; TF-1 ; UT-7).

GM-CSF can be employed for the physiological reconstitution of hematopoiesis in all diseases characterized either by an aberrant maturation of blood cells or by a reduced production of leukocytes. The main and most important clinical application of GM-CSF is probably the treatment of life-threatening neutropenia following chemo- and/or radiotherapy, which is markedly reduced under GM-CSF treatment. GM-CSF can be used also to correct chemotherapy-induced cytopenias and to counteract cytopenia-related predisposition to infections and hemorrhages.

In order to avoid potential complications following the administration of GM-CSF careful clinical monitoring is required in certain patient groups, for example those with myelodysplastic syndrome, acute myeloid leukemia, inflammatory disease, autoimmune thrombocytopenia or malfunctional immunological responsiveness.

Several studies have demonstrated that the use of GM-CSF enhances tolerance to cytotoxic drug treatment and can be used to prevent dose reductions necessitated by the side effects of cytotoxic drug treatment. GM-CSF treatment frequently permits to increase the doses of cytotoxic drugs per course. These studies have also revealed a significantly reduced morbidity under GM-CSF treatment.

The transduction of murine tumor cells with a functional GM-CSF gene has been shown to lead to the rejection of the genetically modified cells by syngeneic hosts. Moreover, vaccination with GM-CSF transduced tumor cells prevents growth of a subsequent inoculum of wild-type syngeneic tumor cells.

The chemokine family of cytokines consists of relatively small, structurally similar polypeptides that induce chemotaxis in leukocytes. Chemokines have molecular masses of 8-10 kDa and show approximately 20-50% sequence homology among each other at the protein level. The proteins also share common gene structures and tertiary structures. All chemokines possess a number of conserved cysteine residues involved in intramolecular disulfide bond formation.

According to the chromosomal locations of individual genes two different subfamilies of chemokines are distinguished. Members of the alpha-chemokines are referred to also as the 4q chemokine family because the genes encoding members of this family map to human chromosome 4q12-21. The first two cysteine residues of members of this family are separated by a single amino acids and these proteins, therefore, are called also C-X-C chemokines . This subfamily includes 9E3 (e.g.,Genbank protein P08317), AMCF (e.g.,polypeptides encoded by Genbank Accession No. M99367, M99368), beta-thromboglobulin (e.g.,as disclosed in Begg GS et al (1978), Biochemistry 17: 1739-44), CINC family members (e.g.,polypeptides encoded by Genbank Accession No. D21095), ENA-78 (e.g.,polypeptides encoded by Genbank Accession No. X78686), eotaxin (e.g.,polypeptides encoded by Genbank Accession No. U46572, U40672), GCP-2 (e.g.,polypeptides encoded by Genbank Accession No. Y08770, U83303), IL8, IP-10 (e.g.,polypeptides encoded by Genbank Accession No. L07417, X02530), KC (e.g.,polypeptides encoded by Genbank Accession No. J04596), LIX (e.g.,polypeptides encoded by Genbank Accession No. U27267), mig (e.g.,polypeptides encoded by Genbank Accession No. M34815, Z24725), MGSA (e.g.,polypeptides encoded by Genbank Accession No. X12510), mob-1 (e.g.,polypeptides encoded by Genbank Accession No. U17035), NAP-2 (as described in Clark-Lewis I et al (1991) Biochemistry 30: 3128-35, Cohen AB et al (1992) American Journal of Physiology 263: L249-56), NAP-3 (as described in : Schröder JM et al (1991) Journal of Experimental Medicine 171: 1091-100), NAP-4 (as described in Schröder JM et al (1990) Biochemical and Biophysical Research Communications 172: 898-904), PBSF (SDF) (e.g.,polypeptides encoded by Genbank Accession No. D21072, U16752, D50645), and PF4 (e.g.,polypeptides encoded by Genbank Accession No. M25897).

IL8 , MGSA , mouse KC , MIP-2 (e.g.,polypeptides encoded by Genbank Accession No. X65647 and as descibed in Blum S et al Three human homologues of a murine gene encoding an inhibitor of stem cell proliferation. DNA Cell Biol. 9: 589-602 (1990); Clements JM et al Biological and structural properties of MIP-1 alpha expressed in yeast. Cytokine 4: 76-82 (1992); Devatelis G et al Cloning and characterization of a cDNA for murine macrophage inflammatory protein (MIP), a novel monokine with inflammatory and chemokinetic properties. Journal of Experimental Medicine 167: 1939-44 (1988) (erratum in JEM 170: 2189 (1989)); Farber JM A macrophage mRNA selectively induced by gamma-interferon encodes a member of the platelet factor 4 family of cytokines. Proceedings of the National Academy of Science (USA) 87: 5238-42 (1990); Haskill S et al Identification of three related human GRO genes encoding cytokine functions. Proceedings of the National Academy of Science (USA) 87: 7732-6 (1990); Poltorak AN et al (1995) Journal of Inflammation 45(3): 207-19; Rossi DL et al (1997) Journal of Immunology 158(3): 1033-1036; Sherry B et al (1988) Journal of Experimental Medicine 168: 2251-9; Tekamp-Olson P et al (1990) Journal of Experimental Medicine 172: 911-9; Wolpe SD et al (1989) Proceedings of the National Academy of Science (USA) 86: 612-16; Wolpe SD et al (1989) FASEB Journal 3: 2565-73), NAP-2 , ENA-78 , and GCP-2 comprise a subgroup of the human C-X-C-chemokines defined by the conserved ELR sequence motif (glutamic acid-leucine-arginine) immediately preceding the first cysteine residue near the amino-terminal end. Chemokines with an ELR sequence motif have been found to chemoattract and activate primarily neutrophils. Chemokines without the ELR sequence motif appear to chemoattract and activate monocytes, dendritic cells, T-cells, NK-cells, B-lymphocytes, basophils, and eosinophils.

Members of the beta-chemokines or 17q chemokine family map to human chromosome 17q11-32 (murine chromosome11). The first two cysteine residues are adjacent and, therefore, these proteins are called also C-C chemokines. This subfamily includes ACT-2 (e.g.,polypeptides encoded by Genbank Accession No. J04130), C10 (e.g.,as described in Berger MS et al (1993) DNA Cell Biol. 12: 839-47; Berger MS et al (1996) 8: 439-447), CCF18 (e.g.,as described in Hara T et al (1995) Journal of Immunology 155: 5352-8), DC-CK1 (e.g.,as described in Adema GJ et al (1997) Nature 387: 713-717), ELC (e.g.,polypeptides encoded by Genbank Accession No. AB000887, AF059208), Eotaxin-2 (e.g.,as described in Forssmann U et al (1997) Journal of Experimental Medicine 185: 2171-2176), Exodus (e.g.,polypeptides encoded by Genbank Accession No. U64197, U88320, U88321, U88322), FIC (e.g.,polypeptides encoded by Genbank Accession No. L04694), GDCF and GDCF-2 (e.g.,as descrbed in Kuratsu J et al (1989) Journal of the National Cancer Institute 81: 347-51; Yoshimura T et al (1989) Journal of Experimental Medicine 169: 1449-59; Yoshimura T et al (1989) Journal of Immunology 142: 1956-62), HC-21 (e.g.,as described in Chang HC & Reinherz EL (1989) European Journal of Immunology 19:1045-1051), HCC-1 (e.g.,polypeptides encoded by Genbank Accession No. Z49270), I-309 (e.g.,polypeptides encoded by Genbank Accession No. M57502), JE (e.g.,polypeptides encoded by Genbank Accession No. AF058786, M28226), LAG-1 (lymphocyte activation gene-1) (e.g.,polypeptides encoded by Genbank Accession No. X53683), LARC D86955), LD78 E03130, E03131, MARC (e.g.,as described in Thirion S et al (1994) Biochemical and Biophysical Research Communications 201: 493-499), MCAF M24545 and as described in Apella E et al (1990) Progress in Clinical and Biological Research 349: 405-17), MCP-1 (e.g.,polypeptides encoded by Genbank Accession No. X14768), MCP-2 (e.g.,polypeptides encoded by Genbank Accession No. Y16645), MCP-3 (e.g.,polypeptides encoded by Genbank Accession No. X72308, S71251), MCP-4 (e.g.,polypeptides encoded by Genbank Accession No. X98306), MCP-5 (e.g.,polypeptides encoded by Genbank Accession No. U50712), MIP (macrophage inflammatory protein) (e.g.,polypeptides encoded by Genbank Accession No. U77180, U77035, U49513, M35590), MRP-2 (e.g.,as described in Youn BS et al (1995) Journal of Immunology 155: 2661-7), RANTES SDF (e.g.,polypeptides encoded by Genbank Accession No. M21121, M77747), TARC (e.g Genbank protein Accession No. Q92583).

In addition there are several other factors that are related to chemokines but that either have not been assigned yet to one of the two chemokine groups or that do not possess the classical features of either of the two chemokine groups (for example, ATAC (e.g.,polypeptides encoded by Genbank Accession No. X86474), Ltn (e.g.,polypeptides encoded by Genbank Accession No. U15607, U23772), SCM-1 (e.g.,polypeptides encoded by Genbank Accession No. D63789, D63790, D43769). These have been referred to as C-type chemokines or gamma-chemokines .

Yet another group of chemokines has been identified that comprises neurotactin (e.g.,polypeptides encoded by Genbank Accession No. AF010586, which is characterized by a CX(3)C cysteine signature motif. The existence of clearly defined subgroups of chemokines on the basis of structural and functional properties illustrates the importance of chemoattractant diversity in the regulation of leukocyte movement through the body.

The biological activities of chemokines are mediated by specific receptors and also by receptors with overlapping ligand specificities that bind several of these proteins which always belong either to the C-C-chemokines or the group of C-X-C-chemokines . Chemokine receptors belong to the large group of G-protein-coupled seven transmembrane domain receptors which contain seven hydrophobic alpha-helical segments that transverse the membrane. These receptors form a structurally related group within the superfamily of G-protein-coupled receptors which mediate signalling via heterotrimeric G-proteins.

The receptors that bind C-X-C chemokines are designated CXCR followed by a number (e.g., CXCR-1 (e.g.,polypeptides encoded by Genbank Accession No. L19591),CXCR-2 (e.g.,polypeptides encoded by Genbank Accession No. M94582), CXCR-3 (e.g.,polypeptides encoded by Genbank Accession No. X95876),CXCR-4 (e.g.,polypeptides encoded by Genbank Accession No. D87747, AF025375) while those binding C-C chemokines are designated CCR followed by a number (e.g., CCR-1 (e.g.,polypeptides encoded by Genbank Accession No.L09230, U29678), CCR-2 (e.g.,polypeptides encoded by Genbank Accession No. U29677, U95626), CCR-3 (e.g.,polypeptides encoded by Genbank Accession No. U51241), CCR-4 (e.g.,polypeptides encoded by Genbank Accession No. X90862, X85740), CCR-5 (e.g.,polypeptides encoded by Genbank Accession No. U54994, U83327), CCR-6 (e.g.,polypeptides encoded by Genbank Accession No. U95626), CCR-7 (e.g.,polypeptides encoded by Genbank Accession No. L31581), CCR-8 (e.g.,polypeptides encoded by Genbank Accession No. Z98206, U45983). Viral chemokine receptor homologues include ECRF-3 , EBI-1 (EBV-induced gene-1), and US28.

It is now assumed that the combinatorial effects of multiple chemokines and other mediators are responsible for the cellular composition at inflammatory sites. In addition, many chemokines also directly activate cells. Some of them activate granulocytes and/or monocytes and cause respiratory bursts, degranulation, and the release of lysosomal enzymes. Others prime immune cells to respond to sub-optimal amounts of other inflammatory mediators. Yet others have been shown to be potent histamine releasing factors for basophils. It has been proposed that erythrocytes through their promiscuous chemokine receptor play an important role in regulating the chemokine network. Chemokines bound to the erythrocyte receptor are known to be inaccessible to their normal target cells. This appears to provide a sink for superfluous chemokines and may serve to limit the systemic effects of these mediators without disrupting localized processes taking place at the site of inflammation.

Certain C-C chemokines exhibit biological activities other than mere chemotaxis. Some chemokines have been shown to be capable of inducing the proliferation and activation of killer cells known as CHAK (C-C-chemokine-activated killer), which are similar to cells activated by IL2.

Another particularly useful cytokine according to the invention is flt-3 ligand (e.g.,polypeptides encoded by Genbank Accession Nos. U04806, U04807, U03858, L23636, U29874, U29875, U44024). This cytokine binds to the flt-3 tyrosine kinase (e.g., polypeptides encoded by Genbank Accession Nos. Z26652, X59398). The human flt-3 ligand also stimulates the proliferation of cells expressing murine flt-3 receptors.

The effects of flt-3 ligand are synergized by coexpression of G-CSF , GM-CSF , M-CSF , IL3 , PIXY-321, and SCF. In combination with SCF and IL3 flt-3 ligand can cause expansion of cells with the marker spectrum CD34 (+)CD38 (-).Alone flt-3 ligand supports the survival of precursor cell types in the lineage of blood-forming cells such as CFU-GM , CFU-GEMM , and the very primitive high proliferative potential colony-forming cells. flt-3 ligand only has marginal effects on erythroid and megakaryocyte progenitor cells.

In the mouse, flt-3 ligand potently enhances growth of various types of progenitor/precursor cells in synergy with G-CSF , GM-CSF , M-CSF , IL3 , IL6 , IL7 , IL11 , IL12 and SCF. flt-3 ligand supports growth of LTC-IC (long-term culture-initiating cells). The ability of flt-3 ligand to promote the survival of hematopoietic progenitor cells is abrogated by TGF-beta and counteracted by TNF-alpha .

A study of the expression of functional flt-3 receptor and the responses to the ligand in AML (acute myeloid leukemia) and ALL (acute lymphoblastic leukemia) shows a considerable heterogeneity. BCP-ALL in particular fails to proliferate in the presence of flt-3 ligand despite strong expression of surface flt-3 receptor.

It has been shown that in patients with aplastic anemia and in cancer patients with chemotherapy-induced transient suppression of hematopoiesis , serum levels of flt-3 ligand fluctuate in an inverse relationship to the degree of bone marrow failure. flt-3 ligand levels in serum inversely correlate with the colony forming ability in vitro of bone marrow precursors from patients with aplastic anemia. flt-3 ligand treatment of mice challenged with syngeneic fibrosarcoma cells has been shown to result in complete tumor regression and in decreased tumor growth rates.

Antitumor cytokines are especially useful in the methods and compositions of the invention. According to the invention, an "antitumor cytokine" is a cytokine that can limit the growth or metastasis of tumor cells in vitro or in vivo, or can prolong the survival of a tumor-bearing animal, when either admixed with the cells or administered to the animal. The cytokine can be formulated as a solution in a biologically compatible buffer, e.g.,PBS, and admixed with tumor cells in vitro. The concentration of cytokine may be from about the picomolar range to about the micromolar range. An antitumor cytokine will, for example, reduce the growth rate of the cells, e.g.,by at least 10% compared to buffer alone, or inhibit metastatic properties of the cells, as may be evidenced by, e.g., increased cell adhesiveness or decreased ability to invade an extracellular matrix substrate, such as an artificial basement membrane. Alternatively, an antitumor cytokine may inhibit the growth or metastasis of a tumor in vivo, or may prolong the survival of a tumor-bearing animal. To evaluate the in vivo antitumor effects of a cytokine, the cytokine may be formulated in a pharmaceutically acceptable carrier and administered, e.g., by intravenous, intratumoral, or intraperitoneal injection. The cytokine may also be administered in association with cells, such as tumor cells that express or are coated with the cytokine.

### ASSAYS FOR BIOACTIVITY

According to the invention, it is preferred that a cytokine be "bioactive", "highly bioactive", "extremely bioactive", "natively bioactive", or "suprabioactive". Different levels of bioactivity relate to the ability to induce a change in a leukocyte (other than mere occcupacy of the leukocyte's receptors for the cytokine). According to the invention, all naturally occuring cytokines are natively bioactive. Many types of assay can demonstrate the bioactivity of a non-naturally occurring cytokine. For example, a cytokine may be shown to induce survival and/or proliferation of a particular cell type. As another example, a cytokine may change the concentration of an intracellular second messenger, such as cAMP, arachidonic acid, calcium ions, or inositol triphosphate. The following are examples of assays for bioactivity:

### Assay 1

Each well of one or more 60-well Lux microtiter trays is loaded with 200 FDC-P1 cells in 10 ul Dulbecco's modified Eagle's medium with a final concentration of 10% newborn calf serum. Cytokine in a concentration in at most the micromolar range is added to each well in a volume of 5 ul. The tray is incubated for 48h at 37°C in 10% CO₂, Viable cell counts are performed. The average number of viable cells/well is counted. This assay is useful, for example, for identifying bioactivity mediated through a murine GM-CSF receptor.

### Assay 2

Cytokine sample and a recombinant standard identical to a naturally occurring cytokine are each diluted serially in complete RPMI-10 in 96-well flat-bottom microtiter plates. Each dilution is plated in triplicate. CT.4S cells in active log-phase growth are collected, washed at least twice in complete RPMI-10, and resuspended in complete RPMI-10 at 1 x 10⁵ cells/ml. 50 ul of the cell suspension is added to each well of the plate, which is then incubated for 24h at 37°C in 5% CO₂. Tritiated thymidine is added to each well and the plate is incubated for an additional 24h. The cells are then harvested and tritium incorporation is measured by liquid scintillation counting.. This assay is useful, for example, for identifying bioactivity mediated through an IL-4 receptor.

### Assay 3 (Colony Formation Assay)

Agar (4% w/v) is melted in sterile water by boiling 3 min. The agar is then cooled to 42°C and added to 42°C RPMI-15 to a final concentration of 0.4%. The solution is maintained at 42°C. Femurs are removed from young mice using sterile technique. Marrow is collected by flushing the opened ends of the bones with sterile Hank's Balanced Salt Solution (HBSS) using a syringe equipped with a 23G needle. Marrow is placed in a 15 ml tissue culture tube and vortexed into a cell suspension. Bone fragments are allowed to settle for 5 min, and the supernatant suspension is removed. The suspension is adjusted to 7.5 x 10⁶ nucleated cells/ml and diluted 1:100 by adding the 42° C RPMI with 0.4% agar. 2-fold serial dilutions of cytokine are added to 35 mm tissue culture dishes in a volume < = 0.2 ml. Control dishes have no cytokine added. 1 ml warm cell suspension is added to each dish and the agar is allowed to set at room temperature. The cultures are incubated for 5-7 days at 37° C in 5% CO₂. Colony formation is then evaluated by microscopy. The average number of colonies of a given type (or aggregate number of colonies of given different types) on the cytokine plates and the average number on the control plates is counted.. This assay is useful, for example, for identifying bioactivity mediated through CSF receptors.

### Assay 4

Cytokine is diluted serially in RPMI 1640/25 mM HEPES/1% BSA. 25 ul of each dilution is plated in triplicate in a multiwell chemotaxis chamber bottom. Wells containing medium alone serve as negative controls and wells containing chemotaxis-inducing naturally occurring cytokine serve as positive controls. A polycarbonate membrane is placed over the chamber bottom and the chamber is assembled. 50 ul of peripheral blood mononuclear cells at 1.5 x 10⁶ cells/ml in the RPMI/HEPESBSA is added to each of the upper wells of the chamber. The chamber is incubated for 90 min at 37°C in 5% CO₂. The membrane is removed, washed, and stained. Migrated cells in 3-5 random fields of each well are counted by microscopy.

### Assay 5

Naturally-occurring cytokine reference standard is diluted to 2 ng/ml in a 17 x 100 mm tube using supplemented medium. 3 further 5-fold serial dilutions are also prepared. Serial dilutions of cytokine are prepared in 17 x 100 mm tubes from 2 ng/ml to 20 pg/ml. 50 ul of PHA-activated human lymphoblasts 4 x 10⁵ cells/ml in supplemental medium is added to each well of a 96-well flat-bottom microtiter plate. 50 ul of each dilution of reference standard or cytokine is added to triplicate wells. Negative control wells receive 50 ul of supplemented media alone. The plate is incubated for 48h at 37°C in 5% CO₂ and the cells are labeled with tritiated thymidine Incorporation is measured by liquid scintillation counting.. This assay is useful, for example, for identifying bioactivity mediated through an IL-12 receptor.

### Assay 6

In another assay for bioactivity, an immunocompetent animal is vaccinated with on the order of 10⁴-10⁸ irradiated cytokine-transduced or cytokine-coated tumor cells, and challenged with on the order of 10⁴-10⁸ live wild-type tumor cells (in any temporal sequence). Readouts of the assay are survival, tumor onset, or number of metastases.

Further examples of cytokine assays can be found, e.g., in : Callard RE et al Assay for human B cell growth and differentiation factors. in: Clemens MJ et al (eds) Lymphokines and Interferons. A practical Approach, pp. 345-64, IRL Press, Oxford 1987; Coligan JE et al Current protocols in immunology. Grene and Wiley-Interscience, New York 1991); Dotsika EN Assays for mediators affecting cellular immune functions. Current Opinion in Immunology 2: 932-5 (1989); Feldmann M et al Cytokine assays: role in evaluation of the pathogenesis of autoimmunity. Immunological Reviews 119: 105-123 (1991); Guiguet M et al Misinterpretation of the biological activity of cytokine-containing preparations attributable to unrecognized interacting components. Analytical Biochemistry 247(2): 441-442 (1997); Hamblin AS & O'Garra A Assays for interleukins and other related factors. In: Lymphocytes, a practical approach, Klaus GGB (edt), pp. 209-28, IRL Press, Oxford, ( 1987); Laska EM & Meisner MJ Statistical methods and applications of bioassay. Annu. Rev. Pharmacol. Toxicol. 27: 385-97 (1987); Mosman TR & Fong TAT Specific assays for cytokine production by T cells Journal of Immunological Methods 116: 151-8 (1989); Newton RC & Uhl J Assays relevant to the detection and quantitation of cytokines and their inhibitors. Modem Methods in Pharmacol. 5: 83-99 (1989); Thorpe R et al Detection and measurement of cytokines. Blood Rev. 6: 133-48 (1992); van Zoelen EJ The use of biological assays for detection of polypeptide growth factors. Progress in Growth Factor Research 2: 131-52 (1990); Winstanley FP Cytokine bioassay. In: Gallagher G et al (eds) Tumor Immunobiology, A practical Approach. Oxford University Press, pp. 179-303 (1993); Wadha M et al Quantitative biological assays for individual cytokines. In: Balkwill FR (edt) Cytokines, A practical approach. Oxford University press, pp. 309-330 (1991)

According to the invention, if a non-naturally occurring cytokine gives a readout in a bioactivity assay that is at least 10% but not more than 29% (to the nearest 1%) of the readout yielded by an equimolar amount of a naturally occurring cytokine (the latter giving a positive result in the assay), then the non-naturally occurring cytokine is "bioactive". According to the invention, if a non-naturally occurring cytokine gives a readout in a bioactivity assay that is at least 30% but not more than 49% (to the nearest 1%) of the readout yielded by an equimolar amount of a naturally occurring cytokine (the latter giving a positive result in the assay), then the non-naturally occurring cytokine is "highly bioactive". According to the invention, if a non-naturally occurring cytokine gives a readout in a bioactivity assay that is at least 50% but not more than 69% (to the nearest 1%) of the readout yielded by an equimolar amount of a naturally occurring cytokine (the latter giving a positive result in the assay), then the non-naturally occurring cytokine is "extremely bioactive". According to the invention, if a non-naturally occurring cytokine gives a readout in a bioactivity assay that is at least 70% but not more than 100% (to the nearest 1%) of the readout yielded by an equimolar amount of a naturally occurring cytokine (the latter giving a positive result in the assay), then the non-naturally occurring cytokine is "natively bioactive". According to the invention, if a non-naturally occurring cytokine gives a readout in a bioactivity assay that is greater than 100% of the readout yielded by an equimolar amount of a naturally occurring cytokine (the latter giving a positive result in the assay), then the non-naturally occurring cytokine is "suprabioactive".

### OPSONINS USEFUL ACCORDING TO THE INVENTION

As defined hereinabove, "opsonin" refers to naturally occurring and non-naturally occurring molecules which bind to both antigens and antigen presenting cells (APCs), such as, for example, phagocytic leukocytes (including monocytes and macrophages), dendritic cells (for example, Langerhans cells of the skin), B lymphocytes and, in humans, endothelial cells, or molecules which can be processed such that at least one product of the processing step or steps can bind to both antigens and antigen presenting cells (APCs), such as, for example, phagocytic leukocytes, dendritic cells, B lymphocytes, and, in humans, endothelial cells.

Without being bound to any one mechanism of action, it is believed that opsonin-enhanced cells provide a beneficial effect according to the invention because the opsonin portion acts as a link or coupling agent between the antigen and the APC to allow more efficient binding, engulfment, and internalization of the antigen. In addition, the opsonin itself can be internalized with the antigen. "Internalization" refers to the cellular uptake of a molecule such that it is brought into the cytoplasm or a compartment within the cytoplasm of the cell. Phagocytosis is a process by which a molecule is internalized by a cell.

Preferred opsonins are non-rodent opsonins, e.g., primate, e.g., human, opsonins. Opsonins useful according to the invention bind to receptors on APCs (e.g., phagocytic leukocytes, e.g., macrophages and other cells of the phagocytic system) such as receptors on cells which play a role in innate immunity, as described herein.

Some sets of opsonins can be regarded as structurally and functionally similar. For example, one family comprises fragments of complement components C3 and C4. These two components are highly structurally homologous, and each possesses an intramolecular thiolester bond that is broken when a peptide (C3a or C4a respectively) is proteolytically cleaved from the native molecule. Disruption of the thiolester makes available a chemical structure that can form an ester linkage with an antigen. The moiety of C3 on which this ester bond resides, i.e.,the non-C3a moiety, is designated C3b, and C4b is the analogous product of C4 cleavage. C3b can be further proteolysed by proteins such as factor I to yield fragments such as C3bi and C3d, which also remain linked to the antigen via the ester bond.

There are four structurally unique proteins that are known to function as high affinity receptors for biologically active, membrane-bound fragments of C3 and/or C4. CR1 is the major receptor for the C3b fragment of C3 and C4b fragment of C4. It is expressed on monocytes and monocyte-derived APCs, among other cell types. CR2 is the major receptor for the fragment of C3 known as C3d, and is expressed on, e.g., mature B lymphocytes, but not on cells of monocytic lineage. The major role of CR2 on B lymphocytes is believed to be direct costimulation of B cells in concert with their cognate antigens.

CR3 is expressed primarily by neutrophils and monocytes and is also expressed on FDC, Kupffer cells, and NK cells. CR3 is a C3 fragment receptor with a primary specificity for C3bi. CR3 has been proposed as an important organizer of cytoskeletal events necessary for adhesive interactions and membrane reorganization during processes such as phagocytosis.

CR4 is a member of the beta2 integrin family, and its alpha chain is structurally similar to the alpha chain of CR3 and LFA-1. Its primary physiologic ligands are believed to be C3d and C3d,g;, however, its biologic activities are less well understood than CR3.

Another example of a family of innate opsonins is the collectins, a group of collagenous C-type lectins that comprises complement component C1q, mannose binding protein, surfactant proteins A and D, and conglutinin. Each molecule comprises a lectin domain that can bind to an antigen, and a collagenous domain that can bind to receptors on phagocytic mononuclear cells, including receptors that are wholly or partially identical to the C1q receptor (Nepomuceno et al, Immunity 6:119-29; Tenner et al, Immunity 3:485-93; Guan et al, J Immunol 152:4005-16; Geertsma et al, Am J Physiol 267:L578-84; Miyamura et al, Biochem J 300:237-42; Malhotra et al, J Exp Med 172:955-9; Malhotra et al, Biochem J 293:15-19). Most known collectins comprise multiple polypeptide chains, in some cases homomeric and in others heteromeric, that are assembled post-translationally, in part by covalent cross-linkage of hydroxyproline and hydroxylysine residues. Collectins are demonstrated to be opsonins in, for example, Pikaar et al, J Infect Dis 172:481-9; Alvarez-Dominguez et al, Infection & Immunity 61:3664-72; Kuhlman et al, J Exp Med 169:1733-45; and Geertsma et al, op cit.

Among the other innate opsonins useful according to the invention are C-reactive protein (CRP), alpha-2 macroglobulin, and fibronectin. CRP, a member of the pentraxin family of molecules, binds to receptors on cells of monocytic lineage and has been shown to be an opsonin (Tebo and Mortenson, J Immunol 144:231-8; Holzer et al, J Immunol 133:1424-30). Alpha-2 macroglobulin, like C3 and C4, comprises an internal thiolester bond that can be disrupted when the molecule is proteolysed. Such disruption allows covalent binding of the molecule to an antigen, and binding of alpha-2 macroglobulin to an APC can promote uptake of the conjugate. Fibronectin binds to the alpha 5 beta 1 integrin and can also bind to various antigens, allowing it to function as an opsonin (Cosio, J Lab Clin Med 103:613-9; Czop and Austen, J Immunol 129:2678-81).

Immunoglobulins (antibodies) can function as opsonins by binding antigens via their variable regions and APCs via their constant regions. Typically, an immunoglobulin comprises two heavy chains which are covalently bound to each other and each of which is bound to one light chain. These heterotetramers can further assemble into higher-order structures, such as the pentamers of IgM. Both heavy and light chain variable regions can contribute to the structure of the antigen binding site, whereas the APC binding site is located on the heavy chain constant region. Recombinant single-chain antibodies have also been described. APC receptors for immunoglobulins include Fc alpha, Fc gamma, Fc epsilon, and Fc mu receptors for IgA, IgG, IgE, and IgM, respectively.

Opsonins that are naturally expressed by multicellular eukaryotic organisms are secreted. The latter characteristic distinguishes opsonins from adhesion molecules. A non-naturally occurring molecule containing a naturally occurring APC-binding moiety shall be considered an opsonin if it contains a moiety through which it can be stably bound or attached to a cell such that the APC-binding moiety is located in the extracellular space, whether or not the molecule contains an antigen-binding moiety of a naturally occurring antigen. Moieties through which molecules can be stably bound to a cell include crosslinking moieties, transmembrane sequences, and lipid moieties. The preparation of proteins containing these sequences or moieties is well-known to one of skill in the art.

An "APC binding moiety of an opsonin" is a sequence or domain of an opsonin which when included in a chimeric molecule permits binding of the chimeric molecule to a receptor that is physiologically expressed on an APC with an affinity at least in the nanomolar range.

There are a number of examples of opsonin fragments that comprise APC binding moieties. Such a fragment may be any length so long as it retains an APC binding function; for example, it may be about 40 amino acids, 100 amino acids, 150 amino acids, 500 amino acids, 800 amino acids, or even as long as 3000 amino acids. For example, Las Holtet et al, 1994, FEBS Lett 344:242 describe a carboxy-terminal fragment of human α2m (val1299-ala1451) that binds with high affinity to the α2m receptor. Fragments comprising amino acids 1314-1451 of human α2m and the corresponding domain of rat α2m also bind to α2m receptors, albeit with 1-2% of the affinities of native α2m (Van Leuven et al, 1986, J Biol Chem 261:11369; Enghild et al, 1989, Biochemistry 28:1406; Salvesen et al, 1992, FEBS Lett 313:198; Sottrup-Jensen et al, 1986, FEBS Lett 205:20).

Becherer and Lambris, 1988, J Biol Chem 263:14586 describe fragments of C3b that bind to CR1, e.g., C3c, fragments of C3 generated by elastase treatment and comprising the N-terminal of the alpha' chain of C3b, and a synthetic peptide comprising the 42 N-terminal amino acids of the C3b alpha' chain. A binding sequence in C3 for CR3 has also been described (Wright et al, 1987, PNAS 84:4235).

"Collagen stalks" of C1q, which are N-terminal fragments obtained by pepsin digestion, bind to the C1q receptor (Reid, 1981, Methods Enzymol 80:16; Malhotra et al, 1993, Biochem J 293:15). Malhotra et al, ibid., also provide evidence that an APC binding moiety of conglutinin is comprised by its 55 N-terminal amino acids. Ezekowitz (US Pat 5, 270, 199) offers a putative APC binding site in human mannose binding protein consisting of nucleotides 370-438 of Fig. 2 in the '199 Patent. In addition, by homology with conglutinin, exon 1 disclosed in the'199 Patent may comprise an APC binding moiety.

An APC binding moiety of IgG comprises the CH2 domain and the lower hinge region, including residues 234-237, as described by Canfield and Morrison, 1991, J Exp Med 173:1483-91; Lund et al, 1991, J Immunol 147:2657-62; and Sarmay et al, 1992, Mol Immunol, 29:633-9.

Examples of opsonins which can be used in the compositions and methods of the invention include fibronectin (e.g., Genbank accessions X02761, K00799, K02273, X82402, X00307, X00739), CRP (e.g., Genbank accessions X17496, M11880, M11881, M11882), complement components such as C1q (e.g., Genbank accessions X66295, M22531, X03084, X58861, and Swiss-Prot accessions P02747, P02745), complement fragments such as C3b and C3d (e.g., Genbank accessions K02782, K02765), mannose binding protein (e.g., Genbank accessions S42292, S42294, X15422), conglutinin (e.g., Genbank accession X71774), alpha-2-macroglobulin (e.g., Genbank accessions M93264, M11313), and surfactant proteins A (e.g., Genbank accessions M68519, S48768) and D (e.g., Genbank accessions L40156, X65018, S38981), immunoglobulins, and their homologues among species.

**Table 1**

| Exemplary Opsonin, APC binding moiety/APC receptor pairs useful according to the invention. | | |
|---|---|---|
| **Opsonin** | **Exemplary APC Binding Moiety** | **Receptor** |
| α-2 macroglobulin | Val(1299)-Ala(1451) of human α-2m | α-2m receptor |
| C3b | 42 N-terminal amino acids of the α' chain of human C3b | CR1 |
| C3bi | C3bi | CR2, CR3 |
| C3d | C3d | CR2, CR4 |
| C1q | Collagen stalks (Reid, 1981, Methods Enzymol. 80:16) | Collectin receptor (Nepomuceno et al., 1997, Immunity 6:119) |
| Conglutinin | 55 N-terminal amino acids of bovine conglutinin | Collectin receptor |
| MBP | 1. Polypeptide encoded by nt 370-438 of Fig. 2, U.S. Pat. 5,270,199 | Collectin receptor |
| | 2. Polypeptide encoded by Econ...... I of Fig. 2, U.S. Pat. 5,270,199 | |
| CRP | CRP | CRP receptor |
| Fibronectin | Fibronectin | α5b1 integrin |
| IgG | CH2 domain plus lower hinge including amino acids 234-237, as described by Lund et al., 1991, J. Immunol. 147:2657 | FcγRI |
| Surfactant Protein A | Surfactant Protein A | Collectin receptor |
| Surfactant Protein D | Surfactant Protein D | |

### DETERMINATION OF OPSONICITY ACCORDING TO THE INVENTION

A given naturally occurring opsonin is considered useful according to the invention if it is determined to possess opsonicity according to one or more of the following assays, and if it is a secreted molecule.

### Assay 1

In one assay of opsonicity, as described by O'Rear and Ross in Current Protocols in Immunology, 1994, John Wiley & Sons, pp. 13.4.5-9, SRBC bound via a physiologically occurring linkage to the candidate opsonin molecule are obtained. APCs from the species to which the candidate opsonin is native are suspended at 4x10⁶/ml in ice-cold HBSS with 1% (w/v) Cohn fraction of BSA. If the candidate opsonin is a fragment of C3, the APCs are freshly drawn, uncultivated peripheral blood monocytes. SRBC linked to the candidate opsonin or control SRBC (identical to the former but not linked to the candidate opsonin) are suspended in the same solution at 2x10⁸/ml. 100ul of SRBC suspension and 100ul of APC suspension are mixed in a 10 x 75 mm plastic tube. The tube is rotated at 40 rpm at 37°C for 2 - 20 min. A small drop of the suspension is placed on a slide, covered with a coverslip, and allowed to stand for 5-10 min. Excess fluid can be removed by pressure on the coverslip, and the coverslip can be sealed to the slide, e.g.,with clear nail polish. The slide is examined microscopically, and the percentage of APCs visibly adherent to 4 or more SRBCs is determined. If the percentage is 50% or greater when there are up to 4x10⁴ candidate opsonin molecules/SRBC', the candidate opsonin can be an opsonin.

### Assay 2 (For protease-activated candidate opsonin)

Candidate opsonin or radiolabeled Candidate opsonin is treated with a 1.5-3 fold molar excess of protease (0.05 M triethanolamine-0.1 M NaCl, pH 8.0, room temperature overnight). In this assay, the protease can serve as the antigen or an excess of another antigen can be added. Prior to binding studies, the candidate opsonin-antigen complex is dialyzed against HBSS (4°C).

Candidate opsonin -antigen complex binding to monocytes is measured by incubating labeled ligand at a concentration up to 1.0 M with (1.5-4.0) x 10⁶ monocytes in 200 ml volume on ice. Nonspecific binding of radiolabeled ligands is determined in the presence of a 100-fold molar excess labeled candidate opsonin-antigen complex. The unbound ligand is separated from the cells and cell-bound ligand by rapid vacuum filtration on glass fiber filters. Studies are performed on ice to avoid potential complications due to endocytosis. Binding constarts and the number of sites per cell are determined by analysis and by nonlinear curve fit. If candidate opsonin-antigen complex affinity for a monocyte binding site is in at least the nanomolar range, the candidate opsonin is an opsonin.

### Assay 3

### Part I

To directly evaluate whether candidate opsonin is bound to the surface of *P. carinii,* immunoelectron microscopy is performed. *P. carinii* are isolated from bronchoaveolar lavage (BAL) of moribund infected rats using TBS with 1 mM calcium to preserve surface-bound candidate opsonin. Isolated organisms are fixed in periodate-lysine-paraformaldehyde buffer and embedded in Lowacryl mounting medium (Ted Pella, Inc., Redding, CA). Ultrathin sections are obtained, blocked with normal goat serum (2%) for 1 h, and incubated with either rabbit anti-candidate opsonin or nonimmune rabbit IgG (25 mg/ml) overnight. After washing, the sections are subsequently incubated with goat and rabbit IgG conjugated to 15 nM colloidal gold (Amersham Corp., Arlington Heights, IL). The sections are washed again and examined on a transmission electron microscope (model 6400:JEOL USA, Inc., Peabody, MA).

### Part II

The attachment of *P. carinii* to cultured alveolar macrophages in the presence or absence of antibody to the candidate opsonin or with the addition of purified candidate is quantified as follows. Adherence of *P. carinii* to alveolar macrophages is assayed by ⁵¹Cr-labeling the organisms. *P. carinii* are isolated from infected rats with TBS containing 1 mM calcium to prevent loss of surface-bound candidate opsonin. The organisms are radiolabeled by incubation for 8 h at 37°C in 2 ml of DME containing 20% FCS and 200 mCi of ⁵¹Cr-sodium chromate (New England Nuclear). Normal alveolar macrophages are lavaged from healthy rats and plated in tissue culture plates (1 X 10⁵) cells/well) which are been precoated with normal rat IgG (100 mg/ml X 60 min) in order to ensure firm adherence of the macrophages. After 1 h, the macrophages are gently washed with HBSS to remove nonadherent cells. > 95% of macrophages are adherent after this wash. ⁵¹Cr-*P. carinii* (1 X 10⁶) containing surface-associated candidate opsonin are added to the macrophages and incubated at 37°C for an additional hour. Subsequently, nonadherent *P. carinii* are removed by washing. The macrophage monolayers containing adherent *P. carinii* are solubilized in 1 N NaOH and quantified. Adherence of *P. carinii* is defined as: percentage of adherence = (A/A+ B) X 100, where A = ⁵¹Cr-*P. carinii* associated with the monolayer, and B = unattached ⁵¹ Cr-*P. carinii*. To assess the effect of candidate opsonin on the attachment of *P. carinii* to alveolar macrophage lung cells in culture, *P. carinii* adherence assays are conducted in the presence or absence of a polyclonal rabbit antibody generated against the candidate opsonin (100 mg/ml).

If candidate opsonin binding to *P. carinii* is apparent in Part I and if, in Part II, % adherence is diminished in the presence of anti-candidate opsonin with statistical significance of P < 0.05, the candidate opsonin is an opsonin.

### Assay 4

Association of bacteria with adherent monocytes is measured as follows. Endotoxin level in the modified PBS and in all buffers used is below 50 pg/ml as determined by the Limulus assay. 5 x 10³ monocytes in modified PBS are allowed to adhere to the wells of a Terasaki plate for 2 h at 37°C. After nonadherent cells are removed by three washes with PBS, 5 X 10⁴ FITC-labeled bacteria in 0.5 ml buffer with or without 10-50 micrograms/ml of candidate opsonin are added. A bacteria-to-monocyte ratio of 10:1 to 50:1 is used. After 30 min of incubation at 37°C in the dark, the nonadherent bacteria are removed by five washes with warm PBS. Assays are performed in quadruplicate; in each well, the number of bacteria associated with ³ 100 monocytes is counted under a flourescence microscope using x 400 magnification. Results are expressed as the number of bacteria associated with 100 monocytes. If this number with candidate opsonin can be at least twice that without candidate opsonin, the candidate opsonin is an opsonin.

### Assay 5

### Part I

About 1 x 10⁷ to 6 x 10⁷ bacteria per ml are incubated (20 min, 0°C) with 10 mcg/ml of ¹²⁵I-candidate opsonin in a total volume of 0.7 ml. of PBS aliquots, 100 ml, of the reaction mixtures are layered over 150 ml of an oil cushion (60% dibutyl phthalate, 40% dioctyl phthalate [Eastman Kodak Co., Rochester, N.Y.]), and the mixtures are centrifuged (10,000 x g, 60 s, 4°C). The tip of the tube, containing the cell pellet, is cut with a Mozart razor blade, and the radioactivity is counted.

### Part II

APCs are plated in 96-well tissue culture plates (Costar, Cambridge, Mass.) at 2 x 14⁵ cells per ml the evening before use. 2 x 10⁶ bacteria per well (0.1 ml per well) are added to the culture plates with or without 100 mcg/ml of candidate opsonin. The plates are then centrifuged at 1,000 x g for 7 min. After 15 min at 37°C to allow the uptake of bacteria, free bacteria are removed by several washes with cold PBS. They are then incubated (45 min, 37°C) in RPMI 1640 plus an amount of antibiotic that, when present in the culture for 45 min, kills all extracellular bacteria. The end of this incubation period is considered time zero. Monolayers are washed three times with Hanks' balanced saline solution, and the same volume of RPMI 1640 (R0) is added. The cells are lysed by using several cycles of freezing and thawing. The number (CFU) of viable bacteria per well is determined by quantitative plate counts on blood agar plates (Columbia blood agar; Becton Dickinson, San Jose, Calif.) after 24 h of incubation. Each result is given as the mean of three determinations.

If, in Part I, candidate opsonin-treated bacterial pellet has >75 KCPM and this incorporation can be inhibited by unlabeled candidate opsonin, and if in Part II the CFU with candidate opsonin is greater than without (P< 0.05), the candidate opsonin can be an opsonin.

### Assay 6

200 µl of GHBSS (Hanks Balanced Salt Solution) +0.1%of gelatin containing 10 m mol CaCl₂) containing 10⁷ bacteria is prepared. The bacteria are then incubated at 4°C with 20-100 µg/ml of candidate opsonin. Binding assays are done in the presence or absence of a competitive inhibitor. After incubation for 30 minutes, the bacteria are washed five times in a GHBSS + 10 mmol CaCl₂ at room temperature in a microfuge at 1,300 g for 3 minutes. Thereafter, a 1:1,000 dilution of rabbit anti-candidate opsonin antiserum is incubated with the bacteria for 1 h in PBS + 5% FCS and 10 mmol CaCl₂ and then the bacteria are washed three times in GHBSS + 10 mmol CaCl₂ plus 0.05% Tween 20. Binding of anti-serum to bacteria is detected by a 1:1,000 dilution of goat anti-rabbit IgG conjugated to rhodamine (Fisher Pharmaceuticals, Orangeburg, NY). After incubation, the bacteria are washed five times in GHBSS + 10 mmol CaCl₂ plus 0.05% Tween 20, smeared onto glass slides and allowed to air dry. Thereafter bacteria are fixed with 100% ice cold methanol for 5 minutes. Negative controls included the absence of candidate opsonin and no first step antibody. Numerous fields of triplicate assays are examined by fluorescence microscopy.

### Part II Association of Radiolabeled Bacteria with Cells.

10⁷ radiolabeled bacteria are resuspended in 200 µl of GHBSS + 10 mmol CaCl₂ and are incubated with or without candidate opsonin ranging from 2 µg/ml to 40 µg/ml at 4°C for 30 min. The bacteria are then washed three times in GHBSS + 10 mmol CaCl₂ for 3 min at room temperature in a microfuge at 1,300 g, resuspended in 50 µl of GHBSS and added to a 1-ml suspension containing on the order of 10⁶ APCs (GHBSS). The bacteria and APCs are gently rocked at 37°C for 20 min and thereafter the unattached bacteria are removed by five washes using differential centrifugation at 82 g in a microfuge. Before the last wash, an aliquot from each sample is plated on a Labtek slide and cells are adhered for 10 min, fixed in methanol, stained with Giemsa, and scored by light microscopy. To score the cells plated on the Labtek slides, at least 400 cells are counted. The phagocytic index represented the number of attached or ingested particles per 100 PMNs. The pellet from above containing cells and radiolabeled bacteria is then lysed in 100 µl PBS + 0.5% Triton X-100 and the radioactivity is measured in a scintillation counter. If, in Part I, specific binding of candidate opsonin to bacteria is evident, and in Part II the specific uptake of bacteria, in cpm, is more than three times greater with candidate opsonin than without, the candidate opsonin can be an opsonin.

### Assay 7

### Part I

To investigate binding to *L donovani promastigotes* cultures are seeded at 5 x 10⁵ parasites ml⁻¹. At regular time points up to 9 days, a fraction of parasites are counted, washed, and resuspended in 1% BSA, 0.5 mM Ca²⁺. 0.05% NaN₃, Tris-buffered saline (TBS), (10 mM Tris-HCl, 0.15 M NaCl, pH 8.0) (diluent) to 2 x 10⁵ ml⁻¹. Fifty microliters of this suspension are then added to 200-µl microfuge tubes containing 70 µl 5 µg/ml radiolabled candidate opsonin (0.12 µCi/µg) in diluent without EDTA, which had been layered over 150 µl of a dinonyl phthalate/dibutyl phthalate (40:60 v/v) oil mixture. Parasites are incubated for 1 h and centrifuged through the oil layer, the cell pellet is cut off, and associated candidate is detected by gamma counting. Each assay is performed in triplicate. The concentration dependency of candidate binding to promastigotes is also measured as above, using an activity of 0.045 µCi/µg and a twofold dilution series from 60 to 0.015 µg/ml candidate.

### Part II

APCs are plated out at 1 x 10⁶ cells/well on glass coverslips in a 24-well tissue culture plate. Cells are incubated in RPMI 1640 (Life Technologies) supplemented with 10% PCS, 1 mM glutamine, 200 U/ml penicillin and 200 µg/ml streptomycin in a humidified incubator at 37°C. After 24 h, nonadherent cells are removed and remaining cells are used after 6 days. Promastigotes are incubated with or without candidate at 30 µg/ml in RPMI 1640 for 1 h and then washed three times before adding to the APC cultures at 10⁶/well. Promastigotes are allowed to infect APCs for 1 h, then cells are washed, fixed with methanol, and Geimsa stained (BDH, Poole, Dorset, U.K.) before counting. The percentage of APCs infected and the number of parasites/100 macrophages is determined from quadruplicate cultures.

If in Part I the affinity of candidate opsonin for parasites is at least in the nanomolar range and in Part II the number of parasites taken up/100 APCs is, with candidate opsonin, at least twice that without candidate opsonin, the candidate opsonin can be an opsonin.

### Assay 8

### Part I

Portions (0.5 ml) of [³⁵S] methionine-labeled culture medium containing 5 percent fetal calf serum and the candidate opsonin are incubated for 30 minutes at room temperature with 0.1 ml or 0.2 ml of a 10 percent suspension of a microorganism). The microorganisms tested may include, for example, *Salmonella typhimurium, Bacillus subtilis, Staphylococcus aureus, Escherichia coli,* and *Saccharomyces cerevisiae*. Bound proteins are released by boiling in buffer containing 2 percent SDS and 0.1 M dithiothreitol and are analyzed on a 5 percent SDS gel.

### Part II

Fixed bacteria (0.1 ml; 10 percent by volume; 10¹⁰ organisms per millileter), labeled with [³H]thymidine, are incubated with 0.1 ml of serum with or without depletion of the candidate opsonin. After being washed with PBS, the bacteria are incubated with on the order of 1 x 10⁷ APCs in a final volume of 0.9 ml PBS containing divalent cations. At intervals 0.2 ml is removed to ice-cold PBS with N-ethyimaleimide (2mM) to block further endocytosis, and the cells are washed (at about 100g for 10 seconds)

If in Part I a band corresponding to the candidate opsonin is apparent, and if in Part II the CPM after 6-10 min of incubation is at least three times greater for undepleted samples with serum than with depleted serum, the candidate opsonin can be an opsonin.

In lieu of results form Parts I of assays 3, 5, 6, 7, 8, a candidate opsonin that satisfies Part II of an assay can be an opsonin if it can bind to the antigen of the assay with an affinity in at least the nanomolar range.

### Assay 9

SRBC coated with at least 1.2 x 10⁴ molecules/cell of a fragment of C3 are prepared as described by O'Rear and Ross in Current Protocols in Immunology, 1994, John Wiley & Sons, pp. 13.4.5-9. 250 ul of monocytes at 2 x 10⁵ cells/ml of RPMI with 10% fetal calf serum are added to each well of an 8-well glass tissue culture plate and incubated at 37°C, 5% CO₂ for 3h. The monocytes are washed twice with HBSS, and 50 ul of the SRBC at 1.5 x 10⁸/ml of DVBS²⁺ are added to each well. The plate is centrifuged at 50g for 5 min and then incubated at 37°C, 5% CO₂ for 3h. The walls are washed twice with HBSS, fixed with 0.5% glutaraldehyde, and stained with Giemsa stain. If >40% of the monocytes form rosettes with at least 1 SRBC as determined by light microscopy, the candidate can be an opsonin.

### ENGINEERED OPSONINS, OR CYTOKINES, CONTAINING A LIPID

The attachment of a lipid, e.g.,a long-chain fatty acid, to a molecule, e.g.,a polypeptide, can permit the complex to become stably associated with the plasma membrane when the complex is admixed with a cell (Nagarajan et al, 1995, J Immunol Methods 184:241-51; McHugh et al, 1995, PNAS 92:8059-63; van den Berg et al, 1995, J Cell Biol, 131:669-77). This is believed to occur through intercalation of the lipid into the membrane. A convenient method of producing a lipid-associated polypeptide comprises expressing, in a suitable host cell, a nucleic acid encoding, in part, a signal sequence directing the post-translational addition of a GPI moiety. Using recombinant DNA technology, a naturally non-GPI linked protein can be expressed as a GPI-linked protein by constructing a nucleic acid that encodes the protein linked to a heterologous GPI signal sequence. Nucleotide sequences encoding GPI signal sequences useful for this purpose include, for example, those comprised by decay accelerating factor (e.g., sequences encoding amino acid sequence "22" in Table 1 ofBucht and Hjalmarsson, 1996, Biochim Biophys Acta 1292:223-32; sequences encoding signal sequences disclosed in Caras et al, U.S. Pat. 5, 109,113); brevican (e.g., nt 1982-2047 of Genbank accession number X86406), mesothelin (e.g., nt 1858-1983 of Genbank U40434), coccidioides immitis antigen 2 (e.g., sequences encoding amino acids 172-194 of NCBI Entrez protein database accession # 1256444, Zhu et al, 1996, Gene 181:121-5), acetylcholinesterase (e.g., sequences encoding the peptide "HC" as described in Duval et al, 1992, EMBO J 11:3255-61; (e.g., sequences encoding amino acid sequence "19" in Table 1 of Bucht and Hjalmarsson, 1996, Biochim Biophys Acta 1292:223-32)), human folate receptors alpha and beta (e.g., sequences encoding amino acids 230-257 of NCBI Entrez protein database accession # 182416 or amino acids 228-255 of NCBI Entrez protein database accession # 1655592, Yan and Ratnam, 1995, Biochemistry 34:14594-600), 5' nucleotidase (e.g., sequences encoding amino acids 547-570 or 547-574 of NCBI Entrez protein database accession # 404502, Furukawa et al, 1994, Biochim Biophys Acta 1190:273-8; (e.g., sequences encoding amino acid sequences "5" or "6" in Table 1 ofBucht and Hjalmarsson, 1996, Biochim Biophys Acta 1292:223-32)), CD59 (e.g.,encoded by nt 393-473 of Genbank U48255; sequences encoding amino acid sequence "20" in Table 1 of Bucht and Hjalmarsson, 1996, Biochim Biophys Acta 1292:223-32; sequences encoding amino acids 74-101 of Figure 2 of Powell et al, 1997, J Immunol 158:1692-1702), T-cadherin (e.g., sequences encoding the 76 C-terminal amino acids of chick T cadherin as described by Koller and Ranscht, 1996, J Biol Chem 271:30061-7), aminopeptidase P (e.g., sequences encoding amino acids 649-673 of NCBI Entrez protein database accession # 1517942, Hyde et al, 1996, Biochem J 319:197-201), carboxypeptidase M, CD16B, Thy 1, carbonic anhydrase IV (e.g., sequences encoding amino acids 284-312 of NCBI Entrez protein database accession # 179791, Okuyama et al, 1995, Arch Biochem Biophys 320:315-22), placental alkaline phosphatase (e.g., sequences encoding amino acids 498-529 of NCBI Entrez protein database accession # 178464, Oda et al, 1994, Biochem J 301:577-83), neuronal glycoprotein F3, carcinoembryonic antigen (e.g., sequences encoding amino acid sequence "28" in Table 1 of Bucht and Hjalmarsson, 1996, Biochim Biophys Acta 1292:223-32), MRC-OX45 (e.g., sequences encoding amino acid sequence "2" in Table 1 of Bucht and Hjalmarsson, 1996, Biochim Biophys Acta 1292:223-32), RT 6.2 (e.g., sequences encoding amino acid sequence "3" in Table 1 of Bucht and Hjalmarsson, 1996, Biochim Biophys Acta 1292:223-32), D. discoideum prespore-specific antigen (e.g., sequences encoding amino acid sequence "4" in Table 1 of Bucht and Hjalmarsson, 1996, Biochim Biophys Acta 1292:223-32), microsomal dipeptidase (e.g., sequences encoding amino acid sequence "8" in Table 1 of Bucht and Hjalmarsson, 1996, Biochim Biophys Acta 1292:223-32), CAMPATH-1 (e.g., sequences encoding amino acid sequence "9" in Table 1 of Bucht and Hjalmarsson, 1996, Biochim Biophys Acta 1292:223-32), T. brucei PARP (e.g., sequences encoding amino acid sequence "10" in Table 1 of Bucht and Hjalmarsson, 1996, Biochim Biophys Acta 1292:223-32), T. brucei VSG Mit 118a (e.g., sequences encoding amino acid sequence "11" in Table 1 of Bucht and Hjalmarsson, 1996, Biochim Biophys Acta 1292:223-32), T. brucei VSG Mit 117a (e.g., sequences encoding amino acid sequence "12" in Table 1 ofBucht and Hjalmarsson, 1996, Biochim Biophys Acta 1292:223-32), T. brucei VSG MITat 1.1000 BC (e.g., sequences encoding amino acid sequence "13" in Table 1 of Bucht and Hjalmarsson, 1996, Biochim Biophys Acta 1292:223-32), T. brucei VSG MITat 1.5b (e.g., sequences encoding amino acid sequence "14" in Table 1 of Bucht and Hjalmarsson, 1996, Biochim Biophys Acta 1292:223-32), T. brucei VSG ILTat 1.1 (e.g., sequences encoding amino acid sequence "15" in Table 1 of Bucht and Hjalmarsson, 1996, Biochim Biophys Acta 1292:223-32), T. brucei VSG TxTat 1 (e.g., sequences encoding amino acid sequence "16" in Table 1 of Bucht and Hjalmarsson, 1996, Biochim Biophys Acta 1292:223-32), T. brucei VSG Mit 221 (e.g., sequences encoding amino acid sequence "17" in Table 1 of Bucht and Hjalmarsson, 1996, Biochim Biophys Acta 1292:223-32), prion proteins (e.g., sequences encoding amino acid sequence "18" in Table I of Bucht and Hjalmarsson, 1996, Biochim Biophys Acta 1292:223-32), urokinase receptor (e.g., sequences encoding amino acid sequence "21" in Table 1 of Bucht and Hjalmarsson, 1996, Biochim Biophys Acta 1292:223-32), T. congolense VSG YNat 1.1 (e.g., sequences encoding amino acid sequence "23" in Table 1 of Bucht and Hjalmarsson, 1996, Biochim Biophys Acta 1292:223-32), S. cerevesiae GAS-1 (e.g., sequences encoding amino acid sequence "24" in Table 1 of Bucht and Hjalmarsson, 1996, Biochim Biophys Acta 1292:223-32), Thy-1 (e.g., sequences encoding amino acid sequences "25" or "26" in Table 1 of Bucht and Hjalmarsson, 1996, Biochim Biophys Acta 1292:223-32), L. major PSP (e.g., sequences encoding amino acid sequence "29" in Table 1 of Bucht and Hjalmarsson, 1996, Biochim Biophys Acta 1292:223-32), D. discoideum contact site A glycoprotein (e.g., sequences encoding the 25 C-terminal amino acids as described in Barth et al, 1996, Biochem J 317:533-40)CD24, and synthetic sequences (e.g.,as described by Coyne et al, 1993, J Biol Chem 268:6689-93).

GPI-linked polypeptides can be extracted from cells using the following method. 5 x 10⁶ cells are spun down and frozen at -80° C. The pellet is thawed in 14 ml of 0.15M NaCl/10mM Tris 7.4/0.1mM primaquine/2% Trito X-114 with stirring at 0° C for 1h, then centrifuged at 8800g at 0° C for 10 min. The supernatant is maintained at -20° C overnight, thawed at room temperature, and then placed at 32° C for 12 min. It is then centrifuged at 3000g for 3 min at 32° C. The top layer is decanted and 11 ml of cold Buffer A (0.15M NaCl/10mM Tris 7.4/0.1mM primaquine/0.06% Triton X-114) is added to the bottom layer. This is incubated on ice for 10 min. The 12 min 32° C incubation, 32° C 3000g centrifugation, decanting of top layer, and addition of 11 ml cold Buffer A to bottom layer are repeated. The solution is centrifuged at 18000g for 10 min at 0° C. The 12 min 32° C incubation, 32° C 3000g centrifugation, and decanting of top layer are repeated. 3 vol of cold acetone are added to the final bottom phase. The solution is centrifuged at 12, 000 RPM for 30 min, the supernatant removed, and the protein pellet containing the GPI fraction dried under vacuum. Specific proteins can be purified by methods well-known to those skilled in the art, e.g.,immunoaffinity purification.

Another method of producing a lipid-linked engineered cytokine, or opsonin, is to chemically link the polypeptide to a fatty acid such as palmitate. 1.5 mg/ml of the polypeptide is suspended in PBS, pH 7.8, containing 0.3% deeoxycholic acid, 0.1% sodium bicarbonate, and 0.1% sodium azide. The optimal final pH of the solution is 7.6-8.0. The mixture is warmed to 37°C and the N-hydroxysuccinimide ester of palmitic acid (Research Organics, Cleveland, OH) is added to a final concentration of 0.1 mg/ml. The solution is incubated overnight at room temperature. The polypeptide is purified by passage through a 16 x 250 mm Sephadex G-75 chromatography column equilibrated with 0.15% deoxycholic acid in PBS, pH 7.6.

### CROSSLINKING MOIETIES USEFUL ACCORDING TO THE INVENTION

Another convenient method of linking a cytokine, or an opsonin to a cell or a cell-like structure is to use a crosslinking agent. A "crosslinking agent" is a chemical entity that can react with functional groups on at least two other molecules, e.g.,two polypeptides or a polypeptide and a lipid, such that upon reaction with the crosslinking agent the two molecules become covalently linked.

A wide variety of crosslinking agents, both bifunctional and polyfunctional, are known in the art and are commercially available, e.g.,from Sigma (St. Louis, MO). These include, for example, S-acetylmercaptosuccinic anhydride, S-acetylthioglycolic acid N-hydroxysuccinimide ester, S-acetylthiopropionic acid N-hydroxysuccinimide ester, adipic acid dihydrazide, 4-azidobenzoic acid N-hydroxysuccinimide ester, N-(5-azido-2-nitrobenzyloxy)succinimide, 6-(4-azido-2-nitrophenylamino)hexanoic acid N-hydroxysuccinimide ester, p-azidophenacyl bromide, N-(4-azidophenylthio)phthalimide, 4-azidosalicylic acid N-hydroxysuccinimide ester, bromoacetic acid N-hydroxysuccinimide ester, 1,4-butanediol diglycidyl ether, carbonyl-bis(L-methionine p-nitrophenyl ester), 2-diazo-3,3,3-trifluoropropionic acid p-nitrophenyl ester, diethyl malonimidate, 1, 5-difluoro-2, 4-dinitrobenzene, 4, 4'-diisothiocyanatostilbene-2, 2'-disulfonic acid, dimethyl adipimidate, dimethyl 3, 3'-dithiobispropionimidate, dimethyl pimelimidate, dimethyl suberimidate, 4, 4'-dithiobisphenyl azide, dithiobis(propionic acid N-hydroxysuccinimide ester), ethylene glycol bis-(succinic acid N-hydroxysuccinimide ester), 4-fluoro-3-nitrophenyl azide, bis-(4-fluoro-3-nitrophenyl) sulfone, p-formylbenzoic acid N-hydroxysuccinimide ester, glutaraldehyde, 2-iminothiolane, 6-(iodoacetamido)caproic acid N-hydroxysuccinimide ester, iodoacetic acid N-hydroxysuccinimide ester, 3-malemidoacetic acid N-hydroxysuccinimide ester, 3-malemidobenzoic acid N-hydroxysuccinimide ester, 4-(N-malemido)benzophenone, gamma-malemidobutyric acid N-hydroxysuccinimide ester, epsilon-malemidocaproic acid N-hydroxysuccinimide ester, 4-(N-malemidomethyl)cyclohexanecarboxylic acid N-hydroxysuccinimide ester, 4-(N-malemidomethyl)cyclohexanecarboxylic acid 3-sulfo- N-hydroxysuccinimide ester, beta-malemidopropionic acid N-hydroxysuccinimide ester, N,N'-bis(3-malemidopropionyl)-2-hydroxy-1, 3-propanediamine, 1, 4-phenylene diisothiocyanate, N, N'-o-phenylene dimalemide, N, N'-p-phenylene dimalemide, polyoxyethylene bis(glycidyl ether), bis(polyoxyethylene bis(glycidyl ether)), polyoxyethylene bis(imidazolylcarbonyl), bis(polyoxyethylene bis(imidazolylcarbonyl)), polyoxyethylene bis(p-nitrophenyl carbonate), 3-(2-pyridyldithio)propionic acid N-hydroxysuccinimide ester, suberic acid bis(N-hydroxysuccinimide) ester, succinic acid malemidoethyl N-hydroxysuccinimide ester, 1, 5 bis(succinimidooxycarbonyloxy)-pentane, and bis(N-succinimidyl) carbonate.

### DETERMINATION OF BINDING OF GPI-LINKED OPSONIN, OR CYTOKINE, TO CELLS ACCORDING TO THE INVENTION

In certain embodiments of the invention, an opsonin, or cytokine, is engineered so as to contain GPI or a moiety of GPI which contains a lipid and thus permits binding of the modified polypeptide to a cell via intercalation of the lipid group into the plasma membrane of the cell. For those GPI moieties which consist of more or less than conventional GPI, one of skill in the art can easily determine whether such a moiety will permit binding of the polypeptide to the cell membrane. The following assay is useful for determining whether a GPI-linked molecule is able to bind to a cell.

To quantitate incorporation, GPI-linked polypeptide is first labeled with ¹²⁵I. Sephadex G-25 column is loaded with 0.1mg of the relevant protein in 1 ml PBS, followed by 20-30 ml PBS. 2 mCi Na[¹²⁵I] and 20 ul of 10 uM lactoperoxidase in PBS are added to 2 ml of a 1 mg/ml solution of relevant protein in PBS. 4 ul of 0.03% hydrogen peroxide in 0.025 M phosphate buffer, pH 7.4, is stirred into the protein solution. Addition of hydrogen peroxide is repeated three more times at 1 min intervals. 1 ml of 15 nM NaI in PBS is added. The solution is layered onto the Sephadex column and eluted with 20 ml PBS. The eluant is monitored by Geiger counting, and the first peak of radioactivity, which contains the labeled protein, is collected. The specific activity is determined by gamma counting (assuming 100% recovery). The protein solution is diluted to 40 ug/ml and used for the plasma membrane incorporation procedure described hereinbelow. After washing, 1 ul aliquots of cells are gamma counted. To determine whether association is via the GPI moiety, cells are incubated with B. Thuringiensis phosphatidylinositol-specific phospholipase C (Sigma) at 37°C for 3 hr, centrifuged, and washed prior to counting. This releases GPI-linked proteins from the cell surface.

### ANTIGENS USEFUL ACCORDING TO THE INVENTION

### 1. Viral Antigens

Examples of viral antigens include, but are not limited to, retroviral antigens such as retroviral antigens from the human immunodeficiency virus (HIV) antigens such as gene products of the *gag, pol,* and *env* genes, the Nef protein, reverse transcriptase, and other HIV components; hepatitis viral antigens such as the S. M, and L proteins of hepatitis B virus, the pre-S antigen of hepatitis B virus, and other hepatitis, e.g., hepatitis A, B. and C, viral components such as hepatitis C viral RNA; influenza viral antigens such as hemagglutinin and neuraminidase and other influenza viral components; measles viral antigens such as the measles virus fusion protein and other measles virus components; rubella viral antigens such as proteins E1 and E2 and other rubella virus components; rotaviral antigens such as VP7sc and other rotaviral components; cytomegaloviral antigens such as envelope glycoprotein B and other cytomegaloviral antigen components; respiratory syncytial viral antigens such as the RSV fusion protein, the M2 protein and other respiratory syncytial viral antigen components; herpes simplex viral antigens such as immediate early proteins, glycoprotein D, and other herpes simplex viral antigen components; varicella zoster viral antigens such as gpI, gpII, and other varicella zoster viral antigen components; Japanese encephalitis viral antigens such as proteins E, M-E, M-E-NS 1, NS 1, NS 1 -NS2A, 80%E, and other Japanese encephalitis viral antigen components; rabies viral antigens such as rabies glycoprotein, rabies nucleoprotein and other rabies viral antigen components. See Fundamental Virology, Second Edition, e's. Fields, B.N. and Knipe, D.M. (Raven Press, New York, 1991) for additional examples of viral antigens.

### 2. Bacterial antigens

Bacterial antigens which can be used in the compositions and methods of the invention include, but are not limited to, pertussis bacterial antigens such as pertussis toxin, filamentous hemagglutinin, pertactin, FIM2, FIM3, adenylate cyclase and other pertussis bacterial antigen components; diptheria bacterial antigens such as diptheria toxin or toxoid and other diphtheria bacterial antigen components; tetanus bacterial antigens such as tetanus toxin or toxoid and other tetanus bacterial antigen components; streptococcal bacterial antigens such as M proteins and other streptococcal bacterial antigen components; gram- negative bacilli bacterial antigens such as lipopolysaccharides and other gram-negative bacterial antigen components; Mycobacterium tuberculosis bacterial antigens such as mycolic acid, heat shock protein 65 (HSP65), the 30kDa major secreted protein, antigen 85A and other mycobacterial antigen components; Helicobacter pylori bacterial antigen components; pneumococcal bacterial antigens such as pneumolysin, pneumococcal capsular polysaccharides and other pneumococcal bacterial antigen components; hemophilus influenza bacterial antigens such as capsular polysaccharides and other hemophilus influenza bacterial antigen components; anthrax bacterial antigens such as anthrax protective antigen and other anthrax bacterial antigen components; rickettsiae bacterial antigens such as romps and other rickettsiae bacterial antigen component. Also included with the bacterial antigens described herein are any other bacterial, mycobacterial, mycoplasmal, rickettsial, or chlamydial antigens.

### 3. Fungal antigens

Fungal antigens which can be used in the compositions and methods of the invention include, but are not limited to, candida fungal antigen components; histoplasma fungal antigens such as heat shock protein 60 (HSP60) and other histoplasma fungal antigen components; cryptococcal fungal antigens such as capsular polysaccharides and other cryptococcal fungal antigen components; coccidiodes fungal antigens such as spherule antigens and other coccidiodes fungal antigen components; and tinea fungal antigens such as trichophytin and other coccidiodes fungal antigen components.

### 4. Parasite antigens

Examples of protozoa and other parasitic antigens include, but are not limited to, plasmodium falciparum antigens such as merozoite surface antigens, sporozoite surface antigens, circumsporozoite antigens, gametocyte/gamete surface antigens, blood-stage antigen pf 1 55/RESA and other plasmodial antigen components; toxoplasma antigens such as SAG-1, p30 and other toxoplasma antigen components; schistosomae antigens such as glutathione-S-transferase, paramyosin, and other schistosomal antigen components; leishmania major and other leishmaniae antigens such as gp63, lipophosphoglycan and its associated protein and other leishmanial antigen components; and trypanosoma cruzi antigens such as the 75-77kDa antigen, the 56kDa antigen and other trypanosomal antigen components.

### 5. Tumor antigens.

Tumor antigens which can be used in the compositions and methods of the invention include, but are not limited to, telomerase components; multidrug resistance proteins such as P-glycoprotein; MAGE-1, alpha fetoprotein, carcinoembryonic antigen, mutant p53, papillomavirus antigens, gangliosides or other carbohydrate-containing components of melanoma or other tumor cells. It is contemplated by the invention that antigens from any type of tumor cell can be used in the compositions and methods described herein.

### 6. Antigens relating to autoimmunity.

Antigens involved in autoimmune diseases, allergy, and graft rejection can be used in the compositions and methods of the invention. For example, an antigen involved in any one or more of the following autoimmune diseases or disorders can be used in the present invention: diabetes mellitus, arthritis (including rheumatoid arthritis, juvenile rheumatoid arthritis, osteoarthritis, psoriatic arthritis), multiple sclerosis, myasthenia gravis, systemic lupus erythematosis, autoimmune thyroiditis, dermatitis (including atopic dermatitis and eczematous dermatitis), psoriasis, Sjogren's Syndrome, including keratoconjunctivitis sicca secondary to Sjögren's Syndrome, alopecia areata, allergic responses due to arthropod bite reactions, Crohn's disease, aphthous ulcer, iritis, conjunctivitis, keratoconjunctivitis, ulcerative colitis, asthma, allergic asthma, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Crohn's disease, Graves ophthalmopathy, sarcoidosis, primary biliary cirrhosis, uveitis posterior, and interstitial lung fibrosis. Examples of antigens involved in autoimmune disease include glutamic acid decarboxylase 65 (GAD 65), native DNA, myelin basic protein, myelin proteolipid protein, acetylcholine receptor components, thyroglobulin, and the thyroid stimulating hormone (TSH) receptor. Examples of antigens involved in allergy include pollen antigens such as Japanese cedar pollen antigens, ragweed pollen antigens, rye grass pollen antigens, animal derived antigens such as dust mite antigens and feline antigens, histocompatiblity antigens, and penicillin and other therapeutic drugs. Examples of antigens involved in graft rejection include antigenic components of the graft to be transplanted into the graft recipient such as heart, lung, liver, pancreas, kidney, and neural graft components. An antigen can also be an altered peptide ligand useful in treating an autoimmune disease.

Examples of miscellaneous antigens which can be can be used in the compositions and methods of the invention include endogenous hormones such as luteinizing hormone, follicular stimulating hormone, testosterone, growth hormone, prolactin, and other hormones, drugs of addiction such as cocaine and heroin, and idiotypic fragments of antigen receptors such as Fab-containing portions of an anti-leptin receptor antibody.

### PREPARATION OF A CELL CONTAINING A RECOMBINANT NUCLEIC ACID ACCORDING TO THE INVENTION

Cells are transfected, as taught herein, via conventional methods well-known in the art. Suitable methods for transforming or transfecting host cells can be found in Sambrook et al. (Molecular Cloning: A Laboratory Manual, 2nd Edition, Cold Spring Harbor Laboratory press (1989)), and other laboratory manuals. Additional examples of methods of introducing nucleic acid molecules encoding ligands for CD40, cytokines, or opsonins are described below. The cells containing the introduced nucleic acid molecules encoding, for example, an opsonin, a cytokine, a ligand for CD40, and/or an antigen, can themselves be administered to a subject (as the antigen) according to the methods of the invention, e.g., in a vaccine composition.

### A. Introduction of Naked Nucleic Acid into Cells

1. *Transfection mediated by DEAE-dextran*: Naked nucleic acid can be introduced into cells by forming a mixture of the nucleic acid and DEAE-dextran and incubating the mixture with the cells. A dimethylsulfoxide or chloroquine shock step can be added to increase the amount of nucleic acid uptake. DEAE-dextran transfection is only applicable to *in vitro* modification of cells and can be used to introduce nucleic acid transiently into cells but is not preferred for creating stably transfected cells. Thus, this method can be used for short term production of a gene product but is not a method of choice for long-term production of a gene product. Protocols for DEAE-dextran-mediated transfection can be found in Current Protocols in Molecular Biology, Ausubel, F.M. et al. (e's.) Greene Publishing Associates, (1989), Section 9.2 and in Molecular Cloning: A Laboratory Manual. 2nd Edition. Sambrook et al. Cold Spring Harbor Laboratory Press, (1989), Sections 16.41-16.46 or other standard laboratory manuals.
*2. Electroporation:* Naked nucleic acid can also be introduced into cells by incubating the cells and the nucleic acid together in an appropriate buffer and subjecting the cells to a high-voltage electric pulse. The efficiency with which nucleic acid is introduced into cells by electroporation is influenced by the strength of the applied field, the length of the electric pulse, the temperature, the conformation and concentration of the nucleic acid and the ionic composition of the media. Electroporation can be used to stably (or transiently) transfect a wide variety of cell types and is only applicable to *in vitro* modification of cells. Protocols for electroporating cells can be found in Current Protocols in Molecular Biology, Ausubel, F.M. et al. (e's.) Greene Publishing Associates, (1989), Section 9.3 and in Molecular Cloning: A Laboratory Manual, 2nd Edition, Sambrook et al. Cold Spring Harbor Laboratory Press, (1989), Sections 16.54-16.55 or other standard laboratory manuals.
*3. Liposome-mediated transfection ("lipofection')*: Naked nucleic acid can be introduced into cells by mixing the nucleic acid with a liposome suspension containing cationic lipids. The nucleic acid/liposome complex is then incubated with cells. Liposome mediated transfection can be used to stably (or transiently) transfect cells in culture *in vitro.* Protocols can be found in Current Protocols in Molecular Biology, Ausubel, F.M. et al. (e's.) Greene Publishing Associates, (1989), Section 9.4 and other standard laboratory manuals. Additionally, gene delivery in vivo has been accomplished using liposomes. See for example Nicolau et al. (1987) Meth. Enz. 149:157-176; Wang and Huang (1987) Proc. Natl. Acad Sci. SA 84:7851-785S; Brigham et al. (1989) Am. J. Med. Sci. 298:278; and Gould-Fogerite et al. (1989) Gene 84:429-438.
*4. Direct Injection:* Naked nucleic acid can be introduced into cells by directly injecting the nucleic acid into the cells. For an *in vitro* culture of cells, nucleic acid can be introduced by microinjection. Since each cell is microinjected individually, this approach is very labor intensive when modifying large numbers of cells. However, a situation wherein microinjection is a method of choice is in the production of transgenic animals (discussed in greater detail below). In this situation, the nucleic acid is stably introduced into a fertilized oocyte which is then allowed to develop into an animal. The resultant animal contains cells carrying the nucleic acid introduced into the oocyte. Direct injection has also been used to introduce naked nucleic acid into cells *in vivo* (see e.g., Acsadi et al. (1991) Nature 332: 815-818; Wolff et al. (1990) Science 247:1465-1468). A delivery apparatus (e.g., a "gene gun") for injecting DNA into cells *in vivo* can be used. Such an apparatus is commercially available (e.g., from BioRad).
*5. Receptor-Mediated DNA Uptake:* Naked nucleic acid can also be introduced into cells by complexing the nucleic acid to a cation, such as polylysine, which is coupled to a ligand for a cell-surface receptor (see for example Wu, G. and Wu, C.H. (1988) J. Biol. Chem 263:14621; Wilson et al. (1992) J. Biol. Chem. 267:963-967; and U.S. Patent No. 5,166,320). Binding of the nucleic acid-ligand complex to the receptor facilitates uptake of the nucleic acid by receptor-mediated endocytosis. Receptors to which a nucleic acid-ligand complex have targeted include the transferrin receptor and the asialoglycoprotein receptor. A nucleic acid-ligand complex linked to adenovirus capsids which naturally disrupt endosomes, thereby releasing material into the cytoplasm can be used to avoid degradation of the complex by intracellular lysosomes (see for example Curiel et al. (1991) Proc. Natl. Acad. Sci. USA 88:8850; Cristiano et al. (1993) Proc. Natl. Acad. Sci USA 90:2122-2126). Receptor-mediated nucleic acid uptake can be used to introduce nucleic acid into cells either *in vitro* or in vivo and, additionally, has the added feature that nucleic acid can be selectively targeted to a particular cell type by use of a ligand which binds to a receptor selectively expressed on a target cell of interest.

Generally, when naked nucleic acid is introduced into cells in culture (e.g., by one of the transfection techniques described above) only a small fraction of cells (about 1 out of 10⁵) typically integrate the transfected nucleic acid into their genomes (i.e., the nucleic acid is maintained in the cell episomally). Thus, in order to identify cells which have taken up exogenous nucleic acid, it is advantageous to transfect nucleic acid encoding a selectable marker into the cell along with the nucleic acid(s) of interest. Preferred selectable markers include those which confer resistance to drugs such as G418, hygromycin and methotrexate. Selectable markers may be introduced on the same plasmid as the gene(s) of interest or may be introduced on a separate plasmid.

### B. Viral-Mediated Gene Transfer

A preferred approach for introducing nucleic acid encoding a gene product into a cell is by use of a viral vector containing nucleic acid, e.g.,a cDNA, encoding the gene product. Infection of cells with a viral vector has the advantage that a large proportion of cells receive the nucleic acid, which can obviate the need for selection of cells which have received the nucleic acid. Additionally, molecules encoded within the viral vector, e.g., by a cDNA contained in the viral vector, are expressed efficiently in cells which have taken up viral vector nucleic acid and viral vector systems can be used either in vitro or in vivo.
*1. Retroviruses:* Defective retroviruses are well characterized for use in gene transfer for gene therapy purposes (for a review see Miller, A.D. (1990) Blood 76:271). A recombinant retrovirus can be constructed having a nucleic acid encoding a gene product of interest inserted into the retroviral genome. Additionally, portions of the retroviral genome can be removed to render the retrovirus replication defective. The replication defective retrovirus is then packaged into virions which can be used to infect a target cell through the use of a helper virus by standard techniques. Protocols for producing recombinant retroviruses and for infecting cells *in vitro* or *in vivo* with such viruses can be found in Current Protocols in Molecular Biology, Ausubel, F.M. et al. (eds.) Greene Publishing Associates, (1989), Sections 9.10-9.14 and other standard laboratory manuals. Examples of suitable retroviruses include pLJ, pZIP, pWE and pEM which are well known to those skilled in the art. Examples of suitable packaging virus lines include ϕCrip, ϕCre,_2, and_Am. Retroviruses have been used to introduce a variety of genes into many different cell types, including epithelial cells, endothelial cells, lymphocytes, myoblasts, hepatocytes, bone marrow cells, *in vitro* and/or *in vivo* (see for example Eglitis, et al. (1985) Science 230:1395-1398; Danos and Mulligan (1988) Proc. Natl. Acad. Sci. USA 85:6460-6464; Wilson et al. (1988) Proc. Natl. Acad. Sci. USA 85:3014-3018; Armentano et al. (1990) Proc. Natl. Acad. Sci. USA 87:6141-6145; Huber et al. (1991) Proc. Natl. Acad. Sci. USA 88:8039-8043; Ferry et al. (1991) Proc. Natl. Acad Sci. USA 88:8377-8381; Chowdhury et al. (1991) Science 254:1802-1805; van Beusechem et al. (1992) Proc. Natl. Acad Sci. USA 89:7640-7644; Kay et al. (1992) Human Gene Therapy 3:641-647; Dai et al. (1992) Proc. Natl. Acad Sci. USA 89:10892-10895; Hwu et al. (1993) J. Immunol. 150:4104-115; U.S. Patent No. 4,868,116; U.S. Patent No. 4,980,286; PCT Application WO 89/07136; PCT Application WO 89/02468; PCT Application WO 89/05345; and PCT Application WO 92/07573). Retroviral vectors require target cell division in order for the retroviral genome (and foreign nucleic acid inserted into it) to be integrated into the host genome to stably introduce nucleic acid into the cell. Thus, it may be necessary to stimulate replication of the target cell.
*2. Adenoviruses:* The genome of an adenovirus can be manipulated such that it encodes and expresses a gene product of interest but is inactivated in terms of its ability to replicate in a normal lytic viral life cycle. See for example Berkner et al. (1988) BioTechniques 6:616; Rosenfeld et al. (1991) Science 252:431-434; and Rosenfeld et al. (1992) Cell 68:143-155. Suitable adenoviral vectors derived from the adenovirus strain Ad type 5 d1324 or other strains of adenovirus (e.g., Adz, Ad3, Ad7 etc.) are well known to those skilled in the art. Recombinant adenoviruses are advantageous in that they do not require dividing cells to be effective gene delivery vehicles and can be used to infect a wide variety of cell types, including airway epithelium (Rosenfeld et al. (1992) cited *supra*), endothelial cells (Lemarchand et al. (1992) Proc. Natl. Acad. Sci. USA 89:6482-6486), hepatocytes (Herz and Gerard (1993) Proc. Natl. Acad. Sci. USA 90:2812-2816) and muscle cells (Quantin et al. (1992) Proc. Natl. Acad. Sci. USA 89:2581-2584). Additionally, introduced adenoviral nucleic acid (and foreign DNA contained therein) is not integrated into the genome of a host cell but remains episomal, thereby avoiding potential problems that can occur as a result of insertional mutagenesis in situations where introduced nucleic acid becomes integrated into the host genome (e.g., retroviral DNA). Moreover, the carrying capacity of the adenoviral genome for foreign DNA is large (up to 8 kilobases) relative to other gene delivery vectors (Berkner et al. cited supra; Haj-Ahmand and Graham (1986) J. Virol. 57:267). Most replication-defective adenoviral vectors currently in use are deleted for all or parts of the viral E1 and E3 genes but retain as much as 80 % of the adenoviral genetic material.
*3. Adeno-Associated Viruses:* Adeno-associated virus (AAV) is a naturally occurring defective virus that requires another virus, such as an adenovirus or a herpes virus, as a helper virus for efficient replication and a productive life cycle. (For a review see Muzyczka et al. Curr. Topics in Micro. and Immunol. (1992) 158:97-129). It is also one of the few viruses that may integrate its DNA into non-dividing cells, and exhibits a high frequency of stable integration (see for example Flotte et al. (1992) Am. J. Respir. Cell. Mol. Biol. 7:349-356; Samulski et al. (1989) J. Virol. 63:3822-3828; and McLaughlin et al. (1989) J. Virol 62:1963-1973). Vectors containing as little as 300 base pairs of AAV can be packaged and can integrate. Space for exogenous nucleic acid is limited to about 4.5 kb. An AAV vector such as that described in Tratschin et al. (1985) Mol. Cell. Biol. 5:3251-3260 can be used to introduce nucleic acid into cells. A variety of nucleic acids have been introduced into different cell types using AAV vectors (see for example Hermonat et al. (1984) Proc. Natl. Acad. Sci. USA 81 :6466-6470; Tratschin et al. (1985) Mol. Cell. Biol. 4:2072-2081; Wondisford et al. (1988) Mol. Endocrinol. 2:32-39; Tratschin et al. (1984) J. Virol.51:611 - 619; and Flotte et al. (1993) J. Biol. Chem. 268:3781-3790).

The efficacy of a particular expression vector system and method of introducing nucleic acid into a cell can be assessed by standard approaches routinely used in the art. For example, nucleic acid introduced into a cell can be detected by a filter hybridization technique (e.g., Southern blotting) and RNA produced by transcription of introduced nucleic acid can be detected, for example, by Northern blotting, RNase protection or reverse transcriptase-polymerase chain reaction (RT-PCR). The gene product can be detected by an appropriate assay, for example by immunological detection of a produced protein, such as with a specific antibody, or by a functional assay to detect a functional activity of the gene product, such as an enzymatic assay. If the gene product of interest to be expressed by a cell is not readily assayable, an expression system can first be optimized using a reporter gene linked to the regulatory elements and vector to be used. The reporter gene encodes a gene product which is easily detectable and, thus, can be used to evaluate the efficacy of the system. Standard reporter genes used in the art include genes encoding beta-galactosidase, chloramphenicol acetyl transferase, luciferase and human growth hormone.

### CELLS USEFUL ACCORDING TO THE INVENTION

The invention provides for cytokine-coated cells. Cells useful for preparing cytokine coated cells include but are not limited to the following.

Cytokine-coated cells can be prepared from host cells according to the invention, wherein a host cell can be any cell which is able to act as a carrier for an antigen according to the invention and thus may be be a nucleated cell or a procaryotic cell into which nucleic acid can be artifically introduced. Procaryotic cells useful according to the invention include bacterial cells and yeast. Eucaryotic (nucleated) cells useful according to the invention include cells of a fungus, cells of a parasite and mammalian cells. Mammalian cells useful according to the invention include but are not limited to fibroblasts, including specialized mesenchymal cells such as a synoviocytes; keratinocytes, epithelial cells, endothelial cells, leukocytes and tumor cells.

Cell lines useful according to the invention include but are not limited to B 16, CMS-5 fibrosarcoma cells, Cos1 cells and CHO cells, TS/A, Lewis lung carcinoma, RENCA, Dunning rat prostate carcinoma, and cell lines included in the catalogue of the American Type Culture Collection (Manassas, VA).

Cytokine-coated cells can be prepared from pathogenic cells according to the invention. Pathogenic cells include tumor cells (e.g. B16 cells, CMS-5 fibrosarcoma cells, and cells derived from the tumors included in the section entitled "Tumors for which the Invention is Useful"), and cells derived from pathogenic bacterium, pathogenic fungus, pathogenic virus, pathogenic parasite, or a pathogenic arthropod.

The invention also provides for cytokine-coated cells that are unable to divide in a mammalian host.

### PREPARATION OF CELLS IN ADMIXTURE WITH A CYTOKINE, (AND OPTIONALLY AN OPSONIN,) ACCORDING TO THE INVENTION

The invention also contemplates a preparation of cells that is admixed with a cytokine and optionally an opsonin. Therefore, the cell may already express an antigen, for example, a tumor cell antigen (either an endogenously expressed antigen or a heterologous antigen), and may be mixed with an engineered cytokine, as defined hereinabove, and the preparation considered to be "cytokine-coated cells", according to the invention. In this mixture, the preparation will consist of about 10⁴ - 10⁸ cells mixed with 1ug-100ug/ml ligand in a conventional physiological salt buffer.

Where the invention encompasses a preparation of cells in admixture with a cytokine, and optionally an opsonin, the cytokine, (and optionally an opsonin,) is first prepared according to conventional procedures and then mixed with the cells. The cytokine, (and optionally an opsonin,) may be prepared via recombinant DNA techniques via transfection of a host cell strain or line and isolation of the recombinant protein.

A host cell of the invention, such as a eukaryotic host cell in culture, can be used to produce (i.e., express) polypeptides of the invention. For example, a transfected host cell (into which a recombinant expression vector encoding a polypeptide of the invention has been introduced) may be cultured in a suitable medium until the polypeptide is produced, and isolated from the medium or the host cell.

*Transfection mediated by CaPO4*: Naked nucleic acid can be introduced into cells by forming a precipitate containing the nucleic acid and calcium phosphate. For example, a HEPES-buffered saline solution can be mixed with a solution containing calcium chloride and nucleic acid to form a precipitate and the precipitate is then incubated with cells. A glycerol or dimethyl sulfoxide shock step can be added to increase the amount of nucleic acid taken up by certain cells. CaPO4-mediated transfection can be used to stably (or transiently) transfect cells and is only applicable to *in vitro* modification of cells. Protocols for CaPO4-mediated transfection can be found in Current Protocols in Molecular Biology, Ausubel, F.M. et al. (eds.) Greene Publishing Associates, (1989), Section 9.1 and in Molecular Cloning: A Laboratory Manual. 2nd Edition. Sambrook et al. Cold Spring Harbor Laboratory Press, (1989), Sections 16.32-16.40 or other standard laboratory manuals.

### METHODS OF DETECTING EXPRESSION FROM AN ARTIFICIALLY INTRODUCED RECOMBINANT NUCLEIC ACID SEQUENCE

Methods of detecting a protein (e.g., cytokine or an opsonin) that is expressed from a recombinant nucleic acid molecule that has been artificially introduced into a cell are provided.

### Preparation of Antibodies

Antibodies specific for a protein (e.g., an opsonin) are useful for protein purification, and for the detection of expression of these proteins from cells into which a recombinant nucleic acid molecule expressing these proteins has been artificially introduced. By antibody, we include constructions using the binding (variable) region of such an antibody, and other antibody modifications. Thus, an antibody useful in the invention may comprise a whole antibody, an antibody fragment, a polyfunctional antibody aggregate, or in general a substance comprising one or more specific binding sites from an antibody. The antibody fragment may be a fragment such as an Fv, Fab or F(ab')₂ fragment or a derivative thereof, such as a single chain Fv fragment. The antibody or antibody fragment may be non-recombinant, recombinant or humanized. The antibody may be of an immunoglobulin isotype, e.g., IgG, IgM, and so forth. In addition, an aggregate, polymer, derivative and conjugate of an immunoglobulin or a fragment thereof can be used where appropriate.

Although a protein product (or fragment or oligopeptide thereof) of a protein (e.g., an opsonin) that is useful for the production of antibodies does not require biological activity, it must be antigenic. Peptides used to induce specific antibodies may have an amino acid sequence consisting of at least five amino acids and preferably at least 10 amino acids. Preferably, they should be identical to a region of the natural protein and may contain the entire amino acid sequence of a small, naturally occurring molecule. Short stretches of amino acids corresponding to the protein product of a recombinant nucleic acid encoding a protein (e.g.,an opsonin) may be fused with amino acids from another protein such as keyhole limpet hemocyanin or GST, and antibody will be produced against the chimeric molecule. Procedures well known in the art can be used for the production of antibodies to the protein products of recombinant nucleic acids.

For the production of antibodies, various hosts including goats, rabbits, rats, mice etc... may be immunized by injection with the protein products (or any portion, fragment, or oligonucleotide thereof which retains immunogenic properties) of the recombinant nucleic acid molecules encoding proteins (e.g.,an opsonin ). Depending on the host species, various adjuvants may be used to increase the immunological response. Such adjuvants include but are not limited to Freund's, mineral gels such as aluminum hydroxide, and surface active substances such as lysolecithin, pluronic polyols, polyanions, peptides, oil emulsions, keyhole limpet hemocyanin, and dinitrophenol. BCG (bacilli Calmette-Guerin) and Corynebacterium parvum are potentially useful human adjuvants.

### I. Polyclonal antibodies.

The antigen protein may be conjugated to a conventional carrier in order to increase its immunogenicity, and an antiserum to the peptide-carrier conjugate will be raised. Coupling of a peptide to a carrier protein and immunizations may be performed as described (Dymecki et al., 1992, J. Biol. Chem., 267: 4815). The serum can be titered against protein antigen by ELISA (below) or alternatively by dot or spot blotting (Boersma and Van Leeuwen, 1994, J. Neurosci. Methods, 51: 317). At the same time, the antiserum may be used in tissue sections prepared as described. A useful serum will react strongly with the appropriate peptides by ELISA, for example, following the procedures of Green et al., 1982, Cell, 28: 477.

### 2. Monoclonal antibodies.

Techniques for preparing monoclonal antibodies are well known, and monoclonal antibodies may be prepared using a candidate antigen whose level is to be measured or which is to be either inactivated or affinity-purified, preferably bound to a carrier, as described by Arnheiter et al., 1981, Nature, 294;278.

Monoclonal antibodies are typically obtained from hybridoma tissue cultures or from ascites fluid obtained from animals into which the hybridoma tissue was introduced.

Monoclonal antibody-producing hybridomas (or polyclonal sera) can be screened for antibody binding to the target protein.

### 3. Antibody Detection Methods

Particularly preferred immunological tests rely on the use of either monoclonal or polyclonal antibodies and include enzyme-linked immunoassays (ELISA), immunoblotting and immunoprecipitation (see Voller, 1978, Diagnostic Horizons, 2:1, Microbiological Associates Quarterly Publication, Walkersville, MD; Voller et al., 1978, J. Clin. Pathol., 31: 507; U.S. Reissue Pat. No. 31,006; UK Patent 2,019,408; Butler, 1981, Methods Enzymol., 73: 482; Maggio, E. (ed.), 1980, Enzyme Immunoassay, CRC Press, Boca Raton, FL) or radioimmunoassays (RIA) (Weintraub, B., Principles of radioimmunoassays, Seventh Training Course on Radioligand Assay Techniques, The Endocrine Society, March 1986, pp. 1-5, 46-49 and 68-78). For analysing tissues for the presence or absence of a protein produced by a recombinant nucleic acid encoding a protein (e.g.,an opsonin), immunohistochemistry techniques may be used. It will be apparent to one skilled in the art that the antibody molecule may have to be labelled to facilitate easy detection of a target protein. Techniques for labelling antibody molecules are well known to those skilled in the art (see Harlow and Lane, 1989, Antibodies, Cold Spring Harbor Laboratory).

### DETERMINING WHETHER AN IMMUNE RESPONSE IS MODULATED ACCORDING TO THE INVENTION

Cytokine-coated cells are useful according to the invention to modulate an immune response in a mammalian, preferably a human, to an antigen or antigens contained in the cells. The cells are administered and are taken up (i.e., ingested or phagocytosed) by antigen presenting cells. Alternatively, the cells are contacted with antigen presenting cells in vitro under conditions which allow phagocytosis.

An "immune response" refers to stimulation/activation of a selected response involving the immune system, or suppression, elimination, or attenuation of a selected response. In a preferred embodiment, an immune response refers to stimulation/activation of a selected response involving the immune system by about at least 5%, or preferably between 5 and 50% or more preferably between 50 and 100% or at least 100% or greater, or suppression, elimination, or attenuation of a selected response by about at least 5%, or preferably between 5 and 50% or more preferably between 50 and 100% or at least 100% or greater, as compared to control cells that are not cytokine-coated cells. Thus, to modulate an immune response means that the desired response is more efficient, more rapid, greater in magnitude, and/or more easily induced than when cells, identical in every respect except that they are not cytokine-coated cells, are administered in an identical fashion. Different immune responses in the subject may be modulated differentially, e.g., the cellular immune response may be selectively enhanced while the humoral response may be selectively attenuated, and vice versa.

The following *in vitro* and *in vivo* assays are useful for determining whether an immune response is modulated according to the invention. The assays described in detail below measure stimulation or suppression of cellular or humoral immune responses to an antigen. The antigens referred to in the following assays are representative. It will be apparent to one of skill in the art that an immune response to a selected antigen useful according to the invention may be measured using one or more of the following assays by adapting the assay to that antigen.

### I. Detection of Increased Phagocytosis

The following assay may be used in order to determine whether opsonin-enhanced cells, or cytokine-coated cells, stimulate phagocytosis by antigen presenting cells.

Phagocytosis is examined using monocytes that have been adhered at 37° for 30 min in RPMI without added FCS. Sheep erythrocytes are incubated with an opsonin, a ligand for CD40, a cytokine, or its precursor, under conditions such that there are no more than 300 of such molecules, on average, are deposited on each erythrocyte. If a precursor is used, coated erythrocytes are then processed to convert all precursors to the actual candidate molecule (e.g., See Carlo et al., J. Immunol. 123:523-8(1979)). Fresh monocytes are isolated from the subject, and 5 x 10⁴ - 1 x 10⁵ of these cells suspended in 0.25 - 0.5 ml of RPMI medium with 1% BSA. This aliquot is placed in a tissue culture well and incubated for 30 min at 37° C. An excess of coated erythrocytes, suspended at 1.2 x 10⁸ cells/ml, is overlain on the monocytes, the plate is centrifuged for 5 min at 50g, and incubated for 30 min at 37° C. Non- ingested material is removed in two hypotonic lysis steps using ice-cold lysing buffer before fixing and staining the adherent cells, and examining the cells under light microscopy. Phagocytosis is quantified by determining the percentage of 100 monocytes ingesting one or more target cells, and the total number of ingested E/100 monocyptes (PI) is recorded. Stimulation of phagocytosis according to the invention is indicated by a phagocytic index of equal to or greater than 40.

### II. Amplification of the immune response usually involves proliferation of particular subpopulations of lymphoid cells that are normally in the resting state.

Proliferative assays have the following applications in clinical studies: (1) Assessment of overall immunologic competence of T cells or B cells as manifested in their ability to respond to polyclonal proliferation signals such as mitogens or anti-CD3 antibodies. Defects in the proliferation may be indicative of fundamental cellular immunologic defect. Low proliferation is often found as a nonspecific secondary effect of chronic disease. (2) Assessment of an individual's response to specific antigens, where low responses are indicative of general or specific immunologic defect. (3) Determination of MHC compatibility by the mixed lymphocyte reaction (MLR).

In addition, proliferative assays are useful for estimating lymphokine production, investigating signal transduction, and assessing growth factor requirements (e.g., lymphokines) for T or B cells. The procedure outlined here measures incorporation of [³H]thymidine into DNA, which usually correlates well with cell growth as measured by changes in cell number. However, when the activation stimulus is toxic, as with chemical activators such as ionomycin plus phorbol myristate acetate (PMA), the burst of new DNA synthesis following activation may not be accompanied with a net increase in viable cells, and, in fact, a decline in cell number may be observed. In this instance, [³H]thymidine incorporation in DNA is more indicative of initial cell stimulation than estimation of cell number. In addition, [³H]thymidine incorporation provides information on cell populations, not on individual cells. Alternate methods, such as flow cytometry may be used for studies requiring that type of information.

### Assay For Antigen-Induced T Cell Proliferation

This protocol is designed to test the proliferation of T cells in response to a specific antigen-- tetanus toxoid. It can be modified to test T cell proliferation in response to any protein or polysaccharide antigen. Materials: (T cell suspension, autologous antigen-presenting cell suspension (non-T cells), Tetanus toxoid solution (Connaught or State Laboratory Institute of Massachusetts)). (1) Count T cells and adjust to 1 x 10⁶ cells/ml with complete RPMI-10 AB. (2) Treat antigen- presenting cells with mitomycin C (or irradiate with 2500 rad) as in step 2 of one-way MLR protocol. Adjust concentration of antigen-presenting cells to 2 x 10⁵ cells/ml. Antigen-presenting cells can consist of autologous non-T cells or autologous monocytes/macrophages. (3) Add 100 ul T cell suspension and 50 ul antigen-presenting cell population to wells; mix just before dispensing. (4) Add 50 ul tetanus toxoid solution to give final concentrations of 0, 1, 5, 10, and 20 ug/ml. Prepare three wells for each dilution. (5) Incubate 6 days in a humidified 37°C, 5% CO₂ incubator. (6) Pulse with [³H]thymidine and harvest as described in support protocol.

### Assay For Lymphokine-Dependent Cell Proliferation

This protocol assays the lymphokine-dependent proliferation of a lymphocyte population, in this case, the IL-4 dependent proliferation of B cells. Materials: (Tonsil B cell suspension, Anti-IgM cross-linked to Sepharose beads (Bio-Rad), 10,000 U/ml human rIL-4 (Genzyme) in complete RPMI-10). (1) Count tonsil B cells and adjust concentration to 1 x 10⁶ cells/ml with complete RPMI-10. (2) Dispense 100 ul of tonsil B cells into each well. Prepare three wells for each experimental condition. (3) Dilute 10,000 U/ml rIL-4 solution 1:10, 1:100, and 1:1000. Add 20 ul of the stock or dilution to appropriate wells to yield 1000 U/ml, 100 U/ml, 10 U/ml, and 1U/ml. Include a control well with no rIL-4. (4) Pipet anti-IgM beads into appropriate wells.

Determine the optimal concentration of beads with pilot experiments. It is best to include several concentrations of beads in each experiment to "bracket" the optimal dose. Prepare wells with tonsil B cells and IL-4 dilutions alone, anti-IgM beads alone, culture medium alone, and all the combinations of IL-4 and anti-IgM bead dilutions. (5) Increase the volume of each well to 200 ul with complete RPMI-10 as necessary. (6) Culture 5 days in a humidified 37° C, 5% CO₂ incubator. (7) Pulse with [³H]thymidine and harvest as described in support protocol.

### r³H]Thymidine Pulse And Harvest Of Cell Cultures

This protocol is used in conjunction with the preceding protocols to complete the [³H] thymidine incorporation assay. (1) Add 20 ul of 50 uCi/ml [³H]thymidine to each culture (1.0 uCi) at a fixed time before terminating the culture (usually 6 or 18 hr). (2) Harvest cell cultures using an automated multiwell harvester that aspirates cells, lyses cells, and transfers DNA onto filter paper, while allowing unincorporated [³H]thymidine to wash out. Fill and aspirate each row of the microtiter plate ten times to ensure complete cell transfer and complete removal of unincorporated thymidine. Wash each filter strip with 100% ethanol to facilitate drying. Transfer to scintillation vials. For semiautomated harvester, transfer filter dots for each well into scintillation counting vials. For manual transfer, dry filters under lamp and transfer to scintillation vial with forceps. Add scintillation fluid to each vial. (3) Count samples in scintillation counter until standard deviation is less than 2%. Calculate mean cpm for background cultures and for each experimental condition. There should be less than 20% variation in replicate cultures.

### III. Induction And Measurement Of In Vitro Antibody Responses

The capacity of the human immune system to mount an antibody response *following in vivo* immunization with a protein or polysaccharide antigen is a revealing indication of the overall integrity of both the B and T cell arms of the immune system. As such, *in vivo* immunization followed by measurement of the antibody response is an appropriate test of immune function in the various acquired and congenital immunodeficiencies and in a host of other conditions affecting the immune system. The following procedures are for *in vivo* immunization and for the measurement of the subsequent immune response using an ELISA technique.

### Immuno-Enzymetric Assay For Cytokines Using NIP- And HRPO-Labeled Antibodies

This protocol describes an immunonoenzymetric assay for cytokines using a heterogeneous, noncompetitive immunoassay reaction in which the cytokine is immobilized by a coating antibody bound to a microtiter plate. Unbound material is washed free, and detection is carried out using a different anti-cytokine antibody labeled with the hapten nitroiodophenyl (NIP). This is in turn detected by a horseradish peroxidase (HRPO) conjugate of an anti-NIP antibody, which is revealed with the chromogenic substrate ABTS. In this noncompetitive immunoassay, the immunoassay signal (A₄₀₅) increases as a direct function of the amount of cytokine present in the sample. Antibodies are prepared as described in Current Protocols in Immunology, 1995, 6.20.2 - 6.20.10.

Coat assay plate. (1) Using a multichannel pipettor, transfer 100 ul of an appropriate dilution of coating antibody into all wells of the assay plate that are to be used. (2) Seal plates with microtiter plate sealer or Parafilm and incubate 2 hr. At 37°C. Prepare samples and standards in preparation plate. (3) Dilute each sample (or aliquot of conditioned medium) to be assayed with an equal volume of immunoassay diluent. (4) Pipet less than or equal to 1 ml of each diluted sample to be assayed into the upper chamber of a separate Spin-X microfiltration device. Microcentifuge 5 min. At 10,000 rpm and save the filtrates that collect in the lower chambers. (5) Add 65 ul of each diluted sample to the appropriate well of a preparation plate (i.e., a separate 96-well microtiter plate). (6) Thaw an aliquot of cytokine standard at room temperature and make sure that it is well mixed. Pipet 130 ul into the well of the preparation plate representing the highest concentration on the standard curve. Transfer 65 ul from this well into the next, then continue performing serial 1:1 dilutions in immunoassay diluent so that 65 ul of each concentration represented on the standard curve is placed in appropriate well of the preparation plate. (7) Thaw an aliquot of calibrator at room temperature (if used). Dilute with an equal volume of immunoassay diluent, then pipet 65 ul of diluted calibrator into appropriate well or wells of preparation plate.

Incubate with coating antibody. (8) Remove coated assay plate from incubator. Dip in 2-liter beaker filled with 1 x wash buffer, then invert over sink and flick to remove liquid. Repeat two more times, then bang dry on paper towel. (9) Transfer 50 ul of solution from each well of preparation plate to corresponding well of the assay plate using multichannel pipettor. (10) Seal plate with microtiter plate sealer or Parafilm and incubate 2 hr. at room temperature.

Incubate with detecting antibody. (11) Dilute NIP-labeled detecting antibody specific to cytokine of interest to 1 ug/ml in detecting buffer. (12) Wash assay plate as in step 8. (13) Add 75 ul diluted detecting antibody from step 11 to all wells of assay plate, including unused outer walls. (14) Reseal plate with microtiter plate sealer or Parafilm and incubate 1 hr. at room temperature.

Incubate with HRPO-conjugated anti-NIP antibody. (15) Dilute HRPO-conjugated anti-NIP Mab 1:3000 in detecting buffer. (16) Wash assay plate as in step 8. (17) Add 75 ul of diluted HRPO-labeled anti-NIP antibody from step 15 to all wells of assay plate. (18) Reseal plate with microtiter plate sealer or Parafilm and incubate 1 hr. at room temperature.

Incubate with chromogenic substrate. (19) Wash assay plate as in step 8. (20) Add 100 ul ABTS substrate working solutions to all wells of assay plate. Cover plate and incubate at room temperature until color development reaches desired level (generally until A₄₀₅ for wells containing the highest concentration of standard is between 1.5 and 2). This protocol usually produces an assay that can be read after 30 to 60 min.

Read plate and analyze data. (21) Using microtiter plate reader with computer interface, measure absorbance in all wells at 405 nm in single-wavelength mode or at 405 and 650 nm in dual-wavelength mode. (22) Fit standard data to a curve described by a first-degree (linear), second degree (quadratic), or four-parameter (nonlinear) mathematical function using curve-fitting software. (23) Interpolate absorbance data from unknown cytokine samples to fitted standard curve, and calculate cytokine concentrations.

### IV. Induction of an in vivo antibody response provides an approach to the evaluation of the overall integrity of the immune system.

In the protocols presented here, diptheria and tetanus toxoids are used as representative protein antigens and pneumococcal polysaccharides are used as representative polysaccharide antigens because of their safety and availability. It should be noted, however, that the responses elicited by these antigens are likely to be secondary responses because of past vaccination or natural exposure. To obtain a primary response, an unusual antigen such as keyhole limpet hemocyanin should be used.

When antigens are administered by the intramuscular or subcutaneous route, as they are here, a "systemic" immune response is induced and measurement of circulating antibody is most appropriate. It is, however, sometimes of interest to evaluate "local" or mucosal immune responses. In this case, the antigen is given either intranasally to stimulate respiratory lymphoid tissue or orally to stimulate gastrointestinal lymphoid tissue and bronchial washings or intestinal fluids, rather than blood, is assayed for antibody content; in addition, antigens are used that are more appropriate for stimulation of the local/mucosal response (i.e., influenza virus antigen for respiratory responses and cholera toxin for gastrointestinal responses).

In assaying the *in vivo* antibody response, it is important to determine responses to both protein and polysaccharide antigens because these antigens stimulate different components of the immune system. In this regard, the major antibody response to protein antigen is composed of IgG1 and IgG3 subclass antibodies, whereas the major antibody response to polysaccharide antigen is composed of IgG2 subclass antibody.

A variety of immunoassay techniques have been used to measure antibody responses in materials obtained after *in vivo* immunization. Of these, the ELISA assay is perhaps the most useful because it yields a stable, easily measurable, reproducible, and safe readout.

### Induction Of In Vivo Antibody Responses To Protein/Polysaccharide Antigens

In this protocol antigens are administered by the intramuscular or subcutaneous route and serum is collected for measurement of responses. (1) Draw preimmunized blood sample, allow blood to clot, and separate serum from clot by centrifugation. Store serum at -20°C to -70°C in appropriately labeled plastic tubes. (2) Inject 0.5 ml of toxoid mixture into an appropriately prepared intramuscular site (deltoid or thigh), taking care not to inject material intravenously. (3) Inject 0.5 ml polyvalent pneumococcal vaccine into an appropriately prepared subcutaneous site, taking care not to inject material intravenously. (4) Draw post-immunization blood samples at desired intervals, usually at 1, 2, and 3 weeks. Separate serum and store at -20°C to -70°C. (5) After all serum samples are collected, assay samples for presence of antibodies using ELISA.

The ELISA offers a rapid, sensitive, reproducible, nonradioactive method for measuring *in vivo* antibody responses to a variety of antigens, including protein and polysaccharide antigens in sera obtained from individuals vaccinated with tetanus and diphtheria boosters and the polyvalent pneumococcal polysaccharide vaccine. Assays specific for tetanus, diphtheria and the pneumococcal polysaccharide types I, II, and III are detailed in Current Protocols in Immunology, 1995, Vols. 6 and 7.

### ASSAY USING TUMOR REJECTION

In another assay for immunomodulation, an immunocompent animal is vaccinated with on the order of 10⁴-10⁸ irradiated cytokine-coated tumor cells, and challenged with on the order of 10⁴-10⁸ live wild-type tumor cells (in any temporal sequence). If survival or tumor onset in these animals differs from that of animal vaccinated, using identical parameters, with irradiated non-cytokine coated cells instead of cytokine-coated cells, immunomodulation has occurred. For example, if at least 10% of the animals in the test group survive 100% longer than mean survival in the control group, the test is positive. As another example, onset of tumors in 20% of the test animals might be 50% later than mean onset in the control animals.

### USE

### Tumors for which the Invention is Applicable

The invention contemplates treatment of tumors including but not limited to the following: Melanomas, squamous cell tumors, basal cell carcinomas, astrocytomas, gliomas, glioblastoma multiforme, meningiomas, ependymomas, schwannomas, neuroblastomas, retinoblastomas, meningiomas, glomus tumors, sarcomas, including, e.g., osteosarcomas, Ewing's sarcomas, chondrosarcomas, myosarcomas, synovial cell sarcomas, fibrosarcomas, spindle cell tumors, angiosarcomas, primitive neuroectodermal cell tumors, and Kaposi's sarcomas, lymphomas, acute and chronic leukemias, tumors of the head and neck, nasopharyngeal carcinomas, carcinomas of the pharynx, laryngeal carcinomas, carcinomas of the thyroid, carcinomas of the parathyroids, thymomas, esophageal carcinomas, gastric carcinomas, tumors of the small bowel, carcinomas of the colon and rectum, mesotheliomas, lung carcinomas, including adenocarcinomas, squamous cell carcinomas, bronchoalveolar carcinomas, and small cell tumors, pancreatic carcinomas, islet cell and non-islet cell tumors, carcinomas of the breast, cardiac myxomas, pituitary tumors, carcinoid tumors, hepatomas, cholangiocarcinomas, hepatoblastomas, renal cell carcinomas, nephroblastomas, Wilms' tumors, adrenal carcinomas, pheochromocytomas, germ cell tumors, choriocarcinomas, ovarian carcinomas, testicular tumors, seminomas, endometrial tumors, carcinomas of the prostate, carcinomas of the seminal vesicles, vaginal tumors, carcinomas of the penis, hydatiform moles, carcinomas of the gall bladder, and carcinomas of the urinary bladder.

### TRANSGENIC ANIMALS ACCORDING TO THE INVENTION

A nucleic acid molecule encoding an engineered cytokine as described herein can be used to produce nonhuman transgenic animals, and cells of such transgenic animals can be isolated and used in a vaccine formulation in animal or human vaccination.

For example, in one embodiment, a nucleic acid molecule is introduced into a fertilized oocyte or an embryonic stem cell. Such cells can then be used to create non-human transgenic animals in which exogenous nucleic acid molecules encoding the polypeptides of the invention have been introduced into their genome or homologous recombinant animals in which endogenous nucleic acid molecules have been altered. Such animals are useful for studying the function and/or activity of the molecules of the invention and for identifying and/or evaluating modulators of the activity of the molecules of the invention. As used herein, a "transgenic animal" is a non-human animal, prefers mammal, more preferably a mouse, in which one or more of the cells of the animal includes a transgene. A transgene is exogenous nucleic acid which is integrated into the genome of a cell from which a transgenic animal develops and which remains in the genome of the mature animal, thereby directing the expression of an encoded gene product in one or more cell types or tissues of the transgenic animal.

A transgenic animal of the invention can be created by introducing nucleic acid molecules encoding the polypeptides described herein into the male pronuclei of a fertilized oocyte, e.g., by microinjection, and allowing the oocyte to develop in a pseudopregnant female foster animal. Intronic sequences and polyadenylation signals can also be included in the transgene to increase the efficiency of expression of the transgene. A tissue-specific regulatory sequence(s) can be operably linked to the transgene to direct expression of a polypeptide of the invention to particular cells. Methods for generating transgenic animals via embryo manipulation and microinjection, particularly animals such as mice, have become conventional in the art and are described, for example, in U.S. Patent Nos. 4,736,866 and 4,870,009, both by Leder et al., U.S. Patent No. 4,873,191 by Wagner et al. and in Hogan, B., Manipulating the Mouse Embryo, (Cold Spring Harbor Laboratory Press, Cold Spring Harbor, N.Y., 1986). Similar methods are used for production of other transgenic animals. A transgenic founder animal can be identified based upon the presence of the nucleic acid molecule of the invention, e.g., the transgene in its genome and/or expression of the transgene mRNA in tissues or cells of the animals. A transgenic founder animal can then be used to breed additional animals carrying the transgene. Moreover, transgenic animals carrying a transgene encoding polypeptides of the invention can further be bred to other transgenic animals carrying other transgenes.

The invention is further illustrated by the following exemplifications which should not be construed as being further limiting.

### EXEMPLIFICATIONS

Construction of Engineered Ligands for CD40 (for reference only)

### EXAMPLE 1

### Construction of Human CD154-GPI

A human CD 154-GPI construct is prepared by Polymerase chain reaction (PCR) methods using the PCR parameters and oligonucleotides described below.
The following oligonucleotides are used to amplify either of two portions of CD 154 from human CD4+ T cell total cDNA:
Upstream oligonucleotide either:
5'pCCGAATTCCATAGAAGGTTGGACAAGATAGAA3',
or
5'pCCGAATTCGAGACGAAGAAAGAAAGAAAACAG3';
Downstream oligonucleotide:
5'pTAGCCGGCGAGTTTGAGTAAGCCAAAGGACG3'
PCR parameters are:

| | | |
|---|---|---|
| Denaturation | 94° | 1 minute |
| Annealing | 65° | 1 minute |
| Extension | 72° | 1 minute |
| Hold | 72° | 10 minutes (last cycle) |

PCR is performed using Pfu polymerase for the least number of cycles that yields a visible band on an agarose gel. After completion of the PCR, the reaction is allowed to cool at 4° for 10 minutes. The PCR product is purified after electrophoresis through a 1.2% agarose gel. The DNA band is excised and the DNA fragment purified using a kit purchased from Qiagen.

The purified DNA fragment is digested with EcoRI and NgoM1. After digestion, the reaction mix is extracted with phenol:chloroform (1:1) followed by chloroform. The aqueous phase is adjusted to 0.3M sodium acetate pH 5.2 and the DNA is precipitated with 2 volumes of ethanol at 80° for 2 hours.

The DNA is pelleted by centrifugation, ethanol is removed, and the pellet is rinsed with 70% ethanol. The pellet is dried under vacuum.

The DNA is resuspended in sterile water and ligated to pUC19-GPI 21 (as described hereinbelow) that has previously been digested with EcoRI-NgoM1. Ligation is for one hour at room temperature. PUC19 GPI 21 ligated to the amplified DNA is used to transform competent AG-1 cells. Transformed AG-1 cells are selected on LB plates containing ampicillin. Plasmid DNA is isolated and analyzed as below. Restriction digests are done to confirm the pUC 19 CD154 -GPI 21 chimeric construct. The DNA from a positive clone is isolated and sequenced to confirm its identity.

### EXAMPLE 2 (for reference only)

### Construction of Mouse CD154-GPI

A murine CD154-GPI construct is prepared by PCR methods using the PCR parameters and oligonucleotides described below.

The following oligonucleotides are used to amplify either of two portions of CD 154 from mouse CD4+ T cell total cDNA: Upstream oligonucleotide-either 5'pCCGAATTCCATAGAAGATTGGATAAGGTCGA3',
or
5'pCCGAATTCGAGAAAAAAGAAAACAGCTTTGAAA3';
downstream oligonucleotide:
5'pTAGCCGGCGAGTTTGAGTAAGCCAAAAGATG3'
PCR parameters are:

| | | |
|---|---|---|
| Denaturation | 94° | 1 minute |
| Annealing | 65° | 1 minute |
| Extension | 72° | 1 minute |
| Hold | 72° | 10 minutes (last cycle) |

PCR is performed using Pfu polymerase for the least number of cycles that yields a visible band on agarose gel electrophoresis. After PCR, the reaction is allowed to cool at 4° for 10 minutes. The PCR product is purified after electrophoresis through a 1.2% agarose gel. The DNA band is excised and the DNA fragment purified using a kit purchased from Qiagen.

The purified DNA fragment is digested with EcoRI and NgoM1. After digestion, the reaction mix is extracted with phenol:chloroform (1:1) followed by chloroform. The aqueous phase is adjusted to 0.3M sodium acetate pH 5.2 and the DNA is precipitated with 2 volumes of ethanol at -80° for 2 hours.

The DNA is pelleted by centrifugation, ethanol is removed, and the pellet is rinsed with 70% ethanol. The pellet is dried under vacuum.

The DNA is resuspended in sterile water and ligated to pUC19-GPI 21 (as described hereinbelow) that has previously been digested with EcoRI-NgoM1. Ligation is for one hour at room temperature. PUC19 GPI 21 ligated to the amplified DNA is used to transform competent AG-1 cells. Transformed AG-1 cells are selected on LB plates with ampicillin. Plasmid DNA is isolated and analyzed as below. Restriction digests are done to confirm the pUC 19 CD154 -GPI 21 chimeric construct. The DNA from a positive clone is isolated and sequenced to confirm its identity. To measure expression of the chimeric protein, the fragment is cloned into an expression vector that places a secretory signal sequence, e.g., that of mouse IL-2, in frame with, and immediately upstream of, the CD154 fragment.

### Construction of Engineered Cytokines

### EXAMPLE 3

### Construction of GM-CSF-GPI

A GM-CSF-GPI construct was prepared as described below.

The following two oligonucleotides were purchased from Midland Certified Reagent Company (Midland, TX):
GTX-5 
5'pAATTCCGCGCCGGCACAGTGCTCAGAGACAAACTGGTCAAGTGTGAGGGCATCAGC
CTGCTGGCTCAGAACACCTCGTGGCTGCTGCTGCTCCTGCTGTCCCTCTCCCTCCTCCAG
GCCACGGATTTCATGTCCCTGTGACTGGGTAC3'
 GTX-5 comprises :
a. Sequences at the 5' end suitable for ligating to an EcoRI site (bases 1-5)
b. An NgoM1 site for creating an in-frame chimeric coding sequence (bases 9-14)
c. The coding sequence for the GPI modification sequence of human Thy-1 (Genbank Accession No. M11749) (bases 15-137)
d. A termination codon (bases 138-140)
e. Sequences at the 3' end for ligating to a KpnI site (bases 144-148)

The oligonucleotide complementary to GTX-5, except for staggered ends, called GTX-6 (below) was also purchased.
GTX-6:

5'pCCAGTCACAGGGACATGAAATCCGTGGCCTGGAGGAGGGAGAGGGACAGCAGGAG
CAGCAGCAGCCACGAGGTGTTCTGAGCCAGCAGGCTGATGCCCTCACACTTGACCAGT
TTGTCTCTGAGCACTGTGCCGGCGCGG3'

GTX-5 and GTX-6 were dissolved in individual tubes in sterile water at a final concentration of 1 microgram/lambda. GTX-5 and GTX-6 were mixed at a final concentration of 100ng/lambda and allowed to anneal for 60 minutes at room temperature.

The GTX-5:GTX-6 double stranded oligonucleotide (Thy-GPI) was then cloned into the plasmid pUC 19. Four (4) micrograms of pUC19 DNA was digested with EcoRI and KpnI. After electrophoresis, the linear DNA was purified from a 0.7% agarose gel using a Qiagen (Santa Clarita, CA) gel purification kit according to instructions provided by the manufacturer. 100ng of the GTX-5:GTX-6 oligonucleotide was ligated to 200 ng of the EcoRI-KpnI digested pUC19 in a final volume of 20 microliters at room temperature for 60 minutes.

The plasmid was transformed into competent AG-1 cells, which were purchased from Stratagene. Transformed *E. coli* were inoculated onto LB-amp plates. Bacterial colonies growing on LB plates containing ampicillin (100 micrograms/ml) were picked and inoculated into one ml of LB with amp and grown overnight at 37° with shaking.

Plasmid DNA was isolated using a standard alkaline lysis miniprep protocol. Plasmid DNA was digested with EcoRI and KpnI. DNA was electrophoresed on 1.6% agarose gels stained with ethidium bromide, and colonies containing an EcoRI-KpnI fragment of approximately 148 bp were thus identified. Positive colonies were inoculated into 100ml of LB with ampicillin and grown overnight. Plasmid DNA was purified using kits purchased from Qiagen.

The nucleotide sequence of a Thy-GPI positive clone, now designated pUC-GPI 21, was sequenced to confirm its identity.

The GM-CSF coding sequence was amplified from a mouse lung cDNA library purchased from Clontech (Palo Alto, CA). The primers for PCR were as follows:
Upstream oligonucleotide:
5'CCGAATTCATGTGGCTGCAGAATTTACTTTTCCTGGGCATTGTGGTCTAC3'
Downstream oligonucleotide:
5'CAGCCGGCTTTTTGGACTGGTTTTTTGCATTCAAAGGGGATATCAGTCAG3'
PCR parameters were:

| | | |
|---|---|---|
| Denaturation | 90° | 1 minute |
| Annealing | 60° | 1 minute |
| Extension | 72° | 1 minute |

PCR was performed for 30 cycles using Taq polymerase. After completion of the PCR, the reaction was allowed to cool at 4° for 10 minutes.

The GM-CSF PCR product was purified after electrophoresis through a 1% agarose gel. The DNA band was excised and the DNA fragment purified using a kit purchased from Qiagen.

The purified GM-CSF DNA fragment was then digested with EcoRI and NgoM1. After digestion, the reaction mix was extracted with phenol:chloroform (1:1) followed by chloroform. The aqueous phase was adjusted to 0.3M sodium acetate pH 5.2 and the DNA precipitated with 2 volumes of ethanol at -80° for 2 hours. The DNA was pelleted by centrifugation, ethanol was removed, and the pellet was rinsed with 70% ethanol. The pellet was dried under vacuum.

The GM-CSF cDNA was resuspended in sterile water and ligated to pUC19-GPI 21 that had been digested with EcoRI-NgoM1. Ligation was for one hour at room temperature. PUC 19 GPI 21 ligated to GM-CSF was used to transform competent AG-1 cells. Transformed AG-1 cells were inoculated onto LB-amp plates with. Transformed colonies were inoculated individually into one ml of LB containing 100 micrograms/ml of ampicillin. Plasmid DNA was isolated by the rapid alkaline lysis method. Restriction digests were done to confirm the pUC 19 GM-CSF-GPI 21 chimeric construct. The DNA from a positive clone was isolated and sequenced to confirm its identity.

### EXAMPLE 4

### Construction of IL-4-GPI

A plasmid encoding GPI-linked murine IL-4 is constructed as follows. A cDNA encoding murine IL-4 (Genbank Accession No. M25892) is amplified by PCR (as described above) from a mouse spleen library using the following primers:
Upstream Primer:
   5'CCGAATTCATGGGTCTCAACCCCCAGCT3'
Downstream Primer:
   5'CAGCCGGCCGAGTAATCCATTTGCATGATG3'

The PCR product is isolated by agarose gel electrophoresis as above, digested with EcoRI and NgoM1, and purified as above. The cDNA is ligated into pUC19-GPI 21, and the plasmid is transformed into competent *E. coli* cells. Positive plasmid clones are isolated as above. The chimeric gene is cloned into an expression vector as described below.

### EXAMPLE 5

### Construction of IL-12 p40-GPI

A plasmid encoding GPI-linked murine IL-12 p40 subunit is constructed as follows. A cDNA encoding the p40 subunit of murine IL-12 (Genbank Accession No. M86671) is amplified from a mouse spleen library by PCR (as described above) using the following primers:
Upstream Primer:
   5'CCGAATTCATGTGTCCTCAGAAGCTAACCA3'
Downstream Primer:
   5'CAGCCGGCGGATCGGACCCTGCAGGGA3'

The PCR product is isolated by agarose gel electrophoresis as above, digested with EcoRI and NgoM1, and purified as above. The cDNA is ligated into pUC19-GPI 21, and the plasmid is transformed into competent *E. coli*. Positive plasmid clones are isolated as above. The chimeric gene is cloned into an expression vector as described below.

### EXAMPLE 6

### Construction of IL-12 p35-GPI

A plasmid encoding GPI-linked murine IL-12 p35 subunit is constructed as follows. A cDNA encoding the p35 subunit of murine IL-12 (Genbank Accession No. M86672) is amplified by PCR from a mouse spleen library using the following primers:
Upstream Primer:
   5'CCGAATTCATGTGTCAATCACGCTACCTCCT3'
Downstream Primer:
   5'CAGCCGGCGGCGGAGCTCAGATAGCCCATC3'

The PCR product is isolated by agarose gel electrophoresis as above, digested with EcoRI and NgoM1, and purified as above. The cDNA is ligated into pUC19-GPI 21, and the plasmid is transformed into competent *E. coli*. Positive plasmid clones are isolated as above. The chimeric gene is cloned into an expression vector as described below.

GPI-linked IL-12 p35 and p40 can be coexpressed in a host cell, or either GPI linked construct can be coexpressed with a non-GPI linked version of the other construct.

### Cloning of GPI Chimeric Proteins into a Mammalian Cell Expression Vector

### EXAMPLE 7

PUC 19 GM-CSF-GPI 21 was digested with EcoRI and KpnI, to release the GM-CSF-GPI 21 DNA fragment. The DNA fragment was purified following agarose gel electrophoresis using a kit purchased from Qiagen according to the manufacturer's protocol.

For transient expression in Cos 1 cells, the chimeric GM-CSF-GPI gene was cloned into the mammalian expression vector pSVK3 (Pharmacia Biotech, Piscataway, NJ). For stable expression in murine tumor cells and CHO cells, the gene was cloned into the pCI mammalian expression vector (Promega, Madison, WI). In both cases, the DNA fragment was ligated to plasmid that had been digested with EcoRI and KpnI.

Each ligation mix was used to transform AG-1 cells. Transformed cells were selected by growth on LB plates containing 100 micrograms/ml ampicillin. Transformed colonies were picked and inoculated into one ml of LB with ampicillin. Plasmid DNA was isolated by the rapid alkaline lysis method. Plasmid DNA was digested with restriction enzymes to confirm the presence of the inserts. Clones containing pSVK3 GM-CSF- GPI and pCI GM-CSF-GPI were selected. Plasmid DNA from 100 ml cultures of each clone was isolated for electroporation into Cos 1 cells, B16 murine melanoma or CHO cells.

### Electroporation of GM-CSF-GPI into Cos 1, B16, or CHO cells

### EXAMPLE 8

### Transient expression in Cos-1 cells

Cos 1 cells were grown in DMEM-10% FBS with Pen-Strep. Cells at approximately 75% confluency were harvested by trypsinization. Trypsinized cells were diluted into DMEM-10% FBS with Pen-Strep and pelleted by centrifugation at 1500 rpm (approximately 1500g) for 3 minutes in a Heraeus Biofuge 15R Centifuge. The supernatant was removed and the cells were resuspended in DMEM-10%FBS with Pen-Strep, at a final concentration of approximately 6 x 10⁶ cells/ml.

For electroporation, 0.6 ml of resuspended cells were electroporated with 10 micrograms ( at 1 microgram/microliter) of pSVK3 GM-CSF- GPI 21.

Electroporation was performed using a BioRad Gene Pulser. Conditions were as recommended by the manufacturer. After electroporation the cells were rinsed with DMEM-10% FBS with Pen-Strep (P-S) and plated in tissue culture flasks.

Cells were harvested after 48 hours. Media was removed and the cells were rinsed with sterile PBS. The cells were then scraped from the tissue culture flask and the cells were pelleted by microcentrifugation for 4 minutes at room temperature.

### Stable expression of GM-CSF-GPI in B16 melanoma cells

Plasmid pCI GMCSF-GPI was linearized with the restriction enzyme Bgl II. To select for stable integration of GM-CSF-GPI into B16 melanoma cells, pCI GMCSF-GPI was electroporated into B16 cells along with the gene conferring resistance to G418 (*neo^{r}*) in molar ratios of 20:1 respectively. Conditions for electroporation were 250V, 750 uF, in a 0.4cm electrode gap cuvette using a BioRad Gene Pulser II with a Capacitance Extender Plus. Following electroporation, cells were grown for 2 days in DMEM, 10% FCS, P-S, in the absence of G418. After two days, the cells were fed with DMEM, 10% FCS, P-S, supplemented with 2mg/ml G418 (Clontech). After selection, the G418 resistant B 16 cells that had been electroporated with pCI GMCSF-GPI were propagated in DMEM, 10% FCS, P-S, supplemented with 1 mg/ml G418.

### Stable Expression of GM-CSF-GPI in CHO Cells

Plasmid pCI GMCSF-GPI was linearized with the restriction enzyme Bgl II. To select for stable expression of the GMCSF-GPI gene in CHO cells, pCI GMCSF-GPI was electroporated into cells along with the gene for dihydrofolate reductase (DHFR). CHO DG44 cells (obtained from Dr. Chasin, Columbia University) were electroporated with pCI GMCSF-GPI along with EcoRI digested pSV2 DHFR (ATCC) in a molar ratio of 20:1, respectively. Conditions for electroporation of CHO DG44 cells were 250 V, 1050 uF, in a 0.4 cm electrode gap cuvette using a BioRad Gene Pulser II with a Capacitance Extender Plus. Cells were grown for 2 days in MEM Alpha Media (Gibco BRL), 7% Dialyzed FCS, with HT Supplement (Gibco BRL). After two days, the cells were placed into selective media comprising MEM Alpha Media, 7% Dialyzed FCS without HT Supplement. A stable pool of transfected cells was maintained. To amplify the GM-CSF-GPI sequences, transfected cells were grown in stepwise increasing concentrations (0.02 uM, 0.1uM, 0.5 uM, and 1.0 uM) of methotrexate. A pool of CHO cells, electroporated with pCI GM-CSF-GPI ( and pSV2 DHFR) and resistant to 1.0 uM methotrexate was propagated.

### Vaccination Using Cytokine-coated Cells and Demonstration of Effectiveness of Cytokine-coated Cells

### Example 9

### Vaccination Using Cytokine-coated Cells

The ability of B16 cells transfected with pCI-GM-CSF-GPI and *neo^{r}* to act as a preventive vaccine against tumor formation was compared to that of control cells, viz., B16 cells transfected only with "empty" pCI (i.e., plasmid with no insert) and *neo^{r}*. Four C57BL/6 mice were vaccinated subcutaneously in the abdomen with 5 x 10⁵ irradiated (3500 rads) control cells, and five mice were vaccinated with 5 x 10⁵ irradiated GM-CSF-GPI cells. Seven days later, all mice were challenged with 1 x 10⁶ live wild-type cells injected subcutaneously into the neck. Tumors were detected in the control set of mice on post-challenge days 7, 11 (2 mice), and 15. Tumors were detected in the GM-CSF-GPI set of mice on post-challenge days 11, 12, 15 (2 mice), and 16. The results demonstrated a survival period in the control set of mice of up to 15 (2 mice), 16, and 20 days; and a considerably longer survival period in the GM-CSF-GPI treated mice, i.e., up to days 16, 19, 21 (2 mice), and 32 days.

### EXAMPLE 10 (for reference only)

### Vaccination using CD40 ligand-enhanced cells

B16 tumor cells are irradiated and coated with GPI-linked murine CD154 using the procedure described hereinbelow. C57BL/6 mice are vaccinated in the inguinal fold with 5 x 10⁵ irradiated CO154-GPI coated cells. Control mice are injected with a replicate sample of irradiated cells which are not coated with CD154. One week later, mice are challenged with 1 x 10⁶ non-irradiated, non-CD154 enhanced wild-type B16 cells by injection in the neck. Mice vaccinated with CD154-enhanced cells will either have a mean 60-day survival period that is at least 20% greater than the survival period of control mice, or at 60 days after the challenge step (described above), at least 20% more mice of the that received CD154-GPI coated cells will be alive as compared to control mice.

### EXAMPLE 11 (for reference only)

### Construction of GPI-opsonin chimeras and vaccination using CD140 ligand enhanced, opsonin enhanced cells

The murine Mannose Binding Protein (MBP) coding sequence was amplified by PCR from a mouse liver cDNA library purchased from Clontech. The primers for PCR were as follows:
Upstream Primer:
5'CCGAATTCATGCTTCTGCTTCCATTACTCCCTGTCCTTCTGTGTGTG3'
Downstream Primer:
5'TAGCCGGCTGGGAACTCGCAGACAGCCTTGAAGGAAGCTTGACAGGA3'
PCR parameters were:

| | | |
|---|---|---|
| Denaturation | 95° | 1 minute |
| Annealing | 64° | 1 minute |
| Extension | 72° | 1 minute |
| Hold | 72° | 5 minutes (last cycle) |

PCR was performed for 30 cycles using Vent polymerase. After completion of the PCR, the reaction was allowed to cool at 4° for 10 minutes. The MBP PCR product was purified after electrophoresis through a 1% agarose gel. The DNA band was excised and the DNA fragment purified using a kit purchased from Qiagen.

### Construction of MBP-GPI 21 Chimeric

The purified MBP DNA fragment was digested with EcoRI and NgoMl. After digestion, the reaction mix was extracted with phenol:chloroform (1:1) followed by chloroform. The aqueous phase was adjusted to 0.3M sodium acetate pH 5.2 and the DNA precipitated with 2 volumes of ethanol at -80° for 2 hours. The DNA was pelleted by centrifugation, ethanol was removed, and the pellet was rinsed with 70% ethanol. The pellet was dried under vacuum.

The MBP DNA was resuspended in sterile water and ligated to pUC19-GPI 21 that had been digested with EcoRI-NgoM1. Ligation was for one hour at room temperature. PUC19 GPI 21 ligated to MBP was used to transform competent AG-1 cells. Plasmid DNA was isolated and analyzed as above. The DNA from a positive clone was isolated and sequenced to confirm its identity.

### Construction of C3b alpha chain-GPI 21 Chimeric

The C3b alpha chain coding sequence was amplified by PCR from a mouse liver cDNA library purchased from Clontech. The primers for PCR were as follows:
Upstream Primer: 
5’GCGAATTCCGCCTAGGAGTGAATTGGAGGAAGACATAATTCCAGAAGAAGATATTAT
C3’
Downstream Primer:
5'TAGCCGGCGTTGGGACAACCATAAACCACCATAGATTCTGTGAATGC3'
PCR parameters were:

| | | |
|---|---|---|
| Denaturation | 94° | 1 minute |
| Annealing | 65° | 1 minute |
| Extension | 72° | 1 minute |
| Hold | 72° | 10 minutes (last cycle) |

PCR was performed for 35 cycles using Pfu polymerase. After completion of the PCR, the reaction was allowed to cool at 4° for 10 minutes.

The C3b alpha chain PCR product was purified after electrophoresis through a 1% agarose gel. The DNA band was excised and the DNA fragment purified using a kit purchased from Qiagen.

The purified C3b alpha chain DNA fragment was digested with EcoRI and NgoM1. After digestion, the reaction mix was extracted with phenol:chloroform (1:1) followed by chloroform. The aqueous phase was adjusted to 0.3M sodium acetate pH 5.2 and the DNA was precipitated with 2 volumes of ethanol at -80° for 2 hours. The DNA was pelleted by centrifugation, ethanol wasremoved, and the pellet was rinsed with 70% ethanol. The pellet was dried under vacuum.

The C3b alpha chain DNA was resuspended in sterile water and ligated to pUC19-GPI 21 that had been digested with EcoRI-NgoM1. Ligation was for one hour at room temperature. PUC19 GPI 21 ligated to C3b alpha chain DNA was used to transform competent AG-1 cells. Transformed AG-1 cells were selected on LB plates with ampicillin. Plasmid DNA was isolated and analyzed as above. Restriction digests were performed to confirm the pUC 19 C3b alpha chain -GPI 21 chimeric construct. The DNA from a positive clone was isolated and sequenced to confirm its identity.

These constructs can be artificially introduced into a host cell and the recombinant proteins can be purified by methods known in the art, e.g. immunoaffinity purification.

### Vaccination using CD154-enhanced, opsonin-enhanced cells

C57BL/6 mice are challenged with 1 x 10⁶ non-irradiated, non-enhanced wild-type B16 cells behind the neck. One week later, mice are vaccinated with 5 x 10⁵ irradiated B16 cells admixed, as described hereinbelow, with GPI-linked murine CD154 and a GPI-linked opsonin, e.g., C3b alpha' chain or MBP. Control mice are vaccinated with identical cells which are not enhanced with GPI-CD 154 or GPI-linked opsonin. Mice vaccinated with CD 154-enhanced/opsonin-enhanced cells will either have a mean 60-day survival period that is at least 20% greater than the survival period of control mice, or at 60 days after the challenge step (described above) at least 20% more of the mice that received irradiated B16 cells admixed with GPI-linked CD154 and GPI-linked opsonin will be alive as compared to control mice.

### EXAMPLE 12 (for reference only)

### Vaccination using CD40 mAb-enhanced cells or CD40 mAb-enhanced, opsonin-enhanced cells

A monoclonal antibody to CD40 is coupled to palmitate (Colsky et al , 1989, J. Immunol Methods, 124:179-87). The lipid-linked antibody is then used in lieu of GPI-CD154 in the vaccination and methods described in Example 10 and 11 (above).

### Vaccination using cells enhanced with CD154 and IL-4

### EXAMPLE 13 (for reference only)

GPI-linked IL-4 is prepared as described in Example 4 and the vaccination methods described in Example 10 are performed with GPI-linked IL-4 added to the GPI-CD154 coated vaccines.

### EXAMPLE 14

### Vaccination Using GMCSF-coated cells

B 16 tumor cells are irradiated and coated with GPI-linked GM-CSF using the procedure described hereinbelow. C57BL/6 mice are vaccinated with 5 x 10⁵ GPI-GMCSF coated cells in the inguinal fold. Control mice are injected with identical cells which are not coated with GPI-GM-CSF. One week later, mice are challenged with 1 x 10⁶ non-irradiated, non-coated wild-type B16 cells by injection behind the neck. Mice vaccinated with cytokine-coated cells will have a mean 60-day survival period that is at least 20% greater the survival period of control mice, or at 60 days after the challenge step (described above) at least 20% more of the mice that received GPI-GMCSF coated cells will be alive as compared to control mice.

### EXAMPLE 15

### Vaccination Using GPI-GMCSF, GPI-opsonin enhanced cells

C57BL/6 mice are challenged with 1 x 10⁶ non-irradiated, non-coated wild-type B16 cells by injection behind the neck. One week later, mice are vaccinated with 5 x 10⁵ irradiated B16 cells coated with GPI-linked GM-CSF and a GPI-linked opsonin, e.g., C3b alpha' chain or MBP. Control mice are vaccinated with a replicate sample of irradiated cells which are not coated with GPI-GM-CSF or GPI-linked opsonin. Mice vaccinated with cytokine-coated cells will have a mean 60-day survival period that is at least 20% greater the survival period of control mice, or at 60 days after the challenge step (described above) at least 20% more of the mice that received GMCSF and opsonin coated cells will be alive as compared to control mice.

### EXAMPLE 16

Recombinant IL-12 is coupled to palmitate using the protocol described hereinabove. The lipid-linked IL-12 is then added to the vaccines and methods described in Examples 14 and 15, or substituted for GM-CSF in those examples.

### EXAMPLE 17

The methods of Examples 15 and 16 are performed with GPI-linked IL-4 (prepared as described in Example 4) added to the GPI-GM-CSF coated vaccines.

### Preparation of a Transmembrane Engineered Opsonin

### EXAMPLE 18

In another example, a transmembrane engineered opsonin comprising an APC-binding moiety of C3b is prepared. A portion of the C3b fragment of murine complement C3 is amplified by PCR from mouse liver cDNA using an upstream primer corresponding to nucleotides 2304-2324 of the sequence comprising Genbank Accession #K02782 plus a 5' "ATG" and a downstream primer complementary to nt 2429-2453 of the sequence comprising Genbank accession #K02782. The transmembrane region of mouse IgG3 is amplified from mouse spleen cDNA using an upstream primer which corresponds to nucleotides 100-125 of the sequence comprising Genbank Accession #V01526 and contains 12 bases at the 5' end that are complementary to the 5' end of the antisense strand of the C3b fragment, and a downstream primer which complements nucleotides 823-846 of the sequence comprising Genbank Accession #V01526, and incorporates a downstream stop codon plus an extension containing an appropriate restriction site. Both PCR products are isolated by agarose gel electrophoresis (1.2% agarose for both), eluted from the excised agarose bands using glass beads (Geneclean Glass Beads, Manufacturer's Instructions, Bio 101, Inc., Vista, CA), and quantitated by spectrophotometry. The two amplified sequences are fused to each other in-frame using the overlaping PCR method (Horton et al., 1989, Gene, 77:61-8). Briefly, equimolar amounts of the two fragments are used in a PCR reaction with excess amounts of upstream C3b primer and downstream IgG3 primer.

The above products can be cloned into suitable expression vectors, in the presence or absence of an upstream secretory signal if necessary, and expressed in the appropriate antigen-comprising cells, e.g., malignant tumor cells. The transfected cells can be admixed with a lipid-linked engineered ligand for CD40 and/or a lipid-linked engineered cytokine and then used as a vaccine against an antigen comprised by the cells.

### Use of Engineered Molecules to Prepare Enhanced Cells

### EXAMPLE 19

Cytokine-coated, or opsonin-enhanced cells can be prepared by introducing a nucleic acid encoding an engineered cytokine, or an opsonin into the cells, as described hereinabove. Lipid-linked engineered cytokines, or opsonins can also be used to prepare cytokine-coated or opsonin-enhanced cells using the following procedure (McHugh et al, op. cit.). Cells are suspended in a suitable buffer, e.g.,HBSS, phosphate-buffered saline, or cell culture media at a concentration on the order of 1 x 10⁵ to 1 x 10⁸ cells/ml. A lipid-linked molecule, e.g., palmitate-linked GM-CSF, is added at on the order of 10-200 ug/ml, and the admixture is incubated at 37°C for between 2 and 20h. Cells are washed in buffer three times before use.

Alternatively, in a method that is particularly useful for palmitate-linked molecules, cells are suspended at 5x10⁶/ml serum-free DMEM. 0.9ml aliquots of cell mix are then dispensed into 15ml conical tubes. 0.1ml of engineered protein at 0.1 mg/ml-1 mg/ml in PBS/0.15% deoxycholate/0.1% sodium bicarbonate/pH 7.8 is added to each tube. The tubes are placed on angle in a styrofoam holder in an ice bath, which shaken for 4hrs. The cells are spun down, washed twice with PBS, and resuspended for use.

### EXAMPLE 20

Human peripheral blood mononuclear cells (PBMC) are isolated from whole blood by gradient centrifugation, resuspended in RPMI 1640 with 10% fetal calf serum, and placed in a plastic dish for 2h at 37°C in 5% CO₂. Non-adherent cells are then discarded and adherent cells are cultured for 7 days with 800 U/ml human GM-CSF and 500 U/ml human IL-4. About 1 x 10⁴ to 1 x 10⁸ of these cells are then collected, plated out, and admixed with about 1 x 10⁴ to 1 x 10⁸ cytokine-coated cells which comprise cells harvested from a subject with cancer, e.g. cytokine-coated malignant tumor cells which comprise a ligand for a receptor for TNF-α and/or a ligand for a receptor for IL-12. This admixture is incubated at 37°C for about 5 min to 6h. About 1 x 10⁴ to 1 x 10⁸ of the APC's are then administered to the subject, e.g by subcutaneous injection.

### EXAMPLE 21

Murine bone-marrow macrophages are harvested. About 1 x 10⁴ to 1 x 10⁸ of these cells are then collected, plated out, and admixed with about 1 x 10⁴ to 1 x 10⁸ cytokine-coated B16 melanoma cells, e.g. cytokine-coated B16 cells which comprise a ligand for a receptor for GM-CSF and/or a ligand for a receptor for IL-12. This admixture is incubated at 37°C for about 5 min to 6h. About 1 x 10⁴ to 1 x 10⁸ of the APC's are then administered a B16 tumor-bearing mouse, e.g by subcutaneous injection.

### Dosage, Mode of Administration and Pharmaceutical Formulation

The invention encompasses methods of modulating an immune response in a mammal to a selected antigen, the method comprising administering to a mammal a therapeutic amount of cytokine-coated and/or opsonin-enhanced cells, or administering a therapeutic amount of APCs which have been contacted with, cytokine-coated cells and/or opsonin-enhanced cells in vitro.

Cells described herein may be prepared as injectables, either as liquid solutions or suspensions; solid forms suitable for solution in or suspension in, liquid prior to infection can also be prepared. The preparation can also be emulsified, or the cells encapsulated in liposomes. The active immunogenic ingredients are often mixed with carriers which are pharmaceutically acceptable and compatible with the active ingredient. The term "pharmaceutically acceptable carrier" refers to a carrier that does not cause an allergic reaction or other untoward effect in subjects to whom it is administered. Suitable pharmaceutically acceptable carriers include, for example, one or more of water, saline, phosphate buffered saline, dextrose, glycerol, ethanol, or the like and combinations thereof. In addition, if desired, the vaccine can contain minor amounts of auxiliary substances such as wetting or emulsifying agents, pH buffering agents, and/or adjuvants which enhance the effectiveness of the vaccine. Examples of adjuvants which may be effective include but are not limited to: aluminum hydroxide, N-acetyl-muramyl-L-threonyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2-(1'-2'-dipalmitoyl-sn-glycero-3-hydroxyph osphoryloxy)-ethylamine (COP) 19835A, referred to as MTP-PE), and RIBI, which contains three components extracted from bacteria, monophosporyl lipid A, trehalose dimycolate and cell wall skeleton (MPL+TDM+CWS) in a 2% squalene/Tween 80 emulsion. Other examples of adjuvants include DDA (dimethyldioctadecylammonium bromide), Freund's complete and incomplete adjuvants and QuilA. In addition, immune modulating substances such as lymphokines (e.g., IFN-, IL-2 and IL- 12) or synthetic IFN- inducers such as poly I:C can be used in combination with adjuvants described herein.

Cells of the invention can be administered parenterally, by injection, for example, either subcutaneously or intramuscularly. Additional formulations which are suitable for other modes of administration include suppositories, and in some cases, oral formulations or formulations suitable for distribution as aerosols. In the case of the oral formulations, the manipulation of T-cell subsets employing adjuvants, antigen packaging, or the addition of individual cytokines to various formulations can result in improved oral vaccines with optimized immune responses. For suppositories, traditional binders and carriers may include, for example, polyalkylene glycols or triglycerides; such suppositories may be formed from mixtures containing the active ingredient in the range of 0.5% to 10%, preferably 1%-2%. Oral formulations include such normally employed excipients as, for example, pharmaceutical grades of mannitol, lactose, starch magnesium stearate, sodium saccharine, cellulose, magnesium carbonate, and the like. These compositions take the form of solutions, suspensions, tablets, pills, capsules, sustained release formulations or powders and contain 10%-95% of active ingredient, preferably 25-70%.

The cells of the invention can be formulated into the vaccine compositions as neutral or salt forms. Pharmaceutically acceptable salts include the acid addition salts (formed with free amino groups of the peptide) and which are formed with inorganic acids such as, for example, hydrochloric or phosphoric acids, or with organic acids such as acetic, oxalic, tartaric, maleic, and the like. Salts formed with the free carboxyl groups can also be derived from inorganic bases such as, for example, sodium, potassium, ammonium, calcium, or ferric hydroides, and such organic bases as isopropylamine, trimethylamine, 2-ethylamino ethanol, histidine, procaine, and the like.

Any cellular component of such vaccine compositions can, in preparation for inclusion in such compositions, be subjected to treatments which involve attenuation or inactivation of the cells of the vaccine, including, for example, exposure to ionizing radiation, which can inhibit cell division, antiproliferative agents such as cyclophosphamide, cytochalasin D, or colchicine, or killing with or without fixation.

The cells are administered in a manner compatible with the dosage formulation, and in such amount as will be prophylactically and/or therapeutically effective. The quantity to be administered depends on the subject to be treated, including, e.g., capacity of the subject's immune system to synthesize antibodies, and the degree of protection desired. Suitable dose ranges are on the order of several hundred micrograms active ingredient per vaccination with a preferred range from about 0.1ug to 1000ug, such as in the range from about 1ug to 300ug, and preferably in the range from about 10ug to 50ug. Suitable regiments for initial administration and booster shots are also variable but are typified by an initial administration followed by subsequent inoculations or other administrations. Precise amounts of active ingredient required to be administered depend on the judgment of the practitioner and may be peculiar to each subject. It will be apparent to those of skill in the art that the therapeutically effective amount of cells of this invention will depend, *inter alia*, upon the administration schedule, the unit dose of antigen administered, whether the cells are administered in combination with other therapeutic agents, the immune status and health of the recipient, and the therapeutic activity of the particular composition.

The cells can be given in a single dose schedule, or preferably in a multiple dose schedule. A multiple dose schedule is one in which a primary course of vaccination can include 1-10 separate doses, followed by other doses given at subsequent time intervals required to maintain and or reinforce the immune response, for example, at 1-4 months for a second dose, and if needed, a subsequent dose(s) after several months. Periodic boosters at intervals of 1-5 years, usually 3 years, are preferable to maintain the desired levels of protective immunity. The course of the immunization can be followed by *in vitro* proliferation assays of peripheral blood lymphocytes (PBLs) co-cultured with ESAT6 or ST- CF, and by measuring the levels of IFN- released from the primed lymphocytes. The assays can be performed using conventional labels, such as radionucleotides, enzymes, fluorescent labels and the like. These techniques are known to one skilled in the art and can be found in US Patent Nos. 3,791,932, 4,174,384 and 3,949,064.

### OTHER EMBODIMENTS

The foregoing examples demonstrate experiments performed and contemplated by the present inventors in making and carrying out the invention. It is believed that these examples include a disclosure of techniques which serve to both apprise the art of the practice of the invention and to demonstrate its usefulness. It will be appreciated by those of skill in the art that the techniques and embodiments disclosed herein are preferred embodiments only that in general numerous equivalent methods and techniques may be employed to achieve the same result.

## Claims

1. A vaccine composition comprising a cytokine-coated cell obtainable by admixing a cytokine with a cell, wherein the cytokine is introduced from or produced outside the cell and comprises a heterologous cell-membrane binding moiety which can bind to said cell when admixed with said cell.

2. The vaccine composition of claim 1 wherein said heterologous cell-membrane binding moiety comprises a GPI-moiety.

3. The vaccine composition of claim 1 or 2 wherein said cytokine is a ligand for the GM-CSF receptor.

4. The vaccine composition of claim 1 or 2 wherein said cytokine is a ligand for one of the following receptors: the IL-2 receptor, the IL-4 receptor, the IL-6 receptor, the IL-10 receptor, the IL-12 receptor, the TNF-α receptor, the IFN-γ receptor, or a chemokine receptor.

5. A vaccine composition according to any preceding claim, wherein the cell is a pathogenic cell.

6. The vaccine composition according to claim 5, wherein said cytokine coated cell is a malignant tumour cell.

7. The vaccine composition according to claim 5, wherein said cell of said cytokine-coated cell is selected from the group consisting of: a bacterium, a fungus, or a cell of a parasite.

8. The vaccine composition according to any preceding claim, which further comprises an opsonin-enhanced cell.

9. A vaccine composition according to any preceding claim, wherein said cell is unable to divide in vitro.

10. A method for preparing a cytokine-coated cell for use in a vaccine composition according to any preceding claim, which comprises the step of admixing a cytokine with a cell, wherein the cytokine comprises a heterologous cell-membrane binding moiety.

11. A vaccine composition comprising a cytokine-coated cell according to any of claims 1 to 9, for vaccinating a mammal to a selected antigen, wherein the cytokine-coated cell comprises the selected antigen.

## Patentansprüche

1. Vakzin-Zusammensetzung, die eine Cytokin-beschichtete Zelle umfasst, welche durch Beimischen eines Cytokins zu einer Zelle erhältlich ist, wobei das Cytokin von außen in die Zelle eingeführt wird oder außerhalb der Zelle produziert wird und eine heterologe Zell-Membranbindungsgruppierung umfasst, welche an die Zelle binden kann, wenn sie der Zelle zugemischt wird.

2. Vakzin-Zusammensetzung gemäß Anspruch 1, wobei die heterologe Zell-Membranbindungsgruppierung eine GPI-Gruppierung umfasst.

3. Vakzin-Zusammensetzung gemäß Anspruch 1 oder 2, wobei das Cytokin ein Ligand für den GM-CSF-Rezeptor ist.

4. Vakzin-Zusammensetzung gemäß Anspruch 1 oder 2, wobei das Cytokin ein Ligand für einen der folgenden Rezeptoren ist: den IL-2-Rezeptor, den IL-4-Rezeptor, den IL-6-Rezeptor, den IL-10-Rezeptor, den IL-12-Rezeptor, den TNF-α-Rezeptor, den IFN-γ-Rezeptor oder eignen Chemokin-Rezeptor.

5. Vakzin-Zusammensetzung gemäß einem vorangehenden Anspruch, wobei die Zelle eine pathogene Zelle ist.

6. Vakzin-Zusammensetzung gemäß Anspruch 5, wobei die Cytokin-beschichtete Zelle eine maligne Tumorzelle ist.

7. Vakzin-Zusammensetzung gemäß Anspruch 5, wobei die Zelle der Cytokin-beschichteten Zelle aus der Gruppe, bestehend aus einem Bakterium, einem Pilz oder einer Zelle eines Parasiten, ausgewählt ist.

8. Vakzin-Zussmmensetzung gemäß einem vorangehenden Anspruch, die außerdem eine Opsonin-verstärkte Zelle umfasst.

9. Vakzin-Zusammensetzung gemäß einem vorangehenden Anspruch, wobei die Zelle unfähig ist, sich in vitro zu teilen.

10. Verfahren zur Herstellung einer Cytokin-beschichteten Zelle zur Verwendung in einer vakzin-zusammensetzung gemäß einem vorangehenden Anspruch, umfassend den Schritt des Zumischens eines Cytokins zu einer Zelle, wobei das Cytokin eine heterologe Zell-Membranbindungsgruppierung umfasst.

11. Vakzin-Zusammensetzung, umfassend eine Cytokin-beschichtete Zelle gemäß einem der Ansprüche 1 bis 9 zur Impfung eines Säugers gegen ein ausgewähltes Antigen, wobei die Cytokin-beschichtete Zelle das ausgewählte Antigen umfasst.

## Revendications

1. Composition de vaccin comprenant une cellule recouverte de cytokine, qu'on peut obtenir en ajoutant et mélangeant une cytokine à une cellule, laquelle cytokine provient de la cellule ou est produite à l'extérieur de la cellule et comprend un fragment hétérologue de liaison à la membrane cellulaire, qui peut se lier à ladite cellule quand on l'ajoute et mélange à ladite cellule.

2. Composition de vaccin conforme à la revendication 1, dans laquelle ledit fragment hétérologue de liaison à la membrane cellulaire comprend un fragment GPI.

3. Composition de vaccin conforme à la revendication 1 ou 2, dans laquelle ladite cytokine est un ligand du récepteur de GM-CSF.

4. Composition de vaccin conforme à la revendication 1 ou 2, dans laquelle ladite cytokine est un ligand de l'un des récepteurs suivants : le récepteur de l'IL-2, le récepteur de l'IL-4, le récepteur de l'IL-6, le récepteur de l'IL-10, le récepteur de l'IL-12, le récepteur du TNF-α, le récepteur de l'IFN-γ, et un récepteur de chimiokine.

5. Composition de vaccin conforme à l'une des revendications précédentes, dans laquelle la cellule est une cellule pathogène.

6. Composition de vaccin conforme à la revendication 5, dans laquelle ladite cellule recouverte de cytokine est une cellule de tumeur maligne.

7. Composition de vaccin conforme à la revendication 5, dans laquelle ladite cellule recouverte de cytokine est choisie dans l'ensemble formé par une bactérie, un champignon et une cellule de parasite.

8. Composition de vaccin conforme à l'une des revendications précédentes, qui comporte en outre une cellule à teneur augmentée en opsonine.

9. Composition de vaccin conforme à l'une des revendications précédentes, dans laquelle ladite cellule est incapable de se diviser *in vitro*.

10. Procédé de préparation d'une cellule recouverte de cytokine, destinée à être utilisée dans une composition de vaccin conforme à l'une des revendications précédentes, lequel procédé comporte une étape consistant à ajouter et mélanger une cytokine à une cellule, laquelle cytokine comprend un fragment hétérologue de liaison à la membrane cellulaire.

11. Composition de vaccin comprenant une cellule recouverte de cytokine, conforme à l'une des revendications 1 à 9, conçue pour la vaccination d'un mammifère contre un antigène sélectionné, dans laquelle la cellule recouverte de cytokine comprend l'antigène sélectionné.
